# FASCICULE DE BREVET EUROPEEN

(11) **EP 2 880 174 B1**
(45) Date de publication et mention de la délivrance du brevet: **19.09.2018**
(21) Numéro de dépôt: 13756624.6
(22) Date de dépôt: 30.07.2013
(51) Int. Cl.: C12Q 1/18, C12Q 1/04, G01N 33/68

(54) **PROCEDE DE DETECTION D'AU MOINS UN MECANISME DE RESISTANCE AUX GLYCOPEPTIDES PAR SPECTROMETRIE DE MASSE**
VERFAHREN ZUR ERKENNUNG MINDESTENS EINES MECHANISMUS FÜR RESISTENZ GEGEN GLYCOPEPTIDE DURCH MASSENSPEKTROMETRIE
METHOD FOR DETECTING AT LEAST ONE MECHANISM OF RESISTANCE TO GLYCOPEPTIDES BY MASS SPECTROMETRY

(30) Priorité: 01.08.2012 FR 1257489
(43) Date de publication de la demande: 10.06.2015
(73) Titulaire: Biomérieux, 69280 Marcy l'Étoile (FR)
(72) Inventeur: CHARRETIER, Yannick, F-69690 Courzieu (FR); CHARRIER, Jean-Philippe, F-69160 Tassin La Demi-lune (FR); FRANCESCHI, Christine, F-01800 Meximieux (FR); ZAMBARDI, Gilles, 38230 Tignieu Jameyzieu (FR)
(74) Mandataire: Sprugnoli, Claude Louis Victor
(86) Numéro de dépôt international: PCT/FR2013/051833
(87) Numéro de publication internationale: WO 2014/020276

(56) Documents cités:
- WO-A2-2011/045544
- FR-A1- 2 668 489
- FR-A1- 2 699 537
- P. M. GRIFFIN ET AL: "Use of Matrix-Assisted Laser Desorption Ionization-Time of Flight Mass Spectrometry To Identify Vancomycin-Resistant Enterococci and Investigate the Epidemiology of an Outbreak", JOURNAL OF CLINICAL MICROBIOLOGY, vol. 50, no. 9, 27 juin 2012 (2012-06-27), pages 2918-2931, XP055061863, ISSN: 0095-1137, DOI: 10.1128/JCM.01000-12
- RADHOUANI HAJER ET AL: "Proteomic characterization of vanA-containing Enterococcus recovered from Seagulls at the Berlengas Natural Reserve, W Portugal", PROTEOME SCIENCE, BIOMED CENTRAL, LONDON, GB, vol. 8, no. 1, 21 septembre 2010 (2010-09-21), page 48, XP021078326, ISSN: 1477-5956, DOI: 10.1186/1477-5956-8-48
- S. SUJATHA ET AL: "Glycopeptide Resistance in Gram-Positive Cocci: A Review", INTERDISCIPLINARY PERSPECTIVES ON INFECTIOUS DISEASES, vol. 47, no. 6, 1 janvier 2012 (2012-01-01), pages 902-10, XP055062121, ISSN: 1687-708X, DOI: 10.1128/AAC.48.9.3636-3639.2004 cité dans la demande

### SEQUENCE LISTING

<110> bioMérieux
<120> Procédé de détection d'au moins un mécanisme de résistance aux glycopeptides par spectrométrie de masse
<130> VANCOMS
<150> FR1257489
   <151> 2012-08-01
<160> 154
<170> PatentIn version 3.5
<210> 1
   <211> 359
   <212> PRT
   <213> Unknown
<220>
   <223> peptide from a microbial resistance gene
<400> 1
   Met Lys Lys Pro Leu Lys Thr Val Trp Ile Leu Lys Gly Asp Arg Ser
   1               5                   10                  15
   Met Asn Arg Ile Lys Val Ala Ile Leu Phe Gly Gly Cys Ser Glu Glu
               20                  25                  30
   His Asp Val Ser Val Lys Ser Ala Ile Glu Ile Ala Ala Asn Ile Asn
           35                  40                  45
   Lys Glu Lys Tyr Glu Pro Leu Tyr Ile Gly Ile Thr Lys Ser Gly Val
       50                  55                  60
   Trp Lys Met Cys Glu Lys Pro Cys Ala Glu Trp Glu Asn Asp Asn Cys
   65                  70                  75                  80
   Tyr Ser Ala Val Leu Ser Pro Asp Lys Lys Met His Gly Leu Leu Val
                   85                  90                  95
   Lys Lys Asn His Glu Tyr Glu Ile Asn His Val Asp Val Ala Phe Ser
               100                 105                 110
   Ala Leu His Gly Lys Ser Gly Glu Asp Gly Ser Ile Gln Gly Leu Phe
           115                 120                 125
   Glu Leu Ser Gly Ile Pro Phe Val Gly Cys Asp Ile Gln Ser Ser Ala
       130                 135                 140
   Ile Cys Met Asp Lys Ser Leu Thr Tyr Ile Val Ala Lys Asn Ala Gly
   145                 150                 155                 160
   Ile Ala Thr Pro Ala Phe Trp Val Ile Asn Lys Asp Asp Arg Pro Val
                   165                 170                 175
   Ala Ala Thr Phe Thr Tyr Pro Val Phe Val Lys Pro Ala Arg Ser Gly
               180                 185                 190
   Ser Ser Phe Gly Val Lys Lys Val Asn Ser Ala Asp Glu Leu Asp Tyr
           195                 200                 205
   Ala Ile Glu Ser Ala Arg Gln Tyr Asp Ser Lys Ile Leu Ile Glu Gln
       210                 215                 220
   Ala Val Ser Gly Cys Glu Val Gly Cys Ala Val Leu Gly Asn Ser Ala
   225                 230                 235                 240
   Ala Leu Val Val Gly Glu Val Asp Gln Ile Arg Leu Gln Tyr Gly Ile
                   245                 250                 255
   Phe Arg Ile His Gln Glu Val Glu Pro Glu Lys Gly Ser Glu Asn Ala
               260                 265                 270
   Val Ile Thr Val Pro Ala Asp Leu Ser Ala Glu Glu Arg Gly Arg Ile
           275                 280                 285
   Gln Glu Thr Ala Lys Lys Ile Tyr Lys Ala Leu Gly Cys Arg Gly Leu
               290                 295                 300
   Ala Arg Val Asp Met Phe Leu Gln Asp Asn Gly Arg Ile Val Leu Asn
   305                 310                 315                 320
   Glu Val Asn Thr Leu Pro Gly Phe Thr Ser Tyr Ser Arg Tyr Pro Arg
                   325                 330                 335
   Met Met Ala Ala Ala Gly Ile Ala Leu Pro Glu Leu Ile Asp Arg Leu
               340                 345                 350
   Ile Val Leu Ala Leu Lys Gly
           355
<210> 2
   <211> 343
   <212> PRT
   <213> Unknown
<220>
   <223> peptide from a microbial resistance gene
<400> 2
   Met Asn Arg Ile Lys Val Ala Ile Leu Phe Gly Gly Cys Ser Glu Glu
    1               5                  10                  15
   His Asp Val Ser Leu Lys Ser Ala Ile Glu Ile Ala Ala Asn Ile Asn
               20                  25                  30
   Lys Glu Lys Tyr Glu Pro Leu Tyr Ile Gly Ile Thr Lys Ser Gly Val
           35                  40                  45
   Trp Lys Met Cys Glu Lys Pro Cys Ala Glu Trp Glu Asn Asp Asn Cys
       50                  55                  60
   Tyr Ser Ala Val Leu Ser Pro Asp Lys Lys Met His Gly Leu Leu Val
   65                  70                  75                  80
   Lys Lys Asn His Glu Tyr Glu Ile Asn His Val Asp Val Ala Phe Ser
                   85                  90                  95
   Ala Leu His Gly Lys Ser Gly Glu Asp Gly Ser Ile Gln Gly Leu Phe
               100                 105                 110
   Glu Leu Ser Gly Ile Pro Phe Val Gly Cys Asp Ile Gln Ser Ser Ala
           115                 120                 125
   Ile Cys Met Asp Lys Ser Leu Thr Tyr Ile Val Ala Lys Asn Ala Gly
       130                 135                 140
   Ile Ala Thr Pro Ala Phe Trp Val Ile Asn Lys Asp Asp Arg Pro Val
   145                 150                 155                 160
   Ala Ala Thr Phe Thr Tyr Pro Val Phe Val Lys Pro Ala Arg Ser Gly
                   165                 170                 175
   Ser Ser Phe Gly Val Lys Lys Val Asn Ser Ala Asp Glu Leu Asp Tyr
               180                 185                 190
   Ala Ile Glu Ser Ala Arg Gln Tyr Asp Ser Lys Ile Leu Ile Glu Gln
           195                 200                 205
   Ala Val Ser Gly Cys Glu Val Gly Cys Ala Val Leu Gly Asn Ser Ala
       210                 215                 220
   Ala Leu Val Val Gly Glu Val Asp Gln Ile Arg Leu Gln Tyr Gly Ile
   225                 230                 235                 240
   Phe Arg Ile His Gln Glu Val Glu Pro Glu Lys Gly Ser Glu Asn Ala
                   245                 250                 255
   Val Ile Thr Val Pro Ala Asp Leu Ser Ala Glu Glu Arg Gly Arg Ile
               260                 265                 270
   Gln Glu Thr Ala Lys Lys Ile Tyr Lys Ala Leu Gly Cys Arg Gly Leu
           275                 280                 285
   Ala Arg Val Asp Met Phe Leu Gln Asp Asn Gly Arg Ile Val Leu Asn
       290                 295                 300
   Glu Val Asn Thr Leu Pro Gly Phe Thr Ser Tyr Ser Arg Tyr Pro Arg
   305                 310                 315                 320
   Met Met Ala Ala Ala Gly Ile Ala Leu Pro Glu Leu Ile Asp Arg Leu
                   325                 330                 335
   Ile Val Leu Ala Leu Lys Gly
               340
<210> 3
   <211> 343
   <212> PRT
   <213> Unknown
<220>
   <223> peptide from a microbial resistance gene
<400> 3
   Met Asn Arg Ile Lys Val Ala Ile Leu Phe Gly Gly Cys Ser Glu Glu
    1               5                  10                  15
   His Asp Val Ser Val Lys Ser Ala Ile Glu Ile Ala Ala Asn Ile Asn
               20                  25                  30
   Lys Glu Lys Tyr Glu Pro Leu Tyr Ile Gly Ile Thr Lys Ser Gly Val
           35                  40                  45
   Trp Lys Met Cys Glu Lys Pro Cys Ala Glu Trp Glu Asn Asp Asn Cys
       50                  55                  60
   Tyr Ser Ala Val Leu Ser Pro Asp Lys Lys Met His Gly Leu Leu Val
   65                  70                  75                  80
   Lys Lys Asn His Glu Tyr Glu Ile Asn His Val Asp Val Ala Phe Ser
                   85                  90                  95
   Ala Leu His Gly Lys Ser Gly Glu Asp Gly Ser Ile Gln Gly Leu Phe
               100                 105                 110
   Glu Leu Ser Gly Ile Pro Phe Val Gly Cys Asp Ile Gln Ser Ser Ala
           115                 120                 125
   Ile Cys Met Asp Lys Ser Leu Thr Tyr Ile Val Ala Lys Asn Ala Gly
       130                 135                 140
   Ile Ala Thr Pro Ala Phe Trp Val Ile Asn Lys Asp Asp Arg Pro Val
   145                 150                 155                 160
   Ala Ala Thr Phe Thr Tyr Pro Val Phe Val Lys Pro Ala Arg Ser Gly
                   165                 170                 175
   Ser Ser Phe Gly Val Lys Lys Val Asn Ser Ala Asp Glu Leu Asp Tyr
               180                 185                 190
   Ala Ile Glu Ser Ala Arg Gln Tyr Asp Ser Lys Ile Leu Ile Glu Gln
           195                 200                 205
   Ala Val Ser Gly Cys Glu Val Gly Cys Ala Val Leu Gly Asn Ser Ala
       210                 215                 220
   Ala Leu Ala Val Gly Glu Val Asp Gln Ile Arg Leu Gln Tyr Gly Ile
   225                 230                 235                 240
   Phe Arg Ile His Gln Glu Val Glu Pro Glu Lys Gly Ser Glu Asn Ala
                   245                 250                 255
   Val Ile Thr Val Pro Ala Asp Leu Ser Ala Glu Glu Arg Gly Arg Ile
               260                 265                 270
   Gln Glu Thr Ala Lys Lys Ile Tyr Lys Ala Leu Gly Cys Arg Gly Leu
           275                 280                 285
   Ala Arg Val Asp Met Phe Leu Gln Asp Asn Gly Arg Ile Val Leu Asn
       290                 295                 300
   Glu Val Asn Thr Leu Pro Gly Phe Thr Ser Tyr Ser Arg Tyr Pro Arg
   305                 310                 315                 320
   Met Met Ala Ala Ala Gly Ile Ala Leu Pro Glu Leu Ile Asp Arg Leu
                   325                 330                 335
   Ile Val Leu Ala Leu Lys Gly
               340
<210> 4
   <211> 343
   <212> PRT
   <213> Unknown
<220>
   <223> peptide from a microbial resistance gene
<400> 4
   Met Asn Arg Ile Lys Val Ala Ile Leu Phe Gly Gly Cys Ser Glu Glu
    1               5                  10                  15
   His Asp Val Ser Val Lys Ser Ala Ile Glu Ile Ala Ala Asn Ile Asn
               20                  25                  30
   Lys Glu Lys Tyr Glu Pro Leu Tyr Ile Gly Ile Thr Lys Ser Gly Val
           35                  40                  45
   Trp Lys Met Cys Glu Lys Pro Cys Ala Glu Trp Glu Asn Asp Asn Cys
       50                  55                  60
   Tyr Ser Ala Val Leu Ser Pro Asp Lys Lys Met His Gly Leu Leu Val
   65                  70                  75                  80
   Lys Lys Asn His Glu Tyr Glu Ile Asn His Val Asp Val Ala Phe Ser
                   85                  90                  95
   Ala Leu His Gly Lys Ser Gly Glu Asp Gly Ser Ile Gln Gly Leu Phe
               100                 105                 110
   Glu Leu Ser Gly Ile Pro Phe Val Gly Cys Asp Ile Gln Ser Ser Ala
           115                 120                 125
   Ile Cys Met Asp Lys Ser Leu Thr Tyr Ile Val Ala Lys Asn Ala Gly
       130                 135                 140
   Ile Ala Thr Pro Ala Phe Trp Val Ile Asn Lys Asp Asp Arg Pro Val
   145                 150                 155                 160
   Ala Ala Thr Phe Thr Tyr Pro Val Phe Val Lys Pro Ala Arg Ser Gly
                   165                 170                 175
   Ser Ser Phe Gly Val Lys Lys Val Asn Ser Ala Asp Glu Leu Asp Tyr
               180                 185                 190
   Ala Ile Glu Ser Ala Arg Gln Tyr Asp Ser Lys Ile Leu Ile Glu Gln
           195                 200                 205
   Ala Val Ser Gly Cys Glu Val Gly Cys Ala Val Leu Gly Asn Ser Ala
       210                 215                 220
   Ala Leu Val Val Gly Glu Val Asp Gln Ile Arg Leu Gln Tyr Gly Ile
   225                 230                 235                 240
   Phe Arg Ile His Gln Glu Val Glu Pro Glu Lys Gly Ser Glu Asn Ala
                   245                 250                 255
   Val Ile Thr Val Pro Ala Asp Leu Ser Ala Glu Glu Arg Gly Arg Ile
               260                 265                 270
   Gln Glu Thr Ala Lys Lys Ile Tyr Lys Ala Leu Gly Cys Arg Gly Leu
           275                 280                 285
   Ala Arg Val Asp Met Phe Leu Gln Asp Asn Gly Arg Ile Val Leu Asn
       290                 295                 300
   Glu Val Asn Thr Leu Pro Gly Phe Thr Ser Tyr Ser Arg Tyr Pro Arg
   305                 310                 315                 320
   Met Met Ala Ala Ala Gly Ile Ala Leu Pro Glu Leu Ile Asp Arg Leu
                   325                 330                 335
   Ile Val Leu Ala Leu Lys Gly
               340
<210> 5
   <211> 9
   <212> PRT
   <213> Unknown
<220>
   <223> peptide from a microbial resistance gene
<400> 5
   Ile His Gln Glu Val Glu Pro Glu Lys
    1               5
<210> 6
   <211> 7
   <212> PRT
   <213> Unknown
<220>
   <223> peptide from a microbial resistance gene
<400> 6
   Leu Ile Val Leu Ala Leu Lys
   1               5
<210> 7
   <211> 7
   <212> PRT
   <213> Unknown
<220>
   <223> peptide from a microbial resistance gene
<400> 7
   Leu Gln Tyr Gly Ile Phe Arg
   1               5
<210> 8
   <211> 7
   <212> PRT
   <213> Unknown
<220>
   <223> peptide from a microbial resistance gene
<400> 8
   Met His Gly Leu Leu Val Lys
   1               5
<210> 9
   <211> 15
   <212> **PRT**
   <213> Unknown
<220>
   <223> peptide from a microbial resistance gene
<400> 9
   Met Met Ala Ala Ala Gly Ile Ala Leu Pro Glu Leu Ile Asp Arg
   1               5                   10                  15
<210> 10
   <211> 14
   <212> PRT
   <213> Unknown
<220>
   <223> peptide from a microbial resistance gene
<400> 10
   Asn Ala Gly Ile Ala Thr Pro Ala Phe Trp Val Ile Asn Lys
   1 5 10
<210> 11
   <211> 11
   <212> PRT
   <213> Unknown
<220>
   <223> peptide from a microbial resistance gene
<400> 11
   Ser Ala Ile Glu Ile Ala Ala Asn Ile Asn Lys
   1 5 10
<210> 12
   <211> 8
   <212> PRT
   <213> Unknown
<220>
   <223> peptide from a microbial resistance gene
<400> 12
   Ser Gly Ser Ser Phe Gly Val Lys
   1 5
<210> 13
   <211> 8
   <212> PRT
   <213> Unknown
<220>
   <223> peptide from a microbial resistance gene
<400> 13
   Ser Leu Thr Tyr Ile Val Ala Lys
   1 5
<210> 14
   <211> 10
   <212> PRT
   <213> Unknown
<220>
   <223> peptide from a microbial resistance gene
<400> 14
   Val Asp Met Phe Leu Gln Asp Asn Gly Arg
   1 5 10
<210> 15
   <211> 15
   <212> PRT
   <213> Unknown
<220>
   <223> peptide from a microbial resistance gene
<400> 15
   Val Asn Ser Ala Asp Glu Leu Asp Tyr Ala Ile Glu Ser Ala Arg
   1               5                   10                  15
<210> 16
   <211> 10
   <212> PRT
   <213> Unknown
<220>
   <223> peptide from a microbial resistance gene
<400> 16
   Tyr Glu Pro Leu Tyr Ile Gly Ile Thr Lys
   1               5                   10
<210> 17
   <211> 342
   <212> PRT
   <213> Unknown
<220>
   <223> peptide from a microbial resistance gene
<400> 17
   Met Asn Lys Ile Lys Val Ala Ile Ile Phe Gly Gly Cys Ser Glu Glu
   1               5                   10                  15
   His Asp Val Ser Val Lys Ser Ala Ile Glu Ile Ala Ala Asn Ile Asn
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Phe Ser Pro Asp Arg Lys Thr His Gly Leu Leu Val Met
   65                  70                  75                  80
   Lys Glu Arg Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Glu
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Glu Lys Gly Asp Lys Pro Glu Ala
   145                 150                 155                 160
   Arg Thr Leu Thr Tyr Pro Val Phe Val Lys Pro Ala Arg Ser Gly Ser
                   165                 170                 175
   Ser Phe Gly Val Thr Lys Val Asn Ser Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Ile Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Ala Ala Ala Ala Gly Ile Thr Leu Pro Ala Leu Ile Asp Ser Leu Ile
                   325                 330                 335
   Thr Leu Ala Ile Glu Arg
               340
<210> 18
   <211> 342
   <212> PRT
   <213> Unknown
<220>
   <223> peptide from a microbial resistance gene
<400> 18
   Met Asn Arg Ile Lys Val Ala Ile Ile Phe Gly Gly Cys Ser Glu Glu
    1               5                  10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Glu
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Met Ala Ala Ala Gly Ile Thr Leu Pro Ala Leu Ile Asp Ser Leu Ile
                   325                 330                 335
   Thr Leu Ala Leu Lys Arg
               340
<210> 19
   <211> 342
   <212> PRT
   <213> Unknown
<220>
   <223> peptide from a microbial resistance gene
<400> 19
   Met Asn Arg Ile Lys Val Ala Ile Ile Phe Gly Gly Cys Ser Glu Glu
     1               5                  10                  15
   His Asp Val Ser Val Lys Ser Ala Ile Glu Ile Ala Ala Asn Ile Asp
                20                  25                  30
   Thr Glu Lys Phe Asp Pro His Tyr Ile Gly Ile Thr Lys Asn Gly Val
            35                  40                  45
   Trp Lys Leu Cys Lys Lys Pro Cys Thr Glu Trp Glu Ala Asp Ser Leu
        50                  55                  60
   Pro Ala Ile Leu Ser Pro Asp Arg Lys Thr His Gly Leu Leu Val Met
    65                  70                  75                  80
   Lys Glu Ser Glu Tyr Glu Thr Arg Arg Ile Asp Val Ala Phe Pro Val
                    85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Ile Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Met Pro Gly Phe Thr Ser Tyr Ser Arg Tyr Pro Arg Met
   305                 310                 315                 320
   Val Ala Ala Ala Gly Ile Thr Leu Pro Ala Leu Ile Asp Ser Leu Ile
                   325                 330                 335
   Thr Leu Ala Leu Lys Arg
               340
<210> 20
   <211> 342
   <212> PRT
   <213> Unknown
<220>
   <223> peptide from a microbial resistance gene
<400> 20
   Met Asn Arg Ile Lys Val Ala Ile Ile Phe Gly Gly Cys Ser Glu Glu
   1               5                   10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Ile Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Met Pro Gly Phe Thr Ser Tyr Ser Arg Tyr Thr Arg Met
   305                 310                 315                 320
   Val Ala Ala Ala Gly Ile Thr Leu Pro Ala Leu Ile Asp Ser Leu Ile
                   325                 330                 335
   Thr Leu Ala Leu Lys Arg
               340
<210> 21
   <211> 342
   <212> PRT
   <213> Unknown
<220>
   <223> peptide from a microbial resistance gene
<400> 21
   Met Asn Arg Ile Lys Val Ala Ile Ile Phe Gly Gly Cys Ser Glu Glu
    1               5                  10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Leu Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Met Ala Ala Ala Gly Ile Thr Leu Pro Ala Leu Ile Asp Ser Leu Ile
                   325                 330                 335
   Thr Leu Ala Leu Lys Arg
               340
<210> 22
   <211> 342
   <212> PRT
   <213> Unknown
<220>
   <223> peptide from a microbial resistance gene
<400> 22
   Met Asn Arg Ile Lys Val Ala Ile Ile Phe Gly Gly Cys Ser Glu Glu
    1               5                  10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Met Ala Ala Ala Gly Ile Thr Leu Pro Ala Leu Ile Asp Ser Leu Ile
                   325                 330                 335
   Thr Leu Ala Leu Lys Arg
               340
<210> 23
   <211> 342
   <212> PRT
   <213> Unknown
<220>
   <223> peptide from a microbial resistance gene
<400> 23
   Met Asn Arg Ile Lys Val Ala Ile Ile Phe Gly Gly Cys Ser Glu Glu
   1               5                   10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Val
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Val Ala Ala Ala Gly Ile Thr Leu Pro Ala Leu Ile Asp Ser Leu Ile
                   325                 330                 335
   Thr Leu Ala Leu Lys Arg
               340
<210> 24
   <211> 344
   <212> PRT
   <213> Unknown
<220>
   <223> peptide from a microbial resistance gene
<400> 24
   Met Asn Arg Ile Lys Val Ala Ile Ile Phe Gly Gly Cys Ser Glu Glu
   1               5                   10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Gly Gly Glu Asp Gly Ala Ile Gln Gly Leu
               100                 105                 110
   Phe Val Leu Ser Gly Ile Pro Tyr Val Gly Cys Asp Ile Gln Ser Ser
           115                 120                 125
   Ala Ala Cys Met Asp Lys Ser Leu Ala Tyr Ile Leu Thr Lys Asn Ala
       130                 135                 140
   Gly Ile Ala Val Pro Glu Phe Gln Met Ile Asp Lys Gly Asp Lys Pro
   145                 150                 155                 160
   Glu Ala Gly Ala Leu Thr Tyr Pro Val Phe Val Lys Pro Ala Arg Ser
                   165                 170                 175
   Gly Ser Ser Phe Gly Val Thr Lys Val Asn Gly Thr Glu Glu Leu Asn
               180                 185                 190
   Ala Ala Ile Glu Ala Ala Gly Gln Tyr Asp Gly Lys Ile Leu Ile Glu
           195                 200                 205
   Gln Ala Ile Ser Gly Cys Glu Val Gly Cys Ala Val Met Gly Asn Glu
       210                 215                 220
   Asp Asp Leu Ile Val Gly Glu Val Asp Gln Ile Arg Leu Ser His Gly
   225                 230                 235                 240
   Ile Phe Arg Ile His Gln Glu Asn Glu Pro Glu Lys Gly Ser Glu Asn
                   245                 250                 255
   Ala Met Ile Thr Val Pro Ala Asp Ile Pro Val Glu Glu Arg Asn Arg
               260                 265                 270
   Val Gln Glu Thr Ala Lys Lys Val Tyr Arg Val Leu Gly Cys Arg Gly
           275                 280                 285
   Leu Ala Arg Val Asp Leu Phe Leu Gln Glu Asp Gly Gly Ile Val Leu
       290                 295                 300
   Asn Glu Val Asn Thr Leu Pro Gly Phe Thr Ser Tyr Ser Arg Tyr Pro
   305                 310                 315                 320
   Arg Met Met Ala Ala Ala Gly Ile Thr Leu Pro Ala Leu Ile Asp Ser
                   325                 330                 335
   Leu Ile Thr Leu Ala Leu Lys Arg
               340
<210> 25
   <211> 342
   <212> PRT
   <213> Unknown
<220>
   <223> peptide from a microbial resistance gene
<400> 25
   Met Asn Arg Ile Lys Val Ala Ile Ile Phe Gly Gly Cys Ser Glu Glu
   1               5                   10                  15
   His Asp Val Ser Val Lys Ser Ala Ile Glu Ile Ala Ala Asn Ile Asn
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Glu
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Val Ala Ala Ala Gly Ile Thr Leu Pro Ala Leu Ile Asp Ser Leu Ile
                   325                 330                 335
   Thr Leu Ala Leu Lys Arg
               340
<210> 26
   <211> 342
   <212> PRT
   <213> Unknown
<220>
   <223> peptide from a microbial resistance gene
<400> 26
   Met Asn Arg Ile Lys Val Ala Thr Ile Phe Gly Gly Cys Ser Glu Glu
    1               5                  10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ala Gly Ser
                   165                 170                 175
   Ser Phe Gly Leu Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Met Ala Ala Ala Gly Ile Thr Leu Pro Ala Leu Ile Asp Ser Leu Ile
                   325                 330                 335
   Thr Leu Ala Leu Lys Arg
               340
<210> 27
   <211> 342
   <212> PRT
   <213> Unknown
<220>
   <223> peptide from a microbial resistance gene
<400> 27
   Met Asn Arg Ile Lys Val Ala Thr Ile Phe Gly Gly Cys Ser Glu Glu
   1               5                   10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Leu Thr Lys Val Asn Gly Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Leu Pro Gly Phe Thr Ser Tyr Ser Arg Tyr Pro Arg Met
   305                 310                 315                 320
   Met Ala Ala Ala Gly Ile Thr Leu Pro Ala Met Ile Asp Ser Leu Ile
                   325                 330                 335
   Thr Leu Ala Leu Lys Arg
               340
<210> 28
   <211> 342
   <212> PRT
   <213> Unknown
<220>
   <223> peptide from a microbial resistance gene
<400> 28
   Met Asn Arg Ile Lys Val Ala Thr Ile Phe Gly Gly Cys Ser Glu Glu
    1               5                  10                  15
   His Asp Val Ser Val Lys Ser Ala Ile Glu Ile Ala Ala Asn Ile Asp
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Leu Ser Pro Asp Arg Lys Thr His Gly Leu Leu Val Met
   65                  70                  75                  80
   Lys Glu Ser Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Val
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Asp Lys Gly Asp Lys Pro Glu Ala
   145                 150                 155                 160
   Gly Ala Leu Thr Tyr Pro Val Phe Val Lys Pro Ala Arg Ser Gly Ser
                   165                 170                 175
   Ser Phe Gly Val Thr Lys Val Asn Gly Pro Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Thr Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
       290                 295                 300
   Val Asn Thr Met Pro Gly Phe Thr Ser Tyr Ser Arg Tyr Pro Arg Met
   305                 310                 315                 320
   Val Ala Ala Ala Gly Ile Thr Leu Pro Ala Leu Ile Asp Ser Leu Ile
                   325                 330                 335
   Thr Leu Ala Leu Lys Arg
               340
<210> 29
   <211> 10
   <212> PRT
   <213> Unknown
<220>
   <223> peptide from a microbial resistance gene
<400> 29
   Phe Asp Pro His Tyr Ile Gly Ile Thr Lys
    1               5                  10
<210> 30
   <211> 11
   <212> PRT
   <213> Unknown
<220>
   <223> peptide from a microbial resistance gene
<400> 30
   Ile Asp Val Ala Phe Pro Val Leu His Gly Lys
   1 5 10
<210> 31
   <211> 9
   <212> PRT
   <213> Unknown
<220>
   <223> peptide from a microbial resistance gene
<400> 31
   Ile His Gln Glu Asn Glu Pro Glu Lys
   1 5
<210> 32
   <211> 7
   <212> PRT
   <213> Unknown
<220>
   <223> peptide from a microbial resistance gene
<400> 32
   Leu Ser His Gly Ile Phe Arg
   1 5
<210> 33
   <211> 8
   <212> PRT
   <213> Unknown
<220>
   <223> peptide from a microbial resistance gene
<400> 33
   Ser Leu Ala Tyr Ile Leu Thr Lys
   1 5
<210> 34
   <211> 8
   <212> PRT
   <213> Unknown
<220>
   <223> peptide from a microbial resistance gene
<400> 34
   Thr His Gly Leu Leu Val Met Lys
   1 5
<210> 35
   <211> 6
   <212> PRT
   <213> Unknown
<220>
   <223> peptide from a microbial resistance gene
<400> 35
   Val Gln Glu Thr Ala Lys
   1 5
<210> 36
   <211> 343
   <212> PRT
   <213> Unknown
<220>
   <223> peptide from a microbial resistance gene
<400> 36
   Met Lys Lys Ile Ala Val Leu Phe Gly Gly Asn Ser Pro Glu Tyr Ser
    1               5                  10                  15
   Val Ser Leu Ala Ser Ala Ala Ser Val Ile Gln Ala Ile Asp Pro Leu
               20                  25                  30
   Lys Tyr Glu Val Met Thr Ile Gly Ile Ala Pro Thr Met Asp Trp Tyr
           35                  40                  45
   Leu Tyr Gln Gly Asn Leu Ala Asn Val Arg Asn Asp Thr Trp Leu Glu
       50                  55                  60
   Asp His Lys Asn Cys His Gln Leu Thr Phe Ser Ser Gln Gly Phe Ile
   65                  70                  75                  80
   Leu Gly Glu Lys Arg Ile Val Pro Asp Val Leu Phe Pro Val Leu His
                   85                  90                  95
   Gly Lys Tyr Gly Glu Asp Gly Cys Ile Gln Gly Leu Leu Glu Leu Met
               100                 105                 110
   Asn Leu Pro Tyr Val Gly Cys His Val Ala Ala Ser Ala Leu Cys Met
           115                 120                 125
   Asn Lys Trp Leu Leu His Gln Leu Ala Asp Thr Met Gly Ile Ala Ser
       130                 135                 140
   Ala Pro Thr Leu Leu Leu Ser Arg Tyr Glu Asn Asp Pro Ala Thr Ile
   145                 150                 155                 160
   Asp Arg Phe Ile Gln Asp His Gly Phe Pro Ile Phe Ile Lys Pro Asn
                   165                 170                 175
   Glu Ala Gly Ser Ser Lys Gly Ile Thr Lys Val Thr Asp Lys Thr Ala
               180                 185                 190
   Leu Gln Ser Ala Leu Thr Thr Ala Phe Ala Tyr Gly Ser Thr Val Leu
           195                 200                 205
   Ile Gln Lys Ala Ile Ala Gly Ile Glu Ile Gly Cys Gly Ile Leu Gly
       210                 215                 220
   Asn Glu Gln Leu Thr Ile Gly Ala Cys Asp Ala Ile Ser Leu Val Asp
   225                 230                 235                 240
   Gly Phe Phe Asp Phe Glu Glu Lys Tyr Gln Leu Ile Ser Ala Thr Ile
                   245                 250                 255
   Thr Val Pro Ala Pro Leu Pro Leu Ala Leu Glu Ser Gln Ile Lys Glu
               260                 265                 270
   Gln Ala Gln Leu Leu Tyr Arg Asn Leu Gly Leu Thr Gly Leu Ala Arg
           275                 280                 285
   Ile Asp Phe Phe Val Thr Asn Gln Gly Ala Ile Tyr Leu Asn Glu Ile
       290                 295                 300
   Asn Thr Met Pro Gly Phe Thr Gly His Ser Arg Tyr Pro Ala Met Met
   305                 310                 315                 320
   Ala Glu Val Gly Leu Ser Tyr Glu Ile Leu Val Glu Gln Leu Ile Ala
                   325                 330                 335
   Leu Ala Glu Glu Asp Lys Arg
               340
<210> 37
   <211> 343
   <212> PRT
   <213> Unknown
<220>
   <223> peptide from a microbial resistance gene
<400> 37
   Met Lys Lys Ile Ala Val Leu Phe Gly Gly Asn Ser Pro Glu Tyr Ser
   1               5                   10                  15
   Val Ser Leu Ala Ser Ala Ala Ser Val Ile Gln Ala Ile Asp Pro Leu
               20                  25                  30
   Lys Tyr Glu Val Met Thr Ile Gly Ile Ala Pro Thr Met Asp Trp Tyr
           35                  40                  45
   Trp Tyr Gln Gly Asn Leu Ala Asn Val Arg Asn Asp Thr Trp Leu Glu
       50                  55                  60
   Asp His Lys Asn Cys His Gln Leu Thr Phe Ser Ser Gln Gly Phe Ile
   65                  70                  75                  80
   Leu Gly Glu Lys Arg Ile Val Pro Asp Val Leu Phe Pro Val Leu His
                   85                  90                  95
   Gly Lys Tyr Gly Glu Asp Gly Cys Ile Gln Gly Leu Leu Glu Leu Met
               100                 105                 110
   Asn Leu Pro Tyr Val Gly Cys His Val Ala Ala Ser Ala Leu Cys Met
           115                 120                 125
   Asn Lys Trp Leu Leu His Gln Leu Ala Asp Thr Met Gly Ile Ala Ser
       130                 135                 140
   Ala Pro Thr Leu Leu Leu Ser Arg Tyr Glu Asn Asp Pro Ala Thr Ile
   145                 150                 155                 160
   Asp Arg Phe Ile Gln Asp His Gly Phe Pro Ile Phe Ile Lys Pro Asn
                   165                 170                 175
   Glu Ala Gly Ser Ser Lys Gly Ile Thr Lys Val Thr Asp Lys Thr Ala
               180                 185                 190
   Leu Gln Ser Ala Leu Thr Thr Ala Phe Ala Tyr Gly Ser Thr Val Leu
           195                 200                 205
   Ile Gln Lys Ala Ile Ala Gly Ile Glu Ile Gly Cys Gly Ile Leu Gly
       210                 215                 220
   Asn Glu Gln Leu Thr Ile Gly Ala Cys Asp Ala Ile Ser Leu Val Asp
   225                 230                 235                 240
   Gly Phe Phe Asp Phe Glu Glu Lys Tyr Gln Leu Ile Ser Ala Thr Ile
                   245                 250                 255
   Thr Val Pro Ala Pro Leu Pro Leu Ala Leu Glu Ser Gln Ile Lys Glu
               260                 265                 270
   Gln Ala Gln Leu Leu Tyr Arg Asn Leu Gly Leu Thr Gly Leu Ala Arg
           275                 280                 285
   Ile Asp Phe Phe Val Thr Asn Gln Gly Ala Ile Tyr Leu Asn Glu Ile
       290                 295                 300
   Asn Thr Met Pro Gly Phe Thr Gly His Ser Arg Tyr Pro Ala Met Met
   305                 310                 315                 320
   Ala Glu Val Gly Leu Ser Tyr Glu Ile Leu Val Glu Gln Leu Ile Ala
                   325                 330                 335
   Leu Ala Glu Glu Asp Lys Arg
               340
<210> 38
   <211> 343
   <212> PRT
   <213> Unknown
<220>
   <223> peptide from a microbial resistance gene
<400> 38
   Met Lys Lys Ile Ala Val Leu Phe Gly Gly Asn Ser Pro Glu Tyr Ser
   1 5 10 15
   Val Ser Leu Ala Ser Ala Ala Ser Val Ile Gln Ala Ile Asp Pro Leu
   20 25 30
   Lys Tyr Glu Val Met Thr Ile Gly Ile Ala Pro Thr Met Asp Trp Tyr
   35 40 45
   Trp Tyr Gln Gly Asn Leu Ala Asn Val Arg Asn Asp Thr Trp Leu Glu
   50 55 60
   Asp His Lys Asn Cys His Gln Leu Thr Phe Ser Ser Gln Gly Phe Ile
   65 70 75 80
   Leu Gly Glu Lys Arg Ile Val Pro Asp Val Leu Phe Pro Val Leu His
   85 90 95
   Gly Lys Tyr Gly Glu Asp Gly Cys Ile Gln Gly Leu Leu Glu Leu Met
   100 105 110
   Asn Leu Pro Tyr Val Gly Cys His Val Ala Ala Thr Ala Leu Cys Met
   115 120 125
   Asn Lys Trp Leu Leu His Gln Leu Ala Asp Thr Met Gly Ile Ala Ser
   130 135 140
   Ala Pro Thr Leu Leu Leu Ser Arg Tyr Glu Asn Asp Pro Ala Thr Ile
   145 150 155 160
   Asp Arg Phe Ile Gln Asp His Gly Phe Pro Ile Phe Ile Lys Pro Asn
   165 170 175
   Glu Ala Gly Ser Ser Lys Gly Ile Thr Lys Val Thr Asp Lys Thr Ala
   180 185 190
   Leu Gln Ser Ala Leu Thr Thr Ala Phe Ala Tyr Gly Ser Thr Val Leu
   195 200 205
   Ile Gln Lys Ala Ile Ala Gly Ile Glu Ile Gly Cys Gly Ile Leu Gly
   210 215 220
   Asn Glu Gln Leu Thr Ile Gly Ala Cys Asp Ala Ile Ser Leu Val Asp
   225 230 235 240
   Gly Phe Phe Asp Phe Glu Glu Lys Tyr Gln Leu Ile Ser Ala Thr Ile
   245 250 255
   Thr Val Pro Ala Pro Leu Pro Leu Ala Leu Glu Ser Gln Ile Lys Glu
   260 265 270
   Gln Ala Gln Leu Leu Tyr Arg Asn Leu Gly Leu Thr Gly Leu Ala Arg
   275 280 285
   Ile Asp Phe Phe Val Thr Asn Gln Gly Ala Ile Tyr Leu Asn Glu Ile
   290 295 300
   Asn Thr Met Pro Gly Phe Thr Gly His Ser Arg Tyr Pro Ala Met Met
   305 310 315 320
   Ala Glu Val Gly Leu Ser Tyr Glu Ile Leu Val Glu Gln Leu Ile Ala
   325 330 335
   Leu Ala Glu Glu Asp Lys Arg
   340
<210> 39
   <211> 343
   <212> PRT
   <213> Unknown
<220>
   <223> peptide from a microbial resistance gene
<400> 39
   Met Lys Lys Ile Ala Val Leu Phe Gly Gly Asn Ser Pro Glu Tyr Ser
    1               5                  10                  15
   Val Ser Leu Thr Ser Ala Ala Ser Val Ile Gln Ala Ile Asp Pro Leu
               20                  25                  30
   Lys Tyr Glu Val Met Thr Ile Gly Ile Ala Pro Thr Met Asp Trp Tyr
           35                  40                  45
   Trp Tyr Gln Gly Asn Leu Ala Asn Val Arg Asn Asp Thr Trp Leu Glu
       50                  55                  60
   Asp His Lys Asn Cys His Gln Leu Thr Phe Ser Ser Gln Gly Phe Ile
   65                  70                  75                  80
   Leu Gly Glu Lys Arg Ile Val Pro Asp Val Leu Phe Pro Val Leu His
                   85                  90                  95
   Gly Lys Tyr Gly Glu Asp Gly Cys Ile Gln Gly Leu Leu Glu Leu Met
               100                 105                 110
   Asn Leu Pro Tyr Val Gly Cys His Val Ala Ala Ser Ala Leu Cys Met
           115                 120                 125
   Asn Lys Trp Leu Leu His Gln Leu Ala Asp Thr Met Gly Ile Ala Ser
       130                 135                 140
   Ala Pro Thr Leu Leu Leu Ser Arg Tyr Glu Asn Asp Pro Ala Thr Ile
   145                 150                 155                 160
   Asp Arg Phe Ile Gln Asp His Gly Phe Pro Ile Phe Ile Lys Pro Asn
                   165                 170                 175
   Glu Ala Gly Ser Ser Lys Gly Ile Thr Lys Val Thr Asp Lys Thr Ala
               180                 185                 190
   Leu Gln Ser Ala Leu Thr Thr Ala Phe Ala Tyr Gly Ser Thr Val Leu
           195                 200                 205
   Ile Gln Lys Ala Ile Ala Gly Ile Glu Ile Gly Cys Gly Ile Leu Gly
       210                 215                 220
   Asn Glu Gln Leu Thr Ile Gly Ala Cys Asp Ala Ile Ser Leu Val Asp
   225                 230                 235                 240
   Gly Phe Phe Asp Phe Glu Glu Lys Tyr Gln Leu Ile Ser Ala Thr Ile
                   245                 250                 255
   Thr Val Pro Ala Pro Leu Pro Leu Ala Leu Glu Ser Gln Ile Lys Glu
               260                 265                 270
   Gln Ala Gln Leu Leu Tyr Arg Asn Leu Gly Leu Thr Gly Leu Ala Arg
           275                 280                 285
   Ile Asp Phe Phe Val Thr Asn Gln Gly Ala Ile Tyr Leu Asn Glu Ile
       290                 295                 300
   Asn Thr Met Pro Gly Phe Thr Gly His Ser Arg Tyr Pro Ala Met Met
   305                 310                 315                 320
   Ala Glu Val Gly Leu Ser Tyr Glu Ile Leu Val Glu Gln Leu Ile Ala
                   325                 330                 335
   Leu Ala Glu Glu Asp Lys Arg
               340
<210> 40
   <211> 343
   <212> PRT
   <213> Unknown
<220>
   <223> peptide from a microbial resistance gene
<400> 40
   Met Lys Lys Ile Ala Val Leu Phe Gly Gly Asn Ser Pro Glu Tyr Ser
   1               5                   10                  15
   Val Ser Leu Thr Ser Ala Ala Ser Val Ile Gln Ala Ile Asp Pro Leu
               20                  25                  30
   Lys Tyr Glu Val Met Thr Ile Gly Ile Ala Pro Thr Met Asp Trp Tyr
           35                  40                  45
   Trp Tyr Gln Gly Asn Leu Ala Asn Val Arg Asn Asp Thr Trp Leu Glu
       50                  55                  60
   Asp His Lys Asn Cys His Gln Leu Thr Phe Ser Ser Gln Gly Phe Ile
   65                  70                  75                  80
   Leu Gly Glu Lys Arg Ile Val Pro Asp Val Leu Phe Pro Val Leu His
                   85                  90                  95
   Gly Lys Tyr Gly Glu Asp Gly Cys Ile Gln Gly Leu Leu Glu Leu Met
               100                 105                 110
   Asn Leu Pro Tyr Val Gly Cys His Val Ala Ala Ser Ala Leu Cys Met
           115                 120                 125
   Asn Lys Trp Leu Leu His Gln Leu Ala Asp Thr Met Gly Ile Ala Ser
       130                 135                 140
   Ala Pro Thr Leu Leu Leu Ser Arg Tyr Glu Asn Asp Pro Ala Thr Ile
   145                 150                 155                 160
   Asp Arg Phe Ile Gln Asp His Gly Phe Pro Ile Phe Ile Lys Pro Asn
                   165                 170                 175
   Glu Ala Gly Ser Ser Lys Gly Ile Thr Lys Val Thr Asp Lys Thr Ala
               180                 185                 190
   Leu Gln Ser Ala Leu Thr Thr Ala Phe Ala Tyr Gly Ser Thr Val Leu
           195                 200                 205
   Ile Gln Lys Ala Ile Ala Gly Ile Glu Ile Gly Cys Gly Ile Leu Gly
       210                 215                 220
   Asn Glu Gln Leu Thr Ile Gly Ala Cys Asp Ala Ile Ser Leu Val Asp
   225                 230                 235                 240
   Gly Phe Phe Asp Phe Glu Glu Lys Tyr Gln Leu Ile Ser Ala Thr Ile
                   245                 250                 255
   Thr Val Pro Ala Pro Leu Pro Leu Ala Leu Glu Ser Gln Ile Lys Glu
               260                 265                 270
   Gln Ala Gln Leu Leu Tyr Arg Asn Leu Gly Leu Thr Gly Leu Ala Arg
           275                 280                 285
   Ile Asp Phe Phe Val Thr Asn Gln Gly Ala Ile Tyr Leu Asn Glu Ile
       290                 295                 300
   Asn Thr Met Pro Gly Phe Thr Gly His Ser Arg Tyr Pro Ala Met Met
   305                 310                 315                 320
   Ala Glu Val Gly Leu Ser Tyr Glu Ile Leu Val Glu Lys Leu Ile Ala
                   325                 330                 335
   Leu Ala Glu Glu Asp Lys Arg
               340
<210> 41
   <211> 343
   <212> PRT
   <213> Unknown
<220>
   <223> peptide from a microbial resistance gene
<400> 41
   Met Lys Lys Ile Ala Val Leu Phe Gly Gly Asn Ser Pro Glu Tyr Ser
    1               5                  10                  15
   Val Ser Leu Thr Ser Ala Glu Ser Val Ile Gln Ala Ile Asn Pro Leu
               20                  25                  30
   Lys Tyr Glu Val Met Thr Ile Gly Ile Ala Pro Thr Met Asp Trp Tyr
           35                  40                  45
   Trp Tyr Gln Gly Asn Leu Ala Asn Val Arg Asn Asp Thr Trp Leu Glu
       50                  55                  60
   Asp His Lys Asn Cys His Gln Leu Thr Phe Ser Ser Gln Gly Phe Ile
   65                  70                  75                  80
   Leu Gly Glu Lys Arg Ile Val Pro Asp Val Leu Phe Pro Val Leu His
                   85                  90                  95
   Gly Lys Tyr Gly Glu Asp Gly Cys Ile Gln Gly Leu Leu Glu Leu Met
               100                 105                 110
   Asn Leu Pro Tyr Val Gly Cys His Val Ala Ala Ser Ala Leu Cys Met
           115                 120                 125
   Asn Lys Trp Leu Leu His Gln Leu Ala Asp Thr Met Gly Ile Ala Ser
       130                 135                 140
   Ala Pro Thr Leu Leu Leu Ser Arg Tyr Glu Asn Asp Pro Ala Thr Ile
   145                 150                 155                 160
   Asp Arg Phe Ile Gln Asp His Gly Phe Pro Ile Phe Ile Lys Pro Asn
                   165                 170                 175
   Glu Ala Gly Ser Ser Lys Gly Ile Thr Lys Val Thr Asp Lys Thr Ala
               180                 185                 190
   Leu Gln Ser Ala Leu Thr Thr Ala Phe Ala Tyr Gly Ser Thr Val Leu
           195                 200                 205
   Ile Gln Lys Ala Ile Ala Gly Ile Glu Ile Gly Cys Gly Ile Leu Gly
       210                 215                 220
   Asn Glu Gln Leu Thr Ile Gly Ala Cys Asp Ala Ile Ser Leu Val Asp
   225                 230                 235                 240
   Gly Phe Phe Asp Phe Glu Glu Lys Tyr Gln Leu Ile Ser Ala Thr Ile
                   245                 250                 255
   Thr Val Pro Ala Pro Leu Pro Leu Ala Leu Glu Ser Gln Ile Lys Glu
               260                 265                 270
   Gln Ala Gln Leu Leu Tyr Arg Asn Leu Gly Leu Thr Gly Leu Ala Arg
           275                 280                 285
   Ile Asp Phe Phe Val Thr Asn Gln Gly Ala Ile Tyr Leu Asn Glu Ile
       290                 295                 300
   Asn Thr Met Pro Gly Phe Thr Gly His Ser Arg Tyr Pro Ala Met Met
   305                 310                 315                 320
   Ala Glu Val Gly Leu Ser Tyr Glu Ile Leu Val Glu Gln Leu Ile Ala
                   325                 330                 335
   Leu Ala Glu Glu Asp Lys Arg
               340
<210> 42
   <211> 344
   <212> PRT
   <213> Unknown
<220>
   <223> peptide from a microbial resistance gene
<400> 42
   Met Met Lys Lys Ile Ala Val Leu Phe Gly Gly Asn Ser Pro Glu Tyr
    1               5                  10                  15
   Ser Val Ser Leu Ala Ser Ala Ala Ser Val Ile Gln Ala Ile Asp Pro
               20                  25                  30
   Leu Lys Tyr Glu Val Met Thr Ile Gly Ile Ala Pro Thr Met Asp Trp
               35                  40                  45
   Tyr Trp Tyr Gln Gly Asn Leu Ala Asn Val Arg Asn Asp Thr Trp Leu
           50                  55                  60
   Glu Asp His Lys Asn Cys His Gln Leu Thr Phe Ser Ser Gln Gly Phe
   65                  70                  75                  80
   Ile Leu Gly Glu Lys Arg Ile Val Pro Asp Val Leu Phe Pro Val Leu
                   85                  90                  95
   His Gly Lys Tyr Gly Glu Asp Gly Cys Ile Gln Gly Leu Leu Glu Leu
               100                 105                 110
   Met Asn Leu Pro Tyr Val Gly Cys His Val Ala Ala Ser Ala Leu Cys
           115                 120                 125
   Met Asn Lys Trp Leu Leu His Gln Leu Ala Asp Thr Met Gly Ile Ala
       130                 135                 140
   Ser Ala Pro Thr Leu Leu Leu Ser Arg Tyr Glu Asn Asp Pro Ala Thr
   145                 150                 155                 160
   Ile Asp Arg Phe Ile Gln Asp His Gly Phe Pro Ile Phe Ile Lys Pro
                   165                 170                 175
   Asn Glu Ala Gly Ser Ser Lys Gly Ile Thr Lys Val Thr Asp Lys Thr
               180                 185                 190
   Ala Leu Gln Ser Ala Leu Thr Thr Ala Phe Ala Tyr Gly Ser Thr Val
           195                 200                 205
   Leu Ile Gln Lys Ala Ile Ala Gly Ile Glu Ile Gly Cys Gly Ile Leu
       210                 215                 220
   Gly Asn Glu Gln Leu Thr Ile Gly Ala Cys Asp Ala Ile Ser Leu Val
   225                 230                 235                 240
   Asp Gly Phe Phe Asp Phe Glu Glu Lys Tyr Gln Leu Ile Ser Ala Thr
                   245                 250                 255
   Ile Thr Val Pro Ala Pro Leu Pro Leu Ala Leu Glu Ser Gln Ile Lys
               260                 265                 270
   Glu Gln Ala Gln Leu Leu Tyr Arg Asn Leu Gly Leu Thr Gly Leu Ala
           275                 280                 285
   Arg Ile Asp Phe Phe Val Thr Asn Gln Gly Ala Ile Tyr Leu Asn Glu
       290                 295                 300
   Ile Asn Thr Met Pro Gly Phe Thr Gly His Ser Arg Tyr Pro Ala Met
   305                 310                 315                 320
   Met Ala Glu Val Gly Leu Ser Tyr Glu Ile Leu Val Glu Gln Leu Ile
                   325                 330                 335
   Ala Leu Ala Glu Glu Asp Lys Arg
               340
<210> 43
   <211> 344
   <212> PRT
   <213> Unknown
<220>
   <223> peptide from a microbial resistance gene
<400> 43
   Met Met Lys Lys Ile Ala Val Leu Phe Gly Gly Asn Ser Pro Glu Tyr
    1               5                  10                  15
   Ser Val Ser Leu Thr Ser Ala Ala Ser Val Ile Gln Ala Ile Asp Pro
               20                  25                  30
   Leu Lys Tyr Glu Val Met Thr Ile Gly Ile Ala Pro Thr Met Asp Trp
           35                  40                  45
   Tyr Trp Tyr Gln Gly Asn Leu Ala Asn Val Arg Asn Asp Thr Trp Leu
       50                  55                  60
   Glu Asp His Lys Asn Cys His Gln Leu Thr Phe Ser Ser Gln Gly Phe
   65                  70                  75                  80
   Ile Leu Gly Glu Lys Arg Ile Val Pro Asp Val Leu Phe Pro Val Leu
                   85                  90                  95
   His Gly Lys Tyr Gly Glu Asp Gly Cys Ile Gln Gly Leu Leu Glu Leu
               100                 105                 110
   Met Asn Leu Pro Tyr Val Gly Cys His Val Ala Ala Ser Ala Leu Cys
           115                 120                 125
   Met Asn Lys Trp Leu Leu His Gln Leu Ala Asp Thr Met Gly Ile Ala
       130                 135                 140
   Ser Ala Pro Thr Leu Leu Leu Ser Arg Tyr Glu Asn Asp Pro Ala Thr
   145                 150                 155                 160
   Ile Asp Arg Phe Ile Gln Asp His Gly Phe Pro Ile Phe Ile Lys Pro
                   165                 170                 175
   Asn Glu Ala Gly Ser Ser Lys Gly Ile Thr Lys Val Thr Asp Lys Thr
               180                 185                 190
   Ala Leu Gln Ser Ala Leu Thr Thr Ala Phe Ala Tyr Gly Ser Thr Val
           195                 200                 205
   Leu Ile Gln Lys Ala Ile Ala Gly Ile Glu Ile Gly Cys Gly Ile Leu
       210                 215                 220
   Gly Asn Glu Gln Leu Thr Ile Gly Ala Cys Asp Ala Ile Ser Leu Val
   225                 230                 235                 240
   Asp Gly Phe Phe Asp Phe Glu Glu Lys Tyr Gln Leu Ile Ser Ala Thr
                   245                 250                 255
   Ile Thr Val Pro Ala Pro Leu Pro Leu Ala Leu Glu Ser Gln Ile Lys
               260                 265                 270
   Glu Gln Ala Gln Leu Leu Tyr Arg Asn Leu Gly Leu Thr Gly Leu Ala
           275                 280                 285
   Arg Ile Asp Phe Phe Val Thr Asn Gln Gly Ala Ile Tyr Leu Asn Glu
       290                 295                 300
   Ile Asn Thr Met Pro Gly Phe Thr Gly His Ser Arg Tyr Pro Ala Met
   305                 310                 315                 320
   Met Ala Glu Val Gly Leu Ser Tyr Glu Ile Leu Val Glu Gln Leu Ile
                   325                 330                 335
   Ala Leu Ala Glu Glu Asp Lys Arg
               340
<210> 44
   <211> 350
   <212> PRT
   <213> Unknown
<220>
   <223> peptide from a microbial resistance gene
<400> 44
   Met Lys Lys Ile Ala Ile Ile Phe Gly Gly Asn Ser Pro Glu Tyr Ala
   1               5                   10                  15
   Val Ser Leu Ala Ser Ala Thr Ser Ala Ile Glu Ala Leu Gln Ser Ser
               20                  25                  30
   Pro Asp Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Ala Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Ala Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Asp Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr His Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Ile Gly Leu Ser Tyr Gln Glu
                   325                 330                 335
   Leu Leu Gln Lys Leu Leu Val Leu Ala Lys Glu Glu Val Lys
               340                 345                 350
<210> 45
   <211> 350
   <212> PRT
   <213> Unknown
<220>
   <223> peptide from a microbial resistance gene
<400> 45
   Met Lys Lys Ile Ala Ile Ile Phe Gly Gly Asn Ser Pro Glu Tyr Ala
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Ala Ser Ala Leu Cys Met Asn Lys Trp Leu Leu His Gln Ala Ala
       130                 135                 140
   Glu Ala Ile Gly Val Gln Ser Ala Pro Thr Ile Leu Leu Thr Asn Gln
   145                 150                 155                 160
   Asp Asn Gln Gln Arg Gln Ile Glu Ala Phe Ile Gln Thr His Asp Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Pro Ala Leu Lys Glu Ala Phe
           195                 200                 205
   Ala Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Glu Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr His Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Ile Gly Leu Ser Tyr Gln Glu
                   325                 330                 335
   Leu Leu Gln Lys Leu Leu Val Leu Ala Lys Glu Glu Gly Lys
               340                 345                 350
<210> 46
   <211> 350
   <212> PRT
   <213> Unknown
<220>
   <223> peptide from a microbial resistance gene
<400> 46
   Met Lys Lys Ile Ala Ile Ile Phe Gly Gly Asn Ser Pro Glu Tyr Ala
   1               5                   10                  15
   Val Ser Leu Ala Ser Ala Thr Ser Ala Leu Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 47
   <211> 350
   <212> PRT
   <213> Unknown
<220>
   <223> peptide from a microbial resistance gene
<400> 47
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 48
   <211> 350
   <212> PRT
   <213> Unknown
<220>
   <223> peptide from a microbial resistance gene
<400> 48
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Glu Arg Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 49
   <211> 350
   <212> PRT
   <213> Unknown
<220>
   <223> peptide from a microbial resistance gene
<400> 49
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
               35                  40                  45
   Asp Trp Tyr Leu Tyr Thr Gly Glu Leu Glu Asn Ile Arg Gln Asp Thr
           50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Val Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 50
   <211> 350
   <212> PRT
   <213> Unknown
<220>
   <223> peptide from a microbial resistance gene
<400> 50
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Ser Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Val Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Glu Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Ile Leu Ala Lys Glu Glu Val Lys
               340                 345                 350
<210> 51
   <211> 350
   <212> PRT
   <213> Unknown
<220>
   <223> peptide from a microbial resistance gene
<400> 51
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Thr Gln Lys Ile Lys Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 52
   <211> 350
   <212> PRT
   <213> Unknown
<220>
   <223> peptide from a microbial resistance gene
<400> 52
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Thr Gln Lys Ile Gln Pro Leu Phe Glu
   65                  70                  75                  80
   Gly Asn Gly Phe Trp Ile Ser Glu Ala Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Met Phe Glu Leu Met Lys Leu Pro Tyr Val Gly Cys Gly Val
           115                 120                 125
   Ala Ala Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Arg Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Pro Ala Leu Lys Glu Ala Phe
           195                 200                 205
   Ala Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr His Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Gly Lys
               340                 345                 350
<210> 53
   <211> 350
   <212> PRT
   <213> Unknown
<220>
   <223> peptide from a microbial resistance gene
<400> 53
   Met Lys Lys Ile Ala Ile Ile Phe Gly Gly Asn Ser Pro Glu Tyr Thr
     1               5                  10                  15
   Val Ser Leu Ala Ser Ala Thr Ser Ala Ile Glu Ala Leu Gln Ser Ser
                20                  25                  30
   Pro Tyr Asp Tyr Asp Leu Ser Leu Ile Gly Ile Ala Pro Asp Ala Met
            35                  40                  45
   Asp Trp Tyr Leu Tyr Thr Gly Glu Leu Glu Asn Ile Arg Gln Asp Thr
        50                  55                  60
   Trp Leu Leu Asp Thr Lys Gln Lys Gln Lys Ile Gln Pro Leu Phe Glu
    65                  70                  75                  80
   Gly Asn Gly Phe Trp Leu Ser Glu Glu Gln Gln Thr Leu Val Pro Asp
                    85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn His
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                       325                 330                 335
   Leu Leu Gln Lys Leu Leu Val Leu Ala Lys Glu Glu Val Lys
               340                 345                 350
<210> 54
   <211> 350
   <212> PRT
   <213> Unknown
<220>
   <223> peptide from a microbial resistance gene
<400> 54
   Met Lys Lys Ile Ala Ile Ile Phe Gly Gly Asn Ser Pro Glu Tyr Thr
   1               5                   10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys Gln Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Ser Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Val Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Glu Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Ile Leu Ala Lys Glu Glu Val Lys
               340                 345                 350
<210> 55
   <211> 350
   <212> PRT
   <213> Unknown
<220>
   <223> peptide from a microbial resistance gene
<400> 55
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys Gln Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Met Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn His
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 56
   <211> 350
   <212> PRT
   <213> Unknown
<220>
   <223> peptide from a microbial resistance gene
<400> 56
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Thr Pro Asp Ala Met
                   35                  40                  45
   Asp Trp Tyr Leu Tyr Thr Gly Glu Leu Glu Asn Ile Arg Gln Asp Thr
           50                  55                  60
   Trp Leu Leu Asp Thr Lys His Lys Gln Lys Ile Gln Pro Leu Phe Glu
    65                  70                  75                  80
   Gly Asn Gly Phe Trp Leu Ser Glu Glu Gln Gln Thr Leu Val Pro Asp
                    85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 57
   <211> 350
   <212> PRT
   <213> Unknown
<220>
   <223> peptide from a microbial resistance gene
<400> 57
   Met Lys Lys Ile Ala Ile Ile Phe Gly Gly Asn Ser Pro Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Thr Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Lys Gln Lys Ile Gln Pro Leu Phe Glu
   65                  70                  75                  80
   Gly Asn Gly Phe Trp Leu Ser Glu Glu Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Gly Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Ala Asn Gln Gln Glu Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Ser Ala Leu Lys Glu Ala Phe
           195                 200                 205
   Thr Tyr Cys Ser Ala Val Leu Leu Gln Lys Asn Ile Val Gly Val Glu
       210                 215                 220
   Ile Gly Cys Gly Ile Leu Gly Asn Asp Ser Leu Thr Val Gly Ala Cys
   225                 230                 235                 240
   Asp Thr Ile Ser Leu Val Asp Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr Arg Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Val Gly Leu Ser Tyr Gln Glu
                   325                 330                 335
   Leu Leu Gln Lys Leu Leu Val Leu Ala Lys Glu Glu Val Lys
               340                 345                 350
<210> 58
   <211> 350
   <212> PRT
   <213> Unknown
<220>
   <223> peptide from a microbial resistance gene
<400> 58
   Met Lys Lys Ile Ala Ile Ile Phe Gly Gly Asn Ser Ser Glu Tyr Thr
    1               5                  10                  15
   Val Ser Leu Ala Ser Ala Thr Ser Ala Ile Glu Ala Leu Gln Ser Ser
               20                  25                  30
   Pro Tyr Asp Tyr Asp Leu Ser Leu Ile Gly Ile Ala Pro Asp Ala Met
           35                  40                  45
   Asp Trp Tyr Leu Tyr Thr Gly Glu Leu Glu Asn Ile Arg Gln Asp Thr
       50                  55                  60
   Trp Leu Leu Asp Thr Lys His Thr Gln Lys Ile Gln Pro Leu Phe Glu
   65                  70                  75                  80
   Glu Asn Gly Phe Trp Leu Ser Glu Ala Gln Gln Thr Leu Val Pro Asp
                   85                  90                  95
   Val Leu Phe Pro Ile Met His Gly Lys Tyr Gly Glu Asp Gly Ser Ile
               100                 105                 110
   Gln Gly Leu Phe Glu Leu Met Lys Leu Pro Tyr Val Gly Cys Gly Val
           115                 120                 125
   Ala Ala Ser Ala Leu Cys Met Asn Lys Trp Leu Leu His Gln Ala Ala
       130                 135                 140
   Ala Ala Ile Gly Val Gln Ser Ala Pro Thr Ile Leu Leu Thr Asn Gln
   145                 150                 155                 160
   Asp Asn Gln Gln Gln Gln Ile Glu Ala Phe Ile Gln Thr His Gly Phe
                   165                 170                 175
   Pro Val Phe Phe Lys Pro Asn Glu Ala Gly Ser Ser Lys Gly Ile Thr
               180                 185                 190
   Lys Val Thr Cys Val Glu Glu Ile Ala Pro Ala Leu Lys Glu Ala Phe
           195                 200                 205
   Ala Tyr Cys Ser Ala Val Leu Leu Gln Lys Asn Ile Ala Gly Val Glu
       210                 215                 220
   Ile Gly Cys Gly Ile Leu Gly Asn Asp Ser Leu Thr Val Gly Ala Cys
   225                 230                 235                 240
   Asp Ala Ile Ser Leu Val Glu Gly Phe Phe Asp Phe Glu Glu Lys Tyr
                   245                 250                 255
   Gln Leu Ile Ser Ala Lys Ile Thr Val Pro Ala Pro Leu Pro Glu Thr
               260                 265                 270
   Ile Glu Thr Lys Val Lys Glu Gln Ala Gln Leu Leu Tyr His Ser Leu
           275                 280                 285
   Gly Leu Lys Gly Leu Ala Arg Ile Asp Phe Phe Val Thr Asp Gln Gly
       290                 295                 300
   Glu Leu Tyr Leu Asn Glu Ile Asn Thr Met Pro Gly Phe Thr Ser His
   305                 310                 315                 320
   Ser Arg Tyr Pro Ala Met Met Ala Ala Ile Gly Leu Ser Tyr Gln Glu
                   325                 330                 335
   Leu Leu Gln Lys Leu Leu Val Leu Ala Lys Glu Glu Gly Lys
               340                 345                 350
<210> 59
   <211> 8
   <212> PRT
   <213> Unknown
<220>
   <223> peptide from a microbial resistance gene
<400> 59
   Glu Gln Ala Gln Leu Leu Tyr Arg
    1               5
<210> 60
   <211> 20
   <212> PRT
   <213> Unknown
<220>
   <223> peptide from a microbial resistance gene
<400> 60
   Phe Ile Gln Asp His Gly Phe Pro Ile Phe Ile Lys Pro Asn Glu Ala
   1 5 10 15
   Gly Ser Ser Lys
   20
<210> 61
   <211> 13
   <212> PRT
   <213> Unknown
<220>
   <223> peptide from a microbial resistance gene
<400> 61
   Ile Val Pro Asp Val Leu Phe Pro Val Leu His Gly Lys
   1 5 10
<210> 62
   <211> 17
   <212> PRT
   <213> Unknown
<220>
   <223> peptide from a microbial resistance gene
<400> 62
   Asn Cys His Gln Leu Thr Phe Ser Ser Gln Gly Phe Ile Leu Gly Glu
   1 5 10 15
   Lys
<210> 63
   <211> 9
   <212> PRT
   <213> Unknown
<220>
   <223> peptide from a microbial resistance gene
<400> 63
   Asn Asp Thr Trp Leu Glu Asp His Lys
   1 5
<210> 64
   <211> 9
   <212> PRT
   <213> Unknown
<220>
   <223> peptide from a microbial resistance gene
<400> 64
   Asn Leu Gly Leu Thr Gly Leu Ala Arg
   1               5
<210> 65
   <211> 21
   <212> PRT
   <213> Unknown
<220>
   <223> peptide from a microbial resistance gene
<400> 65
   Thr Ala Leu Gln Ser Ala Leu Thr Thr Ala Phe Ala Tyr Gly Ser Thr
   1               5                   10                  15
   Val Leu Ile Gln Lys
               20
<210> 66
   <211> 22
   <212> PRT
   <213> Unknown
<220>
   <223> peptide from a microbial resistance gene
<400> 66
   Trp Leu Leu His Gln Leu Ala Asp Thr Met Gly Ile Ala Ser Ala Pro
   1               5                   10                  15
   Thr Leu Leu Leu Ser Arg
               20
<210> 67
   <211> 10
   <212> PRT
   <213> Unknown
<220>
   <223> peptide from a microbial resistance gene
<400> 67
   Tyr Glu Asn Asp Pro Ala Thr Ile Asp Arg
   1               5                   10
<210> 68
   <211> 23
   <212> PRT
   <213> Unknown
<220>
   <223> peptide from a microbial resistance gene
<400> 68
   Tyr Gln Leu Ile Ser Ala Thr Ile Thr Val Pro Ala Pro Leu Pro Leu
    1               5                  10                  15
   Ala Leu Glu Ser Gln Ile Lys
                20
<210> 69
   <211> 14
   <212> PRT
   <213> Unknown
<220>
   <223> peptide from a microbial resistance gene
<400> 69
   Ile Thr Val Pro Ala Pro Leu Pro Glu Thr Ile Glu Thr Lys
    1               5                  10
<210> 70
   <211> 9
   <212> PRT
   <213> Unknown
<220>
   <223> peptide from a microbial resistance gene
<400> 70
   Gln Asp Thr Trp Leu Leu Asp Thr Lys
    1               5
<210> 71
   <211> 7
   <212> PRT
   <213> Unknown
<220>
   <223> peptide from a microbial resistance gene
<400> 71
   Tyr Gln Leu Ile Ser Ala Lys
    1               5
<210> 72
   <211> 343
   <212> PRT
   <213> Unknown
<220>
   <223> peptide from a microbial resistance gene
<400> 72
   Met Phe Lys Ile Lys Val Ala Val Leu Phe Gly Gly Cys Ser Glu Glu
    1               5                   10                  15
   His Asn Val Ser Ile Lys Ser Ala Met Glu Ile Ala Ala Asn Ile Asp
               20                  25                  30
   Thr Lys Lys Tyr Gln Pro Tyr Tyr Ile Gly Ile Thr Lys Ser Gly Val
           35                  40                  45
   Trp Lys Met Cys Glu Lys Pro Cys Leu Glu Trp Glu Gln Tyr Ala Gly
       50                  55                  60
   Asp Pro Val Val Phe Ser Pro Asp Arg Ser Thr His Gly Leu Leu Ile
   65                  70                  75                  80
   Gln Lys Asp Lys Gly Tyr Glu Ile Gln Pro Val Asp Val Val Leu Pro
                   85                  90                  95
   Met Ile His Gly Lys Phe Gly Glu Asp Gly Ser Ile Gln Gly Leu Leu
               100                 105                 110
   Glu Leu Ser Gly Ile Pro Tyr Val Gly Cys Asp Ile Gln Ser Ser Val
           115                 120                 125
   Thr Cys Met Asp Lys Ala Leu Ala Tyr Thr Val Val Lys Asn Ala Gly
       130                 135                 140
   Ile Ala Val Pro Gly Phe Arg Ile Leu Gln Glu Gly Asp Arg Leu Glu
   145                 150                 155                 160
   Thr Glu Asp Phe Val Tyr Pro Val Phe Val Lys Pro Ala Arg Ser Gly
                   165                 170                 175
   Ser Ser Phe Gly Val Asn Lys Val Cys Lys Ala Glu Glu Leu Gln Ala
               180                 185                 190
   Ala Ile Glu Glu Ala Arg Lys Tyr Asp Ser Lys Ile Leu Ile Glu Glu
           195                 200                 205
   Ala Val Thr Gly Ser Glu Val Gly Cys Ala Ile Leu Gly Asn Gly Asn
       210                 215                 220
   Asp Leu Met Ala Gly Glu Val Asp Gln Ile Glu Leu Arg His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Ala Leu Pro Asp Glu Val Arg Glu Arg Ile
               260                 265                 270
   Gln Lys Thr Ala Met Lys Ile Tyr Arg Ile Leu Gly Cys Arg Gly Leu
           275                 280                 285
   Ala Arg Ile Asp Leu Phe Leu Arg Glu Asp Gly Cys Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Thr Glu Ile Leu Asp Arg Leu
                   325                 330                 335
   Ile Glu Leu Ser Leu Arg Arg
               340
<210> 73
   <211> 343
   <212> PRT
   <213> Unknown
<220>
   <223> peptide from a microbial resistance gene
<400> 73
   Met Phe Lys Ile Lys Val Ala Val Leu Phe Gly Gly Cys Ser Glu Glu
   1               5                   10                  15
   His Asn Val Ser Ile Lys Ser Ala Met Glu Ile Ala Ala Asn Ile Asp
               20                  25                  30
   Thr Lys Lys Tyr Gln Pro Tyr Tyr Ile Gly Ile Thr Lys Ser Gly Val
           35                  40                  45
   Trp Lys Met Cys Glu Lys Pro Cys Leu Gly Trp Glu Gln Tyr Ala Gly
       50                  55                  60
   Asp Pro Val Val Phe Ser Pro Asp Arg Ser Thr His Gly Leu Leu Ile
   65                  70                  75                  80
   Gln Lys Asp Thr Gly Tyr Glu Ile Gln Pro Val Asp Val Val Phe Pro
                   85                  90                  95
   Met Ile His Gly Lys Phe Gly Glu Asp Gly Ser Ile Gln Gly Leu Leu
               100                 105                 110
   Glu Leu Ser Gly Ile Pro Tyr Val Gly Cys Asp Ile Gln Ser Ser Val
           115                 120                 125
   Ile Cys Met Asp Lys Ala Leu Ala Tyr Thr Val Val Lys Asn Ala Gly
       130                 135                 140
   Ile Ala Val Pro Gly Phe Arg Ile Leu Gln Glu Gly Asp Arg Leu Glu
   145                 150                 155                 160
   Thr Glu Asp Leu Val Tyr Pro Val Phe Val Lys Pro Ala Arg Ser Gly
                   165                 170                 175
   Ser Ser Phe Gly Val Asn Lys Val Cys Lys Ala Glu Glu Leu Gln Ala
               180                 185                 190
   Ala Ile Arg Glu Ala Arg Lys Tyr Asp Ser Lys Ile Leu Ile Glu Glu
           195                 200                 205
   Ala Val Thr Gly Ser Glu Val Gly Cys Ala Ile Leu Gly Asn Glu Asn
       210                 215                 220
   Asp Leu Met Ala Gly Glu Val Asp Gln Ile Glu Leu Arg His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Ala Leu Pro Asp Glu Val Arg Glu Arg Ile
               260                 265                 270
   Arg Lys Thr Ala Met Lys Ile Tyr Arg Ile Leu Gly Cys Arg Gly Leu
           275                 280                 285
   Ala Arg Ile Asp Leu Phe Leu Arg Glu Asp Gly Cys Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Ser Glu Ile Leu Asp Arg Leu
                   325                 330                 335
   Ile Glu Phe Ser Leu Arg Arg
               340
<210> 74
   <211> 343
   <212> PRT
   <213> Unknown
<220>
   <223> peptide from a microbial resistance gene
<400> 74
   Met Phe Arg Ile Lys Val Ala Val Leu Phe Gly Gly Cys Ser Glu Glu
   1               5                   10                  15
   His Asn Val Ser Ile Lys Ser Ala Met Glu Ile Ala Ala Asn Ile Asp
               20                  25                  30
   Thr Lys Lys Tyr Gln Pro Tyr Tyr Ile Gly Ile Thr Lys Ser Gly Val
           35                  40                  45
   Trp Lys Met Cys Glu Lys Pro Cys Leu Glu Trp Glu Gln Tyr Ala Gly
       50                  55                  60
   Asp Pro Val Val Phe Ser Pro Asp Arg Ser Thr His Gly Leu Leu Ile
   65                  70                  75                  80
   Gln Lys Asp Lys Gly Tyr Glu Ile Gln Pro Val Asp Val Val Phe Pro
                   85                  90                  95
   Met Ile His Gly Lys Phe Gly Glu Asp Gly Ser Ile Gln Gly Leu Leu
               100                 105                 110
   Glu Leu Ser Gly Ile Pro Tyr Val Gly Cys Asp Ile Gln Ser Ser Val
           115                 120                 125
   Ile Cys Met Asp Lys Ala Leu Ala Tyr Thr Val Val Lys Asn Ala Gly
       130                 135                 140
   Ile Thr Val Pro Gly Phe Arg Ile Leu Gln Glu Gly Asp Arg Leu Glu
   145                 150                 155                 160
   Thr Glu Asp Phe Val Tyr Pro Val Phe Val Lys Pro Ala Arg Ser Gly
                   165                 170                 175
   Ser Ser Phe Gly Val Asn Lys Val Cys Lys Ala Glu Glu Leu Gln Ala
               180                 185                 190
   Ala Ile Glu Glu Ala Arg Lys Tyr Asp Ser Lys Ile Leu Ile Glu Glu
           195                 200                 205
   Ala Val Thr Gly Ser Glu Val Gly Cys Ala Ile Leu Gly Asn Gly Asn
       210                 215                 220
   Asp Leu Met Ala Gly Glu Val Asp Gln Ile Glu Leu Arg His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Ala Leu Pro Asp Glu Val Arg Glu Gln Ile
               260                 265                 270
   Gln Glu Thr Ala Met Lys Ile Tyr Arg Ile Leu Gly Cys Arg Gly Leu
           275                 280                 285
   Ala Arg Ile Asp Leu Phe Leu Arg Glu Asp Gly Cys Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Ser Glu Ile Leu Asp Arg Leu
                   325                 330                 335
   Ile Glu Leu Ser Leu Arg Arg
               340
<210> 75
   <211> 343
   <212> PRT
   <213> Unknown
<220>
   <223> peptide from a microbial resistance gene
<400> 75
   Met Phe Arg Ile Lys Val Ala Val Leu Phe Gly Gly Cys Ser Glu Glu
   1 5 10 15
   His Asn Val Ser Ile Lys Ser Ala Met Glu Ile Ala Ala Asn Ile Asp
   20 25 30
   Thr Lys Lys Tyr Gln Pro Tyr Tyr Ile Gly Ile Thr Lys Ser Gly Val
   35 40 45
   Trp Lys Met Cys Glu Lys Pro Cys Leu Glu Trp Glu Gln Tyr Ala Gly
   50 55 60
   Asp Pro Val Val Phe Ser Pro Asp Arg Ser Thr His Gly Leu Leu Ile
   65 70 75 80
   Gln Lys Asp Thr Gly Tyr Glu Ile Gln Pro Val Asp Val Gly Leu Pro
   85 90 95
   Met Ile His Gly Lys Phe Gly Glu Asp Gly Ser Ile Gln Gly Leu Leu
   100 105 110
   Glu Leu Ser Gly Ile Pro Tyr Val Gly Cys Asp Ile Gln Ser Ser Val
   115 120 125
   Thr Cys Met Asp Lys Ala Leu Ala Tyr Thr Val Val Lys Asn Ala Gly
   130 135 140
   Ile Ala Val Pro Gly Phe Arg Ile Leu Gln Glu Gly Asp Arg Leu Glu
   145 150 155 160
   Thr Glu Asp Phe Val Tyr Pro Val Phe Val Lys Pro Ala Arg Ser Gly
   165 170 175
   Ser Ser Phe Gly Val Asn Lys Val Cys Lys Ala Glu Glu Leu Gln Ala
   180 185 190
   Ala Ile Glu Asp Ala Arg Lys Tyr Asp Ser Lys Ile Leu Ile Glu Glu
   195 200 205
   Ala Val Thr Gly Ser Glu Val Gly Cys Ala Ile Leu Gly Asn Gly Asn
   210 215 220
   Asp Leu Met Ala Gly Glu Val Asp Gln Ile Glu Leu Arg His Gly Phe
   225 230 235 240
   Phe Lys Ile His Gln Glu Ala Gln Pro Glu Lys Gly Ser Glu Asn Ala
   245 250 255
   Val Ile Arg Val Pro Ala Ala Leu Pro Asp Glu Val Ile Glu Arg Ile
   260 265 270
   Gln Lys Thr Ala Met Lys Ile Tyr Arg Ile Leu Gly Cys Arg Gly Leu
   275 280 285
   Ala Arg Ile Asp Leu Phe Leu Arg Glu Asp Gly Cys Ile Val Leu Asn
   290 295 300
   Glu Val Asn Thr Met Pro Gly Phe Thr Ser Tyr Ser Arg Tyr Pro Arg
   305 310 315 320
   Met Met Thr Ala Ala Gly Phe Thr Leu Thr Glu Ile Leu Asp Arg Leu
   325 330 335
   Ile Glu Leu Ser Leu Arg Arg
   340
<210> 76
   <211> 343
   <212> PRT
   <213> Unknown
<220>
   <223> peptide from a microbial resistance gene
<400> 76
   Met Tyr Lys Leu Lys Ile Ala Val Leu Phe Gly Gly Cys Ser Glu Glu
   1               5                   10                  15
   His Asp Val Ser Val Lys Ser Ala Met Glu Val Ala Ala Asn Ile Asn
               20                  25                  30
   Lys Glu Lys Tyr Gln Pro Phe Tyr Ile Gly Ile Thr Lys Ser Gly Ala
           35                  40                  45
   Trp Lys Leu Cys Asp Lys Pro Cys Arg Asp Trp Glu Asn Tyr Ala Gly
       50                  55                  60
   Tyr Pro Ala Val Ile Ser Pro Asp Arg Arg Ile His Gly Leu Leu Ile
   65                  70                  75                  80
   Gln Lys Asp Gly Gly Tyr Glu Ser Gln Pro Val Asp Val Val Leu Pro
                   85                  90                  95
   Met Ile His Gly Lys Phe Gly Glu Asp Gly Thr Ile Gln Gly Leu Leu
               100                 105                 110
   Glu Leu Ser Gly Ile Pro Tyr Val Gly Cys Asp Ile Gln Ser Ser Val
           115                 120                 125
   Ile Cys Met Asp Lys Ser Leu Ala Tyr Met Val Val Lys Asn Ala Gly
       130                 135                 140
   Ile Glu Val Pro Gly Phe Arg Val Leu Gln Lys Gly Asp Ser Leu Glu
   145                 150                 155                 160
   Ala Glu Thr Leu Ser Tyr Pro Val Phe Val Lys Pro Ala Arg Ser Gly
                   165                 170                 175
   Ser Ser Phe Gly Val Asn Lys Val Cys Arg Ala Glu Glu Leu Gln Ala
               180                 185                 190
   Ala Val Thr Glu Ala Gly Lys Tyr Asp Ser Lys Ile Leu Val Glu Glu
           195                 200                 205
   Ala Val Ser Gly Ser Glu Val Gly Cys Ala Ile Leu Gly Asn Gly Asn
       210                 215                 220
   Asp Leu Ile Thr Gly Glu Val Asp Gln Ile Glu Leu Lys His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Ala Leu Pro Asp Glu Val Arg Glu Gln Ile
               260                 265                 270
   Gln Glu Thr Ala Lys Lys Ile Tyr Arg Val Leu Gly Cys Arg Gly Leu
           275                 280                 285
   Ala Arg Ile Asp Leu Phe Leu Arg Glu Asp Gly Ser Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Ser Glu Ile Leu Asp Arg Leu
                   325                 330                 335
   Ile Gly Leu Ser Leu Arg Arg
               340
<210> 77
   <211> 343
   <212> PRT
   <213> Unknown
<220>
   <223> peptide from a microbial resistance gene
<400> 77
   Met Tyr Lys Leu Lys Ile Ala Val Leu Phe Gly Gly Cys Ser Glu Glu
   1               5                   10                  15
   His Asp Val Ser Val Lys Ser Ala Met Glu Val Ala Ala Asn Ile Asn
               20                  25                  30
   Lys Glu Lys Tyr Gln Pro Phe Tyr Ile Gly Ile Thr Lys Ser Gly Ala
           35                  40                  45
   Trp Lys Leu Cys Asp Lys Pro Cys Arg Asp Trp Glu Asn Tyr Ala Gly
       50                  55                  60
   Tyr Pro Ala Val Ile Ser Pro Asp Arg Arg Ile His Gly Leu Leu Ile
   65                  70                  75                  80
   Gln Lys Asp Gly Gly Tyr Glu Ser Gln Pro Val Asp Val Val Leu Pro
                   85                  90                  95
   Met Ile His Gly Lys Phe Gly Glu Asp Gly Thr Ile Gln Gly Leu Leu
               100                 105                 110
   Glu Leu Ser Gly Ile Pro Tyr Val Val Cys Asp Ile Gln Ser Ser Val
           115                 120                 125
   Ile Cys Met Asp Lys Ser Leu Ala Tyr Met Val Val Lys Asn Ala Gly
       130                 135                 140
   Ile Glu Val Pro Gly Phe Arg Val Leu Gln Lys Gly Asp Ser Leu Glu
   145                 150                 155                 160
   Ala Glu Thr Leu Ser Tyr Pro Val Phe Val Lys Pro Ala Arg Ser Gly
                   165                 170                 175
   Ser Ser Phe Gly Val Asn Lys Val Cys Arg Ala Glu Glu Leu Gln Ala
               180                 185                 190
   Ala Val Thr Glu Ala Gly Lys Tyr Asp Ser Lys Ile Leu Val Glu Glu
           195                 200                 205
   Ala Val Ser Gly Ser Glu Val Gly Cys Ala Ile Leu Gly Asn Gly Asn
       210                 215                 220
   Asp Leu Ile Thr Gly Glu Val Asp Gln Ile Glu Leu Lys His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Ala Leu Pro Asp Glu Val Arg Glu Gln Ile
               260                 265                 270
   Gln Glu Thr Ala Lys Lys Ile Tyr Arg Val Leu Gly Cys Arg Gly Leu
           275                 280                 285
   Ala Arg Ile Asp Leu Phe Leu Arg Glu Asp Gly Ser Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Ser Glu Ile Leu Asp Arg Leu
                   325                 330                 335
   Ile Gly Leu Ser Leu Arg Arg
               340
<210> 78
   <211> 343
   <212> PRT
   <213> Unknown
<220>
   <223> peptide from a microbial resistance gene
<400> 78
   Met Tyr Lys Leu Lys Ile Ala Val Leu Phe Gly Gly Cys Ser Glu Glu
   1               5                   10                  15
   His Asp Val Ser Val Lys Ser Ala Met Glu Val Ala Ala Asn Ile Asn
               20                  25                  30
   Lys Glu Lys Tyr Gln Pro Phe Tyr Ile Gly Ile Thr Lys Ser Gly Ala
           35                  40                  45
   Trp Lys Leu Cys Asp Lys Pro Cys Arg Asp Trp Glu Asn Tyr Ala Gly
       50                  55                  60
   Tyr Pro Ala Val Ile Ser Pro Asp Arg Arg Thr His Gly Leu Leu Ile
   65                  70                  75                  80
   Gln Lys Asp Gly Gly Tyr Glu Ser Gln Pro Val Asp Val Val Leu Pro
                   85                  90                  95
   Met Ile His Gly Lys Phe Gly Glu Asp Gly Thr Ile Gln Gly Leu Leu
               100                 105                 110
   Glu Leu Ser Gly Ile Pro Tyr Val Gly Cys Asp Ile Gln Ser Ser Val
           115                 120                 125
   Thr Cys Met Asp Lys Ser Leu Ala Tyr Met Val Val Lys Asn Ala Gly
       130                 135                 140
   Ile Glu Val Pro Gly Phe Arg Val Leu Gln Lys Gly Asp Ser Leu Lys
   145                 150                 155                 160
   Ala Glu Thr Leu Ser Tyr Pro Val Phe Val Lys Pro Ala Arg Ser Gly
                   165                 170                 175
   Ser Ser Phe Gly Val Asn Lys Val Cys Arg Ala Glu Glu Leu Gln Ala
               180                 185                 190
   Ala Val Thr Glu Ala Gly Lys Tyr Asp Cys Lys Ile Leu Val Glu Glu
           195                 200                 205
   Ala Val Ser Gly Ser Glu Val Gly Cys Ala Ile Leu Gly Asn Glu Asn
       210                 215                 220
   Ala Leu Met Ala Gly Glu Val Asp Gln Ile Glu Leu Arg His Gly Phe
   225                 230                 235                 240
   Phe Lys Ile His Gln Glu Ala Gln Pro Glu Lys Gly Ser Glu Asn Ala
                   245                 250                 255
   Val Ile Arg Val Pro Ala Val Leu Pro Asp Glu Val Arg Glu Arg Ile
               260                 265                 270
   Gln Lys Thr Ala Met Lys Ile Tyr Arg Ile Leu Gly Cys Arg Gly Leu
           275                 280                 285
   Ala Arg Ile Asp Leu Phe Leu Arg Glu Asp Gly Cys Ile Val Leu Asn
       290                 295                 300
   Glu Val Asn Thr Met Pro Gly Phe Thr Ser Tyr Ser Arg Tyr Pro Arg
   305                 310                 315                 320
   Met Met Thr Ala Ala Gly Phe Thr Leu Thr Glu Ile Leu Asp Arg Leu
                   325                 330                 335
   Ile Glu Leu Ser Leu Arg Arg
               340
<210> 79
   <211> 343
   <212> PRT
   <213> Unknown
<220>
   <223> peptide from a microbial resistance gene
<400> 79
   Met Tyr Arg Ile Asn Val Ala Val Leu Phe Gly Gly Cys Ser Glu Glu
    1               5                  10                  15
   His Thr Val Ser Ile Lys Ser Ala Met Glu Leu Ala Ala Asn Ile Asp
               20                  25                  30
   Thr Glu Lys Tyr Gln Pro Phe Tyr Ile Gly Ile Thr Lys Ser Gly Val
           35                  40                  45
   Trp Lys Leu Cys Glu Lys Pro Cys Leu Asp Trp Glu Gln Tyr Ala Lys
       50                  55                  60
   Tyr Pro Val Val Phe Ser Pro Gly Arg Asn Thr His Gly Phe Leu Ile
   65                  70                  75                  80
   Gln Lys Glu Asp Arg Tyr Glu Ile Gln Pro Val Asp Val Val Phe Pro
                   85                  90                  95
   Ile Ile His Gly Lys Phe Gly Glu Asp Gly Ser Ile Gln Gly Leu Leu
               100                 105                 110
   Glu Leu Ser Gly Ile Pro Tyr Val Gly Cys Asp Ile Gln Ser Ser Val
           115                 120                 125
   Ile Cys Met Asp Lys Ser Leu Ala Tyr Thr Thr Val Lys Asn Ala Gly
       130                 135                 140
   Ile Glu Val Pro Asp Phe Gln Ile Ile Gln Asp Gly Asp Ser Pro Lys
   145                 150                 155                 160
   Thr Glu Cys Phe Ser Phe Pro Leu Phe Val Lys Pro Ala Arg Ser Gly
                   165                 170                 175
   Ser Ser Phe Gly Val Asn Lys Val Asp Lys Ala Glu Asp Leu Cys Ala
               180                 185                 190
   Ala Ile Asn Glu Ala Arg Gln Tyr Asp Arg Lys Val Leu Ile Glu Gln
           195                 200                 205
   Ala Val Ser Gly Ser Glu Val Gly Cys Ala Val Leu Gly Thr Gly Thr
       210                 215                 220
   Asp Leu Ile Val Gly Glu Val Asp Gln Ile Ser Leu Lys His Gly Phe
   225                 230                 235                 240
   Phe Lys Ile His Gln Glu Ala Gln Pro Glu Lys Gly Ser Glu Asn Ala
                   245                 250                 255
   Thr Ile Glu Val Pro Ala Asp Leu Pro Ala Lys Val Arg Glu Arg Ile
               260                 265                 270
   Gln Lys Thr Ala Lys Lys Ile Tyr Gln Val Leu Gly Cys Arg Gly Leu
           275                 280                 285
   Ala Arg Ile Asp Leu Phe Leu Arg Glu Asp Gly His Ile Val Leu Asn
       290                 295                 300
   Glu Val Asn Thr Met Pro Gly Phe Thr Ser Tyr Ser Arg Tyr Pro Cys
   305                 310                 315                 320
   Met Met Thr Ala Ala Gly Phe Thr Leu Ser Glu Leu Ile Asp Arg Leu
                   325                 330                 335
   Ile Glu Leu Ala Leu Arg Arg
               340
<210> 80
   <211> 8
   <212> PRT
   <213> Unknown
<220>
   <223> peptide from a microbial resistance gene
<400> 80
   Gly Ser Glu Asn Ala Val Ile Arg
    1               5
<210> 81
   <211> 6
   <212> PRT
   <213> Unknown
<220>
   <223> peptide from a microbial resistance gene
<400> 81
   Ile Asp Leu Phe Leu Arg
   1 5
<210> 82
   <211> 9
   <212> PRT
   <213> Unknown
<220>
   <223> peptide from a microbial resistance gene
<400> 82
   Ile His Gln Glu Ala Gln Pro Glu Lys
   1 5
<210> 83
   <211> 9
   <212> PRT
   <213> Unknown
<220>
   <223> peptide from a microbial resistance gene
<400> 83
   Ser Gly Ser Ser Phe Gly Val Asn Lys
   1 5
<210> 84
   <211> 352
   <212> PRT
   <213> Unknown
<220>
   <223> peptide from a microbial resistance gene
<400> 84
   Met Lys Thr Val Ala Ile Ile Phe Gly Gly Val Ser Ser Glu Tyr Glu
   1 5 10 15
   Val Ser Leu Lys Ser Ala Val Ala Ile Ile Lys Asn Met Glu Ser Ile
   20 25 30
   Asp Tyr Asn Val Met Lys Ile Gly Ile Thr Glu Glu Gly His Trp Tyr
   35 40 45
   Leu Phe Glu Gly Thr Thr Asp Lys Ile Lys Lys Asp Arg Trp Phe Leu
   50 55 60
   Asp Glu Ser Cys Glu Glu Ile Val Val Asp Phe Ala Lys Lys Ser Phe
   65 70 75 80
   Val Leu Lys Asn Ser Lys Lys Ile Ile Lys Pro Asp Ile Leu Phe Pro
   85 90 95
   Val Leu His Gly Gly Tyr Gly Glu Asn Gly Ala Met Gln Gly Val Phe
   100 105 110
   Glu Leu Leu Asp Ile Pro Tyr Val Gly Cys Gly Ile Gly Ala Ala Ala
   115 120 125
   Ile Ser Met Asn Lys Ile Met Leu His Gln Phe Ala Glu Ala Ile Gly
   130 135 140
   Val Lys Ser Thr Pro Ser Met Ile Ile Glu Lys Gly Gln Asp Leu Gln
   145 150 155 160
   Lys Val Asp Ala Phe Ala Lys Ile His Gly Phe Pro Leu Tyr Ile Lys
   165 170 175
   Pro Asn Glu Ala Gly Ser Ser Lys Gly Ile Ser Lys Val Glu Arg Lys
   180 185 190
   Ser Asp Leu Tyr Lys Ala Ile Asp Glu Ala Ser Lys Tyr Asp Ser Arg
   195 200 205
   Ile Leu Ile Gln Lys Glu Val Lys Gly Val Glu Ile Gly Cys Gly Ile
   210 215 220
   Leu Gly Asn Glu Gln Leu Val Val Gly Glu Cys Asp Gln Ile Ser Leu
   225 230 235 240
   Val Asp Gly Phe Phe Asp Tyr Glu Glu Lys Tyr Asn Leu Val Thr Ala
   245 250 255
   Glu Ile Leu Leu Pro Ala Lys Leu Ser Ile Asp Lys Lys Glu Asp Ile
   260 265 270
   Gln Met Lys Ala Lys Lys Leu Tyr Arg Leu Leu Gly Cys Lys Gly Leu
   275 280 285
   Ala Arg Ile Asp Phe Phe Leu Thr Asp Asp Gly Glu Ile Leu Leu Asn
   290 295 300
   Glu Ile Asn Thr Met Pro Gly Phe Thr Glu His Ser Arg Phe Pro Met
   305 310 315 320
   Met Met Asn Glu Ile Gly Met Asp Tyr Lys Glu Ile Ile Glu Asn Leu
   325 330 335
   Leu Val Leu Ala Val Glu Asn His Glu Lys Lys Leu Ser Thr Ile Asp
   340 345 350
<210> 85
   <211> 7
   <212> PRT
   <213> Unknown
<220>
   <223> peptide from a microbial resistance gene
<400> 85
   Ala Ile Asp Glu Ala Ser Lys
   1 5
<210> 86
   <211> 13
   <212> PRT
   <213> Unknown
<220>
   <223> peptide from a microbial resistance gene
<400> 86
   Phe Pro Met Met Met Asn Glu Ile Gly Met Asp Tyr Lys
   1 5 10
<210> 87
   <211> 13
   <212> PRT
   <213> Unknown
<220>
   <223> peptide from a microbial resistance gene
<400> 87
   Ile Met Leu His Gln Phe Ala Glu Ala Ile Gly Val Lys
   1 5 10
<210> 88
   <211> 11
   <212> PRT
   <213> Unknown
<220>
   <223> peptide from a microbial resistance gene
<400> 88
   Asn Met Glu Ser Ile Asp Tyr Asn Val Met Lys
   1 5 10
<210> 89
   <211> 7
   <212> PRT
   <213> Unknown
<220>
   <223> peptide from a microbial resistance gene
<400> 89
   Ser Ala Val Ala Ile Ile Lys
   1 5
<210> 90
   <211> 9
   <212> PRT
   <213> Unknown
<220>
   <223> peptide from a microbial resistance gene
<400> 90
   Ser Thr Pro Ser Met Ile Ile Glu Lys
    1               5
<210> 91
   <211> 13
   <212> PRT
   <213> Unknown
<220>
   <223> peptide from a microbial resistance gene
<400> 91
   Tyr Asn Leu Val Thr Ala Glu Ile Leu Leu Pro Ala Lys
    1               5                   10
<210> 92
   <211> 349
   <212> PRT
   <213> Unknown
<220>
   <223> peptide from a microbial resistance gene
<400> 92
   Met Ile Lys Lys Arg Ile Ala Ile Ile Phe Gly Gly Asn Ser Thr Glu
    1               5                   10                  15
   Tyr Glu Val Ser Leu Gln Ser Ala Ser Ala Val Phe Glu Asn Ile Asn
               20                  25                  30
   Thr Lys Lys Phe Asp Ile Val Pro Ile Gly Ile Thr Arg Asn Gly Asp
   35 40 45
   Trp Tyr His Tyr Thr Gly Lys Lys Glu Lys Ile Ala Asn Asn Thr Trp
   50 55 60
   Phe Glu Asp Asn Glu Asn Leu Tyr Ser Val Ala Val Ser Gln Asn Arg
   65 70 75 80
   Ser Val Lys Gly Phe Ile Glu Phe Lys Glu Glu Lys Phe Tyr Ile Ile
   85 90 95
   Lys Val Asp Leu Ile Phe Pro Val Leu His Gly Lys Asn Gly Glu Asp
   100 105 110
   Gly Thr Leu Gln Gly Leu Phe Glu Leu Ala Gly Ile Pro Val Val Gly
   115 120 125
   Cys Asp Thr Leu Ser Ser Ala Leu Cys Met Asp Lys Asp Lys Ala His
   130 135 140
   Lys Leu Val Ser Leu Ala Gly Ile Ser Val Pro Lys Ser Val Thr Phe
   145 150 155 160
   Lys Phe Ser Gly Lys Lys Ala Ala Leu Lys Lys Ile Glu Lys Glu Leu
   165 170 175
   Ser Tyr Pro Leu Phe Val Lys Pro Val Arg Ala Gly Ser Ser Phe Gly
   180 185 190
   Ile Thr Lys Val Thr Lys Gln Gln Glu Leu Glu Asn Ala Ile Gln Leu
   195 200 205
   Ala Phe Glu His Asp Ala Glu Val Ile Val Glu Glu Thr Ile Asn Gly
   210 215 220
   Phe Glu Val Gly Cys Ala Val Leu Gly Ile Asp Glu Leu Ile Val Gly
   225 230 235 240
   Arg Val Asp Glu Ile Glu Leu Ser Ser Gly Phe Phe Asp Tyr Thr Glu
   245 250 255
   Lys Tyr Thr Leu Lys Ser Ser Lys Ile Tyr Met Pro Ala Arg Ile Asp
   260 265 270
   Ala Glu Ala Glu Lys Arg Ile Gln Glu Thr Ala Val Thr Ile Tyr Lys
   275 280 285
   Ala Leu Gly Cys Ser Gly Phe Ser Arg Val Asp Met Phe Tyr Thr Pro
   290 295 300
   Ser Gly Glu Ile Val Phe Asn Glu Val Asn Thr Ile Pro Gly Phe Thr
   305 310 315 320
   Ser His Ser Arg Tyr Pro Asn Met Met Lys Gly Ile Gly Leu Ser Phe
   325 330 335
   Ala Gln Met Leu Asp Lys Leu Ile Gly Leu Tyr Val Glu
   340 345
<210> 93
   <211> 349
   <212> PRT
   <213> Unknown
<220>
   <223> peptide from a microbial resistance gene
<400> 93
   Met Gln Asn Lys Lys Ile Ala Val Ile Phe Gly Gly Asn Ser Thr Glu
   1               5                   10                  15
   Tyr Glu Val Ser Leu Gln Ser Ala Ser Ala Val Phe Glu Asn Ile Asn
               20                  25                  30
   Thr Asn Lys Phe Asp Ile Ile Pro Ile Gly Ile Thr Arg Ser Gly Glu
           35                  40                  45
   Trp Tyr His Tyr Thr Gly Glu Lys Glu Lys Ile Leu Asn Asn Thr Trp
       50                  55                  60
   Phe Glu Asp Ser Lys Asn Leu Cys Pro Val Val Val Ser Gln Asn Arg
   65                  70                  75                  80
   Ser Val Lys Gly Phe Leu Glu Ile Ala Ser Asp Lys Tyr Arg Ile Ile
                   85                  90                  95
   Lys Val Asp Leu Val Phe Pro Val Leu His Gly Lys Asn Gly Glu Asp
               100                 105                 110
   Gly Thr Leu Gln Gly Ile Phe Glu Leu Ala Gly Ile Pro Val Val Gly
           115                 120                 125
   Cys Asp Thr Leu Ser Ser Ala Leu Cys Met Asp Lys Asp Arg Ala His
       130                 135                 140
   Lys Leu Val Ser Leu Ala Gly Ile Ser Val Pro Lys Ser Val Thr Phe
   145                 150                 155                 160
   Lys Arg Phe Asn Glu Glu Ala Ala Met Lys Glu Ile Glu Ala Asn Leu
                   165                 170                 175
   Thr Tyr Pro Leu Phe Ile Lys Pro Val Arg Ala Gly Ser Ser Phe Gly
               180                 185                 190
   Ile Thr Lys Val Ile Glu Lys Gln Glu Leu Asp Ala Ala Ile Glu Leu
           195                 200                 205
   Ala Phe Glu His Asp Thr Glu Val Ile Val Glu Glu Thr Ile Asn Gly
       210                 215                 220
   Phe Glu Val Gly Cys Ala Val Leu Gly Ile Asp Glu Leu Ile Val Gly
   225                 230                 235                 240
   Arg Val Asp Glu Ile Glu Leu Ser Ser Gly Phe Phe Asp Tyr Thr Glu
                   245                 250                 255
   Lys Tyr Thr Leu Lys Ser Ser Lys Ile Tyr Met Pro Ala Arg Ile Asp
               260                 265                 270
   Ala Glu Ala Glu Lys Arg Ile Gln Glu Ala Ala Val Thr Ile Tyr Lys
           275                 280                 285
   Ala Leu Gly Cys Ser Gly Phe Ser Arg Val Asp Met Phe Tyr Thr Pro
       290                 295                 300
   Ser Gly Glu Ile Val Phe Asn Glu Val Asn Thr Ile Pro Gly Phe Thr
   305                 310                 315                 320
   Ser His Ser Arg Tyr Pro Asn Met Met Lys Gly Ile Gly Leu Ser Phe
                   325                 330                 335
   Ser Gln Met Leu Asp Lys Leu Ile Gly Leu Tyr Val Glu
               340                 345
<210> 94
   <211> 349
   <212> PRT
   <213> Unknown
<220>
   <223> peptide from a microbial resistance gene
<400> 94
   Met Gln Asn Lys Lys Ile Ala Val Ile Phe Gly Gly Asn Ser Thr Glu
    1               5                  10                  15
   Tyr Glu Val Ser Leu Gln Ser Ala Ser Ala Val Phe Glu Asn Ile Asn
               20                  25                  30
   Thr Asn Lys Phe Asp Ile Ile Pro Ile Gly Ile Thr Arg Ser Gly Glu
           35                  40                  45
   Trp Tyr His Tyr Thr Gly Glu Lys Glu Lys Ile Leu Asn Asn Thr Trp
       50                  55                  60
   Phe Glu Asp Ser Lys Asn Leu Cys Pro Val Val Val Ser Gln Asn Arg
   65                  70                  75                  80
   Ser Val Lys Gly Phe Leu Glu Ile Ala Ser Asp Lys Tyr Arg Ile Ile
                   85                  90                  95
   Lys Val Asp Leu Val Phe Pro Val Leu His Gly Lys Asn Gly Glu Asn
               100                 105                 110
   Gly Thr Leu Gln Gly Ile Phe Glu Leu Ala Gly Ile Pro Val Val Gly
           115                 120                 125
   Cys Asp Thr Leu Ser Ser Ala Leu Cys Met Asp Lys Asp Arg Ala His
       130                 135                 140
   Lys Leu Val Ser Leu Ala Gly Ile Ser Val Pro Lys Ser Val Thr Phe
   145                 150                 155                 160
   Lys Arg Phe Asn Glu Glu Ala Ala Met Lys Glu Ile Glu Ala Asn Leu
                   165                 170                 175
   Thr Tyr Pro Leu Phe Ile Lys Pro Val Arg Ala Gly Ser Ser Phe Gly
               180                 185                 190
   Ile Thr Lys Val Ile Glu Lys Gln Glu Leu Asp Ala Ala Ile Glu Leu
           195                 200                 205
   Ala Phe Glu His Asp Thr Glu Val Ile Val Glu Glu Thr Ile Asn Gly
       210                 215                 220
   Phe Glu Val Gly Cys Ala Val Leu Gly Ile Asp Glu Leu Ile Val Gly
   225                 230                 235                 240
   Arg Val Asp Glu Ile Glu Leu Ser Ser Gly Phe Phe Asp Tyr Thr Glu
                   245                 250                 255
   Lys Tyr Thr Leu Lys Ser Ser Lys Ile Tyr Met Pro Ala Arg Ile Asp
               260                 265                 270
   Ala Glu Ala Glu Lys Arg Ile Gln Glu Ala Ala Val Thr Ile Tyr Lys
           275                 280                 285
   Ala Leu Gly Cys Ser Gly Phe Ser Arg Val Asp Met Phe Tyr Thr Pro
       290                 295                 300
   Ser Gly Glu Ile Val Phe Asn Glu Val Asn Thr Ile Pro Gly Phe Thr
   305                 310                 315                 320
   Ser His Ser Arg Tyr Pro Asn Met Met Lys Gly Ile Gly Leu Ser Phe
                   325                 330                 335
   Ser Gln Met Leu Asp Lys Leu Ile Gly Leu Tyr Val Glu
               340                 345
<210> 95
   <211> 9
   <212> PRT
   <213> Unknown
<220>
   <223> peptide from a microbial resistance gene
<400> 95
   Ala Gly Ser Ser Phe Gly Ile Thr Lys
    1               5
<210> 96
   <211> 9
   <212> PRT
   <213> Unknown
<220>
   <223> peptide from a microbial resistance gene
<400> 96
   Ala Leu Gly Cys Ser Gly Phe Ser Arg
   1 5
<210> 97
   <211> 7
   <212> PRT
   <213> Unknown
<220>
   <223> peptide from a microbial resistance gene
<400> 97
   Ile Asp Ala Glu Ala Glu Lys
   1 5
<210> 98
   <211> 6
   <212> PRT
   <213> Unknown
<220>
   <223> peptide from a microbial resistance gene
<400> 98
   Ile Tyr Met Pro Ala Arg
   1 5
<210> 99
   <211> 7
   <212> PRT
   <213> Unknown
<220>
   <223> peptide from a microbial resistance gene
<400> 99
   Leu Ile Gly Leu Tyr Val Glu
   1 5
<210> 100
   <211> 11
   <212> PRT
   <213> Unknown
<220>
   <223> peptide from a microbial resistance gene
<400> 100
   Leu Val Ser Leu Ala Gly Ile Ser Val Pro Lys
   1 5 10
<210> 101
   <211> 16
   <212> PRT
   <213> Unknown
<220>
   <223> peptide from a microbial resistance gene
<400> 101
   Val Asp Glu Ile Glu Leu Ser Ser Gly Phe Phe Asp Tyr Thr Glu Lys
   1 5 10 15
<210> 102
   <211> 6
   <212> PRT
   <213> Unknown
<220>
   <223> peptide from a microbial resistance gene
<400> 102
   Tyr Pro Asn Met Met Lys
   1 5
<210> 103
   <211> 349
   <212> PRT
   <213> Unknown
<220>
   <223> peptide from a microbial resistance gene
<400> 103
   Met Met Lys Leu Lys Lys Ile Ala Ile Ile Phe Gly Gly Gln Ser Ser
    1               5                  10                  15
   Glu Tyr Glu Val Ser Leu Lys Ser Thr Val Ser Val Leu Glu Thr Leu
               20                  25                  30
   Ser Thr Cys Asn Phe Glu Ile Ile Lys Ile Gly Ile Asp Leu Gly Gly
           35                  40                  45
   Lys Trp Tyr Leu Thr Thr Ser Asn Asn Lys Asp Ile Glu Tyr Asp Val
       50                  55                  60
   Trp Gln Thr Asp Pro Ser Leu Gln Glu Ile Ile Pro Cys Phe Asn Asn
   65                  70                  75                  80
   Arg Gly Phe Tyr Asn Lys Thr Thr Asn Lys Tyr Phe Arg Pro Asp Val
                   85                  90                  95
   Leu Phe Pro Ile Leu His Gly Gly Thr Gly Glu Asp Gly Thr Leu Gln
               100                 105                 110
   Gly Val Phe Glu Leu Met Asn Ile Pro Tyr Val Gly Cys Gly Val Thr
           115                 120                 125
   Pro Ser Ala Ile Cys Met Asp Lys Tyr Leu Leu His Glu Phe Ala Gln
       130                 135                 140
   Ser Val Gly Val Lys Ser Ala Pro Thr Leu Ile Ile Arg Thr Arg Asn
   145                 150                 155                 160
   Cys Lys Asp Glu Ile Asp Lys Phe Ile Glu Lys Asn Asp Phe Pro Ile
                   165                 170                 175
   Phe Val Lys Pro Asn Glu Ala Gly Ser Ser Lys Gly Ile Asn Lys Val
               180                 185                 190
   Asn Glu Pro Asp Lys Leu Glu Asp Ala Leu Thr Glu Ala Phe Lys Tyr
           195                 200                 205
   Ser Lys Ser Val Ile Ile Gln Lys Ala Ile Ile Gly Arg Glu Ile Gly
       210                 215                 220
   Cys Ala Val Leu Gly Asn Glu Lys Leu Leu Val Gly Glu Cys Asp Glu
   225                 230                 235                 240
   Val Ser Leu Asn Ser Asp Phe Phe Asp Tyr Thr Glu Lys Tyr Gln Met
                   245                 250                 255
   Ile Ser Ala Lys Val Asn Ile Pro Ala Ser Ile Ser Val Glu Phe Ser
               260                 265                 270
   Asn Glu Met Lys Lys Gln Ala Gln Leu Leu Tyr Arg Leu Leu Gly Cys
           275                 280                 285
   Ser Gly Leu Ala Arg Ile Asp Phe Phe Leu Ser Asp Asn Asn Glu Ile
       290                 295                 300
   Leu Leu Asn Glu Ile Asn Thr Leu Pro Gly Phe Thr Glu His Ser Arg
   305                 310                 315                 320
   Tyr Pro Lys Met Met Glu Ala Val Gly Val Thr Tyr Lys Glu Ile Ile
                   325                 330                 335
   Thr Lys Leu Ile Asn Leu Ala Glu Glu Lys Tyr Tyr Gly
               340                 345
<210> 104
   <211> 23
   <212> PRT
   <213> Unknown
<220>
   <223> peptide from a microbial resistance gene
<400> 104
   Asp Ile Glu Tyr Asp Val Trp Gln Thr Asp Pro Ser Leu Gln Glu Ile
    1               5                  10                  15
   Ile Pro Cys Phe Asn Asn Arg
               20
<210> 105
   <211> 11
   <212> PRT
   <213> Unknown
<220>
   <223> peptide from a microbial resistance gene
<400> 105
   Glu Ile Gly Cys Ala Val Leu Gly Asn Glu Lys
    1               5                   10
<210> 106
   <211> 17
   <212> PRT
   <213> Unknown
<220>
   <223> peptide from a microbial resistance gene
<400> 106
   Ile Ala Ile Ile Phe Gly Gly Gln Ser Ser Glu Tyr Glu Val Ser Leu
    1               5                   10                  15
   Lys
<210> 107
   <211> 8
   <212> PRT
   <213> Unknown
<220>
   <223> peptide from a microbial resistance gene
<400> 107
   Ile Gly Ile Asp Leu Gly Gly Lys
    1               5
<210> 108
   <211> 10
   <212> PRT
   <213> Unknown
<220>
   <223> peptide from a microbial resistance gene
<400> 108
   Leu Glu Asp Ala Leu Thr Glu Ala Phe Lys
    1               5                   10
<210> 109
   <211> 8
   <212> PRT
   <213> Unknown
<220>
   <223> peptide from a microbial resistance gene
<400> 109
   Leu Ile Asn Leu Ala Glu Glu Lys
   1 5
<210> 110
   <211> 9
   <212> PRT
   <213> Unknown
<220>
   <223> peptide from a microbial resistance gene
<400> 110
   Leu Leu Gly Cys Ser Gly Leu Ala Arg
   1 5
<210> 111
   <211> 21
   <212> PRT
   <213> Unknown
<220>
   <223> peptide from a microbial resistance gene
<400> 111
   Leu Leu Val Gly Glu Cys Asp Glu Val Ser Leu Asn Ser Asp Phe Phe
   1 5 10 15
   Asp Tyr Thr Glu Lys
   20
<210> 112
   <211> 10
   <212> PRT
   <213> Unknown
<220>
   <223> peptide from a microbial resistance gene
<400> 112
   Met Met Glu Ala Val Gly Val Thr Tyr Lys
   1 5 10
<210> 113
   <211> 16
   <212> PRT
   <213> Unknown
<220>
   <223> peptide from a microbial resistance gene
<400> 113
   Asn Asp Phe Pro Ile Phe Val Lys Pro Asn Glu Ala Gly Ser Ser Lys
   1 5 10 15
<210> 114
   <211> 7
   <212> PRT
   <213> Unknown
<220>
   <223> peptide from a microbial resistance gene
<400> 114
   Gln Ala Gln Leu Leu Tyr Arg
   1 5
<210> 115
   <211> 8
   <212> PRT
   <213> Unknown
<220>
   <223> peptide from a microbial resistance gene
<400> 115
   Ser Ala Pro Thr Leu Ile Ile Arg
   1 5
<210> 116
   <211> 18
   <212> PRT
   <213> Unknown
<220>
   <223> peptide from a microbial resistance gene
<400> 116
   Ser Thr Val Ser Val Leu Glu Thr Leu Ser Thr Cys Asn Phe Glu Ile
   1 5 10 15
   Ile Lys
<210> 117
   <211> 16
   <212> PRT
   <213> Unknown
<220>
   <223> peptide from a microbial resistance gene
<400> 117
   Val Asn Ile Pro Ala Ser Ile Ser Val Glu Phe Ser Asn Glu Met Lys
   1 5 10 15
<210> 118
   <211> 9
   <212> PRT
   <213> Unknown
<220>
   <223> peptide from a microbial resistance gene
<400> 118
   Trp Tyr Leu Thr Thr Ser Asn Asn Lys
   1               5
<210> 119
   <211> 13
   <212> PRT
   <213> Unknown
<220>
   <223> peptide from a microbial resistance gene
<400> 119
   Tyr Leu Leu His Glu Phe Ala Gln Ser Val Gly Val Lys
   1               5                   10
<210> 120
   <211> 7
   <212> PRT
   <213> Unknown
<220>
   <223> peptide from a microbial resistance gene
<400> 120
   Tyr Gln Met Ile Ser Ala Lys
   1               5
<210> 121
   <211> 343
   <212> PRT
   <213> Unknown
<220>
   <223> peptide from a microbial resistance gene
<400> 121
   Met Asn Arg Leu Lys Ile Ala Ile Leu Phe Gly Gly Cys Ser Glu Glu
   1               5                   10                  15
   His Asn Val Ser Val Lys Ser Ala Ala Glu Ile Ala Asn Asn Ile Asp
               20                  25                  30
   Ile Gly Lys Tyr Glu Pro Ile Tyr Ile Gly Ile Thr Gln Ser Gly Val
           35                  40                  45
   Trp Lys Thr Cys Glu Lys Pro Cys Ile Asp Trp Asp Asn Glu His Cys
       50                  55                  60
   Arg Ser Ala Val Leu Ser Pro Asp Lys Lys Met His Gly Leu Leu Ile
   65                  70                  75                  80
   Met Gln Asp Lys Gly Tyr Gln Ile Gln Arg Ile Asp Val Val Phe Ser
                   85                  90                  95
   Val Leu His Gly Lys Ser Gly Glu Asp Gly Ala Ile Gln Gly Leu Phe
               100                 105                 110
   Glu Leu Ser Gly Ile Pro Tyr Val Gly Cys Asp Ile Gln Ser Ser Ala
           115                 120                 125
   Val Cys Met Asp Lys Ser Leu Ala Tyr Ile Ile Ala Lys Asn Ala Gly
       130                 135                 140
   Ile Ala Thr Pro Glu Phe Gln Val Ile Tyr Lys Asp Asp Lys Pro Ala
   145                 150                 155                 160
   Ala Asp Ser Phe Thr Tyr Pro Val Phe Val Lys Pro Ala Arg Ser Gly
                   165                 170                 175
   Ser Ser Tyr Gly Val Asn Lys Val Asn Ser Ala Asp Glu Leu Asp Ser
               180                 185                 190
   Ala Ile Asp Leu Ala Arg Gln Tyr Asp Ser Lys Ile Leu Ile Glu Gln
           195                 200                 205
   Gly Val Leu Gly Tyr Glu Val Gly Cys Ala Val Leu Gly Asn Ser Phe
       210                 215                 220
   Asp Leu Ile Val Gly Glu Val Asp Gln Ile Arg Leu Gln His Gly Ile
   225                 230                 235                 240
   Phe Arg Ile His Gln Glu Ala Glu Pro Glu Lys Gly Ser Glu Asn Ala
                   245                 250                 255
   Thr Ile Thr Val Pro Ala Glu Leu Ser Ala Glu Glu Arg Glu Arg Ile
               260                 265                 270
   Lys Glu Ala Ala Lys Asn Ile Tyr Lys Ala Leu Gly Cys Arg Gly Leu
           275                 280                 285
   Ser Arg Val Asp Met Phe Leu Gln Asp Asn Gly Arg Ile Val Leu Asn
       290                 295                 300
   Glu Val Asn Thr Met Pro Gly Phe Thr Ser Tyr Ser Arg Tyr Pro Arg
   305                 310                 315                 320
   Met Met Val Ser Ala Gly Ile Thr Ile Pro Glu Leu Ile Asp His Leu
                   325                 330                 335
   Ile Val Leu Ala Val Lys Glu
               340
<210> 122
   <211> 19
   <212> PRT
   <213> Unknown
<220>
   <223> peptide from a microbial resistance gene
<400> 122
   Asp Asp Lys Pro Ala Ala Asp Ser Phe Thr Tyr Pro Val Phe Val Lys
   1               5                   10                  15
   Pro Ala Arg
<210> 123
   <211> 18
   <212> PRT
   <213> Unknown
<220>
   <223> peptide from a microbial resistance gene
<400> 123
   Gly Ser Glu Asn Ala Thr Ile Thr Val Pro Ala Glu Leu Ser Ala Glu
   1 5 10 15
   Glu Arg
<210> 124
   <211> 17
   <212> PRT
   <213> Unknown
<220>
   <223> peptide from a microbial resistance gene
<400> 124
   Ile Ala Ile Leu Phe Gly Gly Cys Ser Glu Glu His Asn Val Ser Val
   1 5 10 15
   Lys
<210> 125
   <211> 11
   <212> PRT
   <213> Unknown
<220>
   <223> peptide from a microbial resistance gene
<400> 125
   Ile Asp Val Val Phe Ser Val Leu His Gly Lys
   1 5 10
<210> 126
   <211> 9
   <212> PRT
   <213> Unknown
<220>
   <223> peptide from a microbial resistance gene
<400> 126
   Ile His Gln Glu Ala Glu Pro Glu Lys
   1 5
<210> 127
   <211> 17
   <212> PRT
   <213> Unknown
<220>
   <223> peptide from a microbial resistance gene
<400> 127
   Ile Val Leu Asn Glu Val Asn Thr Met Pro Gly Phe Thr Ser Tyr Ser
    1               5                  10                  15
   Arg
<210> 128
   <211> 7
   <212> PRT
   <213> Unknown
<220>
   <223> peptide from a microbial resistance gene
<400> 128
   Leu Gln His Gly Ile Phe Arg
    1               5
<210> 129
   <211> 10
   <212> PRT
   <213> Unknown
<220>
   <223> peptide from a microbial resistance gene
<400> 129
   Met His Gly Leu Leu Ile Met Gln Asp Lys
    1               5                  10
<210> 130
   <211> 22
   <212> PRT
   <213> Unknown
<220>
   <223> peptide from a microbial resistance gene
<400> 130
   Met Met Val Ser Ala Gly Ile Thr Ile Pro Glu Leu Ile Asp His Leu
    1               5                  10                  15
   Ile Val Leu Ala Val Lys
               20
<210> 131
   <211> 14
   <212> PRT
   <213> Unknown
<220>
   <223> peptide from a microbial resistance gene
<400> 131
   Asn Ala Gly Ile Ala Thr Pro Glu Phe Gln Val Ile Tyr Lys
   1 5 10
<210> 132
   <211> 13
   <212> PRT
   <213> Unknown
<220>
   <223> peptide from a microbial resistance gene
<400> 132
   Ser Ala Ala Glu Ile Ala Asn Asn Ile Asp Ile Gly Lys
   1 5 10
<210> 133
   <211> 8
   <212> PRT
   <213> Unknown
<220>
   <223> peptide from a microbial resistance gene
<400> 133
   Ser Ala Val Leu Ser Pro Asp Lys
   1 5
<210> 134
   <211> 9
   <212> PRT
   <213> Unknown
<220>
   <223> peptide from a microbial resistance gene
<400> 134
   Ser Gly Ser Ser Tyr Gly Val Asn Lys
   1 5
<210> 135
   <211> 8
   <212> PRT
   <213> Unknown
<220>
   <223> peptide from a microbial resistance gene
<400> 135
   Ser Leu Ala Tyr Ile Ile Ala Lys
   1 5
<210> 136
   <211> 15
   <212> PRT
   <213> Unknown
<220>
   <223> peptide from a microbial resistance gene
<400> 136
   Thr Cys Glu Lys Pro Cys Ile Asp Trp Asp Asn Glu His Cys Arg
   1 5 10 15
<210> 137
   <211> 10
   <212> PRT
   <213> Unknown
<220>
   <223> peptide from a microbial resistance gene
<400> 137
   Val Asp Met Phe Leu Gln Asp Asn Gly Arg
   1 5 10
<210> 138
   <211> 15
   <212> PRT
   <213> Unknown
<220>
   <223> peptide from a microbial resistance gene
<400> 138
   Val Asn Ser Ala Asp Glu Leu Asp Ser Ala Ile Asp Leu Ala Arg
   1 5 10 15
<210> 139
   <211> 15
   <212> PRT
   <213> Unknown
<220>
   <223> peptide from a microbial resistance gene
<400> 139
   Tyr Glu Pro Ile Tyr Ile Gly Ile Thr Gln Ser Gly Val Trp Lys
   1 5 10 15
<210> 140
   <211> 343
   <212> PRT
   <213> Unknown
<220>
   <223> peptide from a microbial resistance gene
<400> 140
   Met Lys Lys Ile Ala Leu Ile Phe Gly Gly Thr Ser Ala Glu Tyr Glu
   1               5                   10                  15
   Val Ser Leu Lys Ser Ala Ala Ser Val Leu Ser Val Leu Glu Asn Leu
               20                  25                  30
   Asn Val Glu Ile Tyr Arg Ile Gly Ile Ala Ser Asn Gly Lys Trp Tyr
           35                  40                  45
   Leu Thr Phe Ser Asp Asn Glu Thr Ile Ala Asn Asp Leu Trp Leu Gln
       50                  55                  60
   Asp Lys Lys Leu Asn Glu Ile Thr Pro Ser Phe Asp Gly Arg Gly Phe
   65                  70                  75                  80
   Tyr Asp Gln Ala Glu Lys Val Tyr Phe Lys Pro Asp Val Leu Phe Pro
                   85                  90                  95
   Met Leu His Gly Gly Thr Gly Glu Asn Gly Thr Leu Gln Gly Val Phe
               100                 105                 110
   Glu Cys Met Gln Ile Pro Tyr Val Gly Cys Gly Val Ala Ser Ser Ala
           115                 120                 125
   Ile Cys Met Asn Lys Tyr Leu Leu His Gln Phe Ala Lys Ser Val Gly
       130                 135                 140
   Val Met Ser Thr Pro Thr Gln Leu Ile Ser Ser Thr Asp Glu Gln Gln
   145                 150                 155                 160
   Val Ile Lys Asn Phe Thr Glu Leu Tyr Gly Phe Pro Ile Phe Ile Lys
                   165                 170                 175
   Pro Asn Glu Ala Gly Ser Ser Lys Gly Ile Ser Lys Val His Thr Glu
               180                 185                 190
   Ala Glu Leu Thr Lys Ala Leu Thr Glu Ala Phe Gln Phe Ser Gln Thr
           195                 200                 205
   Val Ile Leu Gln Lys Ala Val Ser Gly Val Glu Ile Gly Cys Ala Ile
       210                 215                 220
   Leu Gly Asn Asp Gln Leu Leu Val Gly Glu Cys Asp Glu Val Ser Leu
   225                 230                 235                 240
   Ala Thr Asp Phe Phe Asp Tyr Thr Glu Lys Tyr Gln Met Thr Thr Ala
                   245                 250                 255
   Lys Leu Thr Val Pro Ala Lys Ile Pro Val Ala Thr Ser Arg Glu Ile
               260                 265                 270
   Lys Arg Gln Ala Gln Leu Leu Tyr Gln Leu Leu Gly Cys Gln Gly Leu
           275                 280                 285
   Ala Arg Ile Asp Phe Phe Leu Thr Glu Ala Gly Glu Ile Leu Leu Asn
       290                 295                 300
   Glu Ile Asn Thr Met Pro Gly Phe Thr Asn His Ser Arg Phe Pro Ala
   305                 310                 315                 320
   Met Met Ala Ala Thr Gly Ile Thr Tyr Gln Glu Leu Ile Ser Thr Leu
                   325                 330                 335
   Ile Thr Leu Ala Glu Asp Lys
               340
<210> 141
   <211> 16
   <212> PRT
   <213> Unknown
<220>
   <223> peptide from a microbial resistance gene
<400> 141
   Ala Leu Thr Glu Ala Phe Gln Phe Ser Gln Thr Val Ile Leu Gln Lys
   1 5 10 15
<210> 142
   <211> 8
   <212> PRT
   <213> Unknown
<220>
   <223> peptide from a microbial resistance gene
<400> 142
   Gly Phe Tyr Asp Gln Ala Glu Lys
   1 5
<210> 143
   <211> 17
   <212> PRT
   <213> Unknown
<220>
   <223> peptide from a microbial resistance gene
<400> 143
   Ile Ala Leu Ile Phe Gly Gly Thr Ser Ala Glu Tyr Glu Val Ser Leu
   1 5 10 15
   Lys
<210> 144
   <211> 8
   <212> PRT
   <213> Unknown
<220>
   <223> peptide from a microbial resistance gene
<400> 144
   Ile Gly Ile Ala Ser Asn Gly Lys
   1 5
<210> 145
   <211> 7
   <212> PRT
   <213> Unknown
<220>
   <223> peptide from a microbial resistance gene
<400> 145
   Ile Pro Val Ala Thr Ser Arg
   1 5
<210> 146
   <211> 11
   <212> PRT
   <213> Unknown
<220>
   <223> peptide from a microbial resistance gene
<400> 146
   Leu Asn Glu Ile Thr Pro Ser Phe Asp Gly Arg
   1 5 10
<210> 147
   <211> 21
   <212> PRT
   <213> Unknown
<220>
   <223> peptide from a microbial resistance gene
<400> 147
   Asn Phe Thr Glu Leu Tyr Gly Phe Pro Ile Phe Ile Lys Pro Asn Glu
   1 5 10 15
   Ala Gly Ser Ser Lys
   20
<210> 148
   <211> 16
   <212> PRT
   <213> Unknown
<220>
   <223> peptide from a microbial resistance gene
<400> 148
   Gln Ala Gln Leu Leu Tyr Gln Leu Leu Gly Cys Gln Gly Leu Ala Arg
   1 5 10 15
<210> 149
   <211> 18
   <212> PRT
   <213> Unknown
<220>
   <223> peptide from a microbial resistance gene
<400> 149
   Ser Ala Ala Ser Val Leu Ser Val Leu Glu Asn Leu Asn Val Glu Ile
   1 5 10 15
   Tyr Arg
<210> 150
   <211> 22
   <212> PRT
   <213> Unknown
<220>
   <223> peptide from a microbial resistance gene
<400> 150
   Ser Val Gly Val Met Ser Thr Pro Thr Gln Leu Ile Ser Ser Thr Asp
   1 5 10 15
   Glu Gln Gln Val Ile Lys
   20
<210> 151
   <211> 9
   <212> PRT
   <213> Unknown
<220>
   <223> peptide from a microbial resistance gene
<400> 151
   Val His Thr Glu Ala Glu Leu Thr Lys
   1 5
<210> 152
   <211> 20
   <212> PRT
   <213> Unknown
<220>
   <223> peptide from a microbial resistance gene
<400> 152
   Trp Tyr Leu Thr Phe Ser Asp Asn Glu Thr Ile Ala Asn Asp Leu Trp
   1 5 10 15
   Leu Gln Asp Lys
   20
<210> 153
   <211> 8
   <212> PRT
   <213> Unknown
<220>
   <223> peptide from a microbial resistance gene
<400> 153
   Tyr Leu Leu His Gln Phe Ala Lys
   1 5
<210> 154
   <211> 7
   <212> PRT
   <213> Unknown
<220>
   <223> peptide from a microbial resistance gene
<400> 154
   Tyr Gln Met Thr Thr Ala Lys
   1 5

## Description

La présente invention concerne le domaine de la microbiologie. Plus précisément, l'invention concerne la détection, par spectrométrie de masse, d'au moins un mécanisme de résistance aux glycopeptides d'au moins un microorganisme issu d'un échantillon.

Depuis la découverte des microbes par Pasteur, les microorganismes sont étudiés par microscopie et analyses biochimiques. Ces méthodes traditionnelles sont souvent longues et fastidieuses et des alternatives analytiques ont très tôt été recherchées. C'est ainsi que l'analyse de bactéries par spectrométrie de masse a été initiée dès 1975 par J. Anhalt et C. Fenselau [1].

Ces travaux préliminaires ont été suivis par l'étude en chromatographie en phase gazeuse couplée à la spectrométrie de masse (GC-MS) d'acides gras de la paroi des microorganismes [2]. Cette méthode a été popularisée sous l'appellation anglo-saxonne de FAME pour Fatty Acid Methyl Ester. Elle constitue actuellement une méthode de référence pour les études taxonomiques. Son utilisation reste cependant limitée à certains laboratoires spécialisés maîtrisant le traitement de l'échantillon par saponification, hydrolyse et dérivation.

En 1996, les travaux de M. Claydon et al [3] ainsi que de T. Krishnamurthy et P. Ross [4] ont montré la possibilité d'identifier différentes espèces bactériennes avec un spectromètre de masse de type MALDI-TOF (acronyme de l'anglais Matrix Assisted Laser Desorption lonization - Time Of Flight). L'analyse associe l'acquisition d'un spectre de masse et l'interprétation d'un logiciel expert. Elle est extrêmement simple et peut être effectuée en quelques minutes. Elle se diffuse actuellement dans les laboratoires d'analyses médicales [5]. Son utilisation clinique est cependant limitée à l'identification d'espèces bactériennes et de levures. Elle n'est pas utilisée en routine pour l'identification des résistances aux antimicrobiens.

Or l'identification de résistances aux antimicrobiens tels que les antibiotiques est un élément essentiel pour assurer une prise en charge optimale des patients.

D'autres procédés de spectrométrie de masse, notamment en tandem, ont été proposés pour répondre à ces besoins. A titre d'exemple, il est possible de citer les travaux de C. Fenselau et al. pour l'identification de β-Lactamase avec un quadripôle-TOF (Q-TOF) [6].

Cependant ces résultats de recherche ne sont pas applicables à une utilisation clinique de routine. Ils ont été obtenus avec des instruments de recherche nécessitant un personnel hautement qualifié. Les temps d'analyse, souvent supérieurs à une heure par échantillon, sont incompatibles avec la charge de travail d'un laboratoire d'analyse microbiologique.

Plus récemment S. Hofstadler et al. [7] ont proposé un procédé associant une amplification du génome microbien par PCR à une détection des produits de PCR par électrospray-TOF (ESI-TOF). Ce procédé est maintenant totalement automatisé [8]. Toutefois, il nécessite une amplification par PCR avec les défauts inhérents à la biologie moléculaire, à savoir rendement d'extraction, coût des sondes, etc.

Dans ce contexte, l'objectif de la présente invention est de proposer un procédé de détection de mécanismes de résistance aux glycopeptides, qui permette de palier les inconvénients des procédés de l'art antérieur, à savoir fournir un procédé peu coûteux, sans réactif spécifique à chaque espèce, notamment par rapport aux procédés de biologie moléculaire, donnant un résultat en un temps court, inférieur à une heure, et utilisable en clinique de routine, sans nécessiter un personnel hautement qualifié.

A cette fin, l'invention propose un nouveau procédé de détection, pour au moins un microorganisme compris dans un échantillon, d'au moins un marqueur de résistance à un glycopeptide qui est la vancomycine, comprenant la détection, par spectrométrie de masse, d'au moins un peptide dudit microorganisme, qui sont des peptides de type Van choisis parmi les peptides de séquence SEQ ID N° 5 à 16, 29 à 35, 59 à 71, 80 à 83, 85 à 91, 95 à 102, 104 à 120, 122 à 139 ou 141 à 154

Avantageusement, des marqueurs de résistance à plusieurs antimicrobiens différents peuvent être détectés simultanément.

De façon surprenante et inattendue, le procédé de la présente invention permet de détecter l'existence de mécanismes de résistance inductibles, en l'absence d'induction.

Comme indiqué dans la demande WO2011045544, des marqueurs de typage et/ou de virulence des microorganismes peuvent être détectés, de la même façon, par spectrométrie de masse, préalablement ou simultanément à la détection des marqueurs de mécanisme de résistance.

Par marqueurs de la résistance à au moins un antimicrobien de la classe des glycopeptides, on entend des molécules d'origine protéique qui sont caractéristiques desdites propriétés de résistance.

Les glycopeptides sont des antibiotiques tels que la vancomycine, la téicoplanine la telavancine, la bleomycine, la ramoplanine, et la decaplanine. La vancomycine est un glycopeptide proprement dit, la téicoplanine est un lipoglycopeptide. Ces 2 molécules viennent inhiber la synthèse de la paroi bactérienne par inhibition de la synthèse du peptidoglycane. En effet, leur structure tri-dimensionnelle, en forme de poche, recouvre le dipeptide D-Ala D-Ala précurseur de la synthèse du peptidoglycane, empêche l'action des glycosyltransférases et des transpeptidases et bloque l'élongation du peptidoglycane. La résistance aux glycopeptides est due à la présence d'opérons codant des enzymes catalysant la formation de précurseurs de faible affinité et des enzymes éliminant les précurseurs de haute affinité produits par la bactérie hôte. Divers types de résistance sont connus selon les enzymes présentes dans l'opéron. La classification de la résistance aux glycopeptides est basée sur la séquence primaire des gènes de structure des ligases de résistance plutôt que sur les niveaux de résistances aux glycopeptides, dans la mesure où les concentrations minimales inhibitrices (CMI) des divers types se chevauchent. Neuf types de résistance ont été individualisés. Huit résultent d'une résistance acquise (VanA, B, D, E, G, L, M et N) et un (VanC) est une résistance naturelle présente chez *E. gallinarum* et à *E. casseliflavus.* (pour une revue voir [9], Sujatha,S. & Praharaj,I. Glycopeptide Resistance in Gram-Positive Cocci: A Review. Interdiscip. Perspect. Infect. Dis 2012, 781679 (2012))

Le type de résistance VanA est caractérisé par une résistance inductible de haut niveau à la vancomycine et à la téicoplanine. A noter que la transmission du type VanA vers *S. aureus* a été mise en évidence mais est toutefois assez rare.

Le type de résistance VanB est caractérisé par une résistance inductible de niveau modéré à haut à la vancomycine et est sensible à la téicoplanine.

En général, les phénotypes VanA et VanB sont les cas cliniques les plus importants et sont fréquemment retrouvés chez *E.faecalis* et chez *E.faecium.*

Le type de résistance VanD est caractérisé par une résistance constitutive de niveau modéré à la vancomycine et à la téicoplanine.

Les types de résistance VanC, E, G, L et N sont caractérisés par une résistance de bas niveau à la vancomycine et par une sensiblité à la téicoplanine.

La résistance peut s'exprimer en absence d'antibiotique, elle est alors dite constitutive. Elle peut aussi s'exprimer en présence d'une certaine concentration d'antibiotique, elle est alors dite inductible. La culture du microorganisme en présence d'une certaine concentration d'antibiotique est alors appelée culture avec induction.

Par détermination de la résistance à au moins un antimicrobien, on entend l'impossibilité de détruire les bactéries ou d'inhiber leur multiplication à une concentration définie en fonction des paramètres pharmocodynamiques et pharmacocinétiques propres à chaque antibiotique.

Par détermination d'un mécanisme de résistance à au moins un antibiotique, on entend la mise en évidence d'au moins un composé synthétisé par la bactérie, lui permettant d'échapper au moins partiellement à l'action de l'antibiotique, le plus souvent par modification de la structure de l'antibiotique ou bien de sa cible métabolique, ou bien encore par sa diminution de pénétration dans la bactérie. Préférentiellement, ledit composé synthétisé par la bactérie est une protéine.

Le procédé de l'invention peut être mis en oeuvre pour détecter des mécanismes de résistance aux glycopeptides chez des bactéries. Ainsi, par exemple, à titre de bactéries chez lesquelles il est possible de rechercher un mécanisme de résistance aux glycopeptides selon le procédé de l'invention, on peut citer, de façon non exhaustive : *Enterococcus faecalis, Enterococcus faecium, Enterococcus gallinarum, Enterococcus casseliflavus, Enterococcus durans, Enterococcus avium, Enterococcus raffinosus* ou *Staphylococcus aureus.* Les agents, majoritairement, responsables d'infections sont les Entérocoques et plus particulièrement *Enterococcus faecalis* et *Enterococcus faecium.*

Ainsi, le procédé selon l'invention permet également de détecter un mécanisme de résistance auxdits antibiotiques.

On entend par échantillon une petite partie ou petite quantité isolée d'une entité pour l'analyse. L'échantillon sur lequel le procédé de l'invention peut être mis en oeuvre est tout échantillon susceptible de contenir un microorganisme cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel, c'est-à-dire que les marqueurs des mécanismes de résistance des bactéries aux glycopeptides sont potentiellement détectables directement dans l'échantillon testé, ou bien il peut subir préalablement à l'analyse, une préparation de type enrichissement, extraction, concentration, purification, culture, selon des méthodes connues de l'homme du métier.

L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages), de viande, de poisson, d'oeuf, de fruit, de légume, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

En amont de la détection par spectrométrie de masse, l'échantillon à analyser est préférentiellement traité au préalable pour générer des peptides à partir de l'ensemble des protéines présentes dans l'échantillon pour fragmenter ces protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré par rapport au traitement physico-chimique car il préserve davantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO2005/098017:
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de *Staphylococcus aureus* qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

La génération de peptides à l'aide d'un réactif chimique ou d'une protéase, peut être obtenu par simple réaction en solution. Elle peut également être mise en oeuvre avec un four à micro-ondes [10], ou sous pression [11], ou bien encore avec un dispositif à ultrasons [12]. Dans ces trois derniers cas, le protocole sera beaucoup plus rapide.

Parmi les peptides ainsi obtenus, les peptides spécifiques de la protéine, sont nommés peptides protéotypiques. Ce sont eux qui seront dosés par spectrométrie de masse.

Selon l'invention, les marqueurs des mécanismes de résistance des bactéries aux glycopeptides sont des protéines de la bactérie chez laquelle les mécanismes de résistance aux glycopeptides sont à rechercher. En particulier, lesdites protéines sont digérées en peptides, de préférence par une enzyme, de préférence encore par la trypsine.

De même, l'échantillon contenant des marqueurs protéiques de caractérisation des mécanismes de résistance des bactéries aux glycopeptides peut également être préalablement traité à des fins de purification. Ce traitement préalable de purification peut être mis en oeuvre avant ou après l'étape de génération de peptides tels que décrits précédemment.

Le traitement préalable de purification d'échantillon est largement connu de l'homme du métier et pourra notamment mettre en oeuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [13], ou H. Keshishian et al. [14]. Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([15] et demande de brevet WO 2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

La spectrométrie de masse à mettre en oeuvre dans le procédé de l'invention est largement connue de l'homme du métier comme un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelle que soit la méthode de spectrométrie de masse utilisée pour la détection, cette dernière comprend une étape d'ionisation de la molécule cible en ions dits moléculaires, dans le cas présent une étape d'ionisation des marqueurs de caractérisation, et une étape de séparation des ions moléculaires obtenus en fonction de leur masse.

Tous les spectromètres de masse comportent :
- une source d'ionisation destinée à ioniser les marqueurs présents dans l'échantillon à analyser, c'est-à-dire à conférer une charge positive ou négative à ces marqueurs;
- un analyseur de masse destiné à séparer les marqueurs ionisés, ou ions moléculaires, en fonction de leur ratio masse sur charge (m/z) ;
- un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être mise en oeuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice (MALDI), activée par une surface (SELDI) ou sur silicium (DIOS)
- l'ionisation-désorption par interaction avec espèces métastables (DART) Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [16]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF), la résonance cyclotronique ionique à transformée de Fourier (FT-ICR), l'orbitrappe.

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS². Lorsque trois séparations MS successives sont réalisées, l'analyse est appelée MS/MS/MS ou MS³ et plus généralement, lorsque n séparations MS successives sont réalisées, l'analyse est appelée MSⁿ.

Parmi les techniques mettant en oeuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS². De même le mode MRM³ est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter. Dans le cas d'une détection en mode MS/MS, essentiellement deux étapes sont ajoutées, par rapport à un dosage MS qui sont :
- une fragmentation des ions moléculaires, alors appelés ions précurseurs, pour donner des ions dit ions fragments de 1^{ère} génération, et
- une séparation des ions dit ions fragments de 1^{ère} génération en fonction de leur masse (m/z)₂, le rapport (m/z)₁ correspondant au rapport (m/z) des ions précurseurs.

C'est alors le rapport masse/charge des ions fragments de 1^{ère} génération ainsi obtenus qui est corrélé à la molécule cible à détecter. Par ion fragment de première génération, on entend un ion issu de l'ion précurseur, suite à une étape de fragmentation et dont le rapport masse sur charge m/z est différent de l'ion précurseur.

Les couples (m/z)₁ et (m/z)₂ sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [17]. Des logiciels commerciaux, tels que les logiciels MIDAS et MRM Pilote d'Applied Biosytems ou encore MRMaid [18] pourront être utilisés par l'homme de l'art pour lui permettre de prédire tous les couples de transitions possibles. Il pourra également être fait appel à une base de données nommée PeptideAtlas, construite par F. Desiere et al. [19] pour compiler l'ensemble des transitions MRM de peptides décrites par la communauté scientifique. Cette base PeptideAtlas est disponible en accès libre sur internet. Pour des molécules non protéiques, il est également possible d'utiliser des bases de données, telles que par exemple celle accessible au travers du logiciel Cliquid de la société Applied Biosytems (Etats Unis d'Amérique).

Une approche alternative pour sélectionner les peptides protéotypiques, (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunions utilisateurs [18]. Elle permet de générer une liste de transitions candidates à partir des peptides identifiés expérimentalement par le logiciel SIEVE (Thermo Scientific). Certains critères ont été détaillés par J. Mead et al. [18] pour le choix des ions (m/z)₁ et (m/z)₂ et sont détaillés ci-après :
- les peptides avec des sites de clivage interne, c'est-à-dire avec de la Lysine ou de l'Arginine interne, doivent être évités, sauf si la Lysine ou l'Arginine est suivie par de la Proline,
- les peptides avec de l'Asparagine ou de la Glutamine doivent être évités car ils peuvent se désaminer,
- les peptides avec de la Glutamine ou de l'Acide Glutamique en N-terminal doivent être évités car ils peuvent se cycliser spontanément,
- les peptides avec de la Méthionine doivent être évités car ils peuvent être oxydés,
- les peptides avec de la Cystéine doivent être évités car ils peuvent être modifiés de façon non reproductible lors d'une éventuelle étape de dénaturation, réduction et blocage des fonctions thiols,
- les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- la sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en oeuvre dans une cellule de fragmentation telle que les modèles de type triple quadripôle [20], ou de type trappe ionique [21], ou encore de type temps de vol (TOF) [22], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e⁻V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Q1q2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être détectés, voire quantifiés.

Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

Lorsque la spectrométrie de masse mise en oeuvre dans le procédé de l'invention est une spectrométrie de masse en tandem (MS², MS³, MS⁴ ou MS⁵), plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Selon des modes de réalisation préférés de l'invention, le procédé de l'invention comprend une ou plusieurs des caractéristiques suivantes :
- la spectrométrie de masse, mise en oeuvre pour les propriétés de résistance potentielle à au moins un antimicrobien, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est de la MRM, ce qui a pour avantage d'utiliser un temps de cycle d'analyse dans le spectromètre de masse de quelques dizaines de millisecondes, ce qui permet de détecter ou de quantifier avec une grande sensibilité, et de façon multiplexée, un grand nombre de molécules différentes ;
- le cas échéant, la détermination des propriétés de typage, et du facteur de virulence est mise en oeuvre dans le même appareil de spectrométrie de masse que la détermination des marqueurs de résistance à au moins un antimicrobien, de préférence simultanément, ce qui a pour avantage de réduire le temps d'analyse et le coût de l'instrument, cela facilite également le traitement et le rendu des résultats.

Outre la détermination de la résistance à un antibiotique, il convient d'identifier le ou les microorganismes présents dans l'échantillon à tester.

Les procédés d'identification de microorganismes sont largement connus de l'homme du métier, comme décrit par exemple par Murray P. R. el al. dans Manuel of Clinical Microbiology, 2007, 9ème édition, et en particulier dans le Vol. I, Section III, chapitres 15 et 16 pour les bactéries et levures, Vol. II, Section VI, chapitre 82 pour les virus, et Vol. II, Section X, chapitre 135 pour les protozoaires. A titre d'exemple de procédés classiques d'identification, on peut citer la détermination du profil biologique, en utilisant par exemple les cartes d'identification du Vitek 2 (bioMérieux™), ou bien encore en utilisant des techniques de biologie moléculaire avec des critères d'identification basés sur l'étude de la présence de certains gènes, sur l'étude de leur séquence. L'identification peut être mise en oeuvre directement à partir de l'échantillon dans lequel on effectue l'identification, ou bien les microorganismes contenus dans l'échantillon peuvent être cultivés par des méthodes bien connues de l'homme du métier avec des milieux de culture et des conditions de culture optimales adaptées en fonction des espèces de microorganismes à rechercher, comme décrit par Murray P.R. et al. dans Manuel of Clinical Microbiology, 2007, 9ème édition, Vol. I, Section III, chapitre 14, et en particulier dans le Vol. I, Section IV, chapitre 21 pour les bactéries, Vol. II, Section VI, chapitre 81 pour les virus, Vol. II, Section VIII, chapitre 117 pour les levures, et Vol. II, Section X, chapitre 134 pour les protozoaires.

Ainsi, de façon générale, dans le cas d'une identification par une méthode biochimique d'une bactérie dans un prélèvement, il faut d'abord l'obtenir en culture pure, par exemple après ensemencement sur gélose. La biologie moléculaire (PCR) peut dans certains cas s'appliquer directement à l'échantillon à analyser.

Au lieu de cultiver les microorganismes, ces derniers peuvent être concentrés par capture directement dans l'échantillon au moyen de surfaces actives. Un tel procédé a été décrit par W.-J. Chen et al. [10] qui ont capturé différentes espèces bactériennes à l'aide de billes magnétiques avec une surface activée au Fe₃O₄/TiO₂. Une capture par d'autres moyens est également possible, telle qu'une capture par des lectines [23], ou par des anticorps [24], ou encore par de la Vancomycine [25]. La capture permet de concentrer les microorganismes et ainsi de réduire ou même de supprimer l'étape de culture. Il s'en suit un gain de temps considérable.

L'identification peut également être mise en oeuvre par spectrométrie de masse, selon les techniques décrites précédemment, de préférence par MS, par MS/MS, ou bien par MS suivie d'une spectrométrie de type MS/MS, ce qui constitue un mode de réalisation de l'invention. Dans ce cas également, l'échantillon peut être soumis préalablement à une étape de culture telle qu'un ensemencement sur gélose.

L'utilisation d'un procédé d'identification par MS est avantageux en ce qu'il peut être réalisé en quelques minutes et en ce qu'il nécessite un spectromètre de masse avec un seul analyseur, c'est à dire un instrument moins complexe qu'un spectromètre de masse en tandem utilisé en MS/MS.

L'utilisation d'un procédé d'identification par MS suivi d'une spectrométrie de type MS/MS est également avantageuse. Elle permet de s'assurer de l'identité des ions observée en MS, ce qui augmente la spécificité de l'analyse.

L'utilisation d'un procédé d'identification par MS/MS de type MRM présente l'avantage d'être plus sensible et plus simple que les approches MS puis MS/MS traditionnelle. Ce procédé ne nécessite ni logiciel performant, nécessaire pour traiter l'information entre l'acquisition du spectre MS et du spectre MS/MS, ni changement du réglage des paramètres machines, nécessaires pour enchaîner des spectres MS puis MS/MS.

Le procédé d'identification par MS peut être mis en oeuvre avec une source électrospray sur échantillon brut, comme décrit par S. Vaidyanathan et al. [26] ou encore par R. Everley et al. [27] après séparation chromatographique. Différentes gammes de m/z permettent alors d'identifier les microorganismes. S. Vaidyanathan et al. ont utilisé une fenêtre entre 200 et 2000 Th, et R. Everley et al. une fenêtre entre 620 et 2450 Th. Les spectres de masse peuvent également être déconvolués pour accéder à la masse des protéines indépendamment de leur état de charge. R. Everley et al. ont ainsi exploité les masses entre environ 5 000 et 50 000 Da. De façon alternative, le procédé d'identification par MS peut être également mis en oeuvre à l'aide d'un MALDI-TOF, comme décrit par Claydon et al [3] et T. Krishnamurthy et P. Ross [4]. L'analyse associe l'acquisition d'un spectre de masse et l'interprétation d'un logiciel expert. Elle est extrêmement simple et peut être effectuée en quelques minutes. Ce procédé d'identification se répand actuellement dans les laboratoires d'analyse médicale [5]

L'identification de bactéries par MS puis MS/MS via leurs protéines présentes dans l'échantillon, a été largement appliquée par de nombreuses équipes. A titre d'exemple, il est possible de citer les travaux récents de Manes N. et al. [28] qui ont étudié le peptidome de *Salmonella enterica,* ou les travaux de R. Nandakumar et al. [29] ou de L. Hernychova et al. [30] qui ont étudié le protéome de bactéries après digestion des protéines avec de la trypsine. L'approche classique consiste à i) acquérir un spectre MS, ii) sélectionner successivement chaque ion précurseur observé sur le spectre MS avec un signal intense, iii) fragmenter successivement chaque ion précurseur et acquérir son spectre MS/MS, iv) interroger des bases de données protéiques telles que SWISS-PROT ou NCBI, au moyen de logiciels tels que Mascot (Matrix Science, Londres, Royaume-Uni) ou SEQUEST (Thermo Scientific, Waltham, Etats-Unis d'Amérique), pour identifier le peptide ayant une forte probabilité de correspondre au spectre MS/MS observé. Cette méthode peut conduire à l'identification d'un microorganisme si une protéine ou un peptide caractéristique de l'espèce est identifié.

Un des avantages de l'utilisation de la spectrométrie de masse réside en ce qu'elle est particulièrement utile pour quantifier des molécules, dans le cas présent les marqueurs des mécanismes de résistance des bactéries aux glycopeptides. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Une calibration est néanmoins nécessaire pour pouvoir corréler l'aire du pic mesurée, correspondant à l'intensité de courant induit par les ions détectés, à la quantité de molécule cible à doser. Pour cela, les calibrations classiquement utilisées en spectrométrie de masse pourront être mis en oeuvre, dans le cadre de l'invention. Les dosages MRM sont classiquement calibrés à l'aide de standards externes ou, de préférence, à l'aide de standards internes tels que décrits par T. Fortin et al. [13]. Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N).

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [31] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. [33] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [32] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée *ex-vivo* avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibration, utilisée dans des dosages par spectrométrie de masse et en particulier dans les dosages MRM ou MS, pourront être mises en oeuvre pour effectuer la calibration, dans le cadre de l'invention.

De façon préférentielle, la spectrométrie de masse utilisée dans le procédé de détection selon l'invention est de type MS/MS. Plus préférentiellement, la spectrométrie de masse est la MRM.

Le procédé de l'invention permet la détection de résistances aux glycopeptides, caractérisée par la détection d'au moins un peptide en tant que marqueur de résistance.

De façon préférentielle, le procédé selon l'invention permet de détecter au moins un marqueur de résistance à la vancomycine.

Selon un premier mode de réalisation, le procédé selon l'invention permet de détecter directement la résistance à un glycopeptide, sans mise en contact du microorganisme potentiellement compris dans l'échantillon avec ledit glycopeptide, c'est-à-dire sans étape d'induction de ladite résistance.

Selon un autre mode de réalisation, le procédé de l'invention comprend une étape supplémentaire, préalable à l'étape de détection, et consistant en l'induction du mécanisme de résistance, par mise en contact dudit échantillon avec le(s)dit(s) glycopeptide(s).

De façon préférentielle, ledit marqueur de résistance est un peptide issu des protéines de type Van, telles que les variants respectifs de type Van A, Van B, Van C, Van D, Van E, Van G, Van L, Van M et Van N.

Selon un mode de réalisation de l'invention, la détection d'un mécanisme de résistance aux glycopeptides, lié à l'expression de la protéine VanA, est caractérisée par la détection d'au moins un peptide appartenant à la protéine VanA et à ses différents variants de séquences **SEQ ID N° 1** à **SEQ ID N° 4.**
**SEQ ID N°1** :
**SEQ ID N°2** :
**SEQ ID N°3** :
**SEQ ID N°4** :
lesdits peptides de type VanA étant choisis, de préférence, parmi les peptides de séquence **SEQ ID N°5** à **SEQ ID N°16** tels que définis ci-après :

| **Peptide SEQ ID N°** | **Séquence d'acides aminés** | **Position du peptide dans la ou les protéines VanA** |
|---|---|---|
| **SEQ ID N°5** | IHQEVEPEK | 243-251 pour les protéines de SEQ N°2, 3, 4; 259-267 pour la protéine de séquence SEQ ID N°1 |
| **SEQ ID N°6** | LIVLALK | 336-342 pour les protéines de SEQ N°2, 3, 4; 352-358 pour la protéine de séquence SEQ ID N°1 |
| **SEQ ID N°7** | LQYGIFR | 236-242 pour les protéines de SEQ N°2, 3, 4; 252-258 pour la protéine de séquence SEQ ID N°1 |
| **SEQ ID N°8** | MHGLLVK | 75-81 pour les protéines de SEQ N°2, 3, 4; 91-97 pour la protéine de séquence SEQ ID N°1 |
| **SEQ ID N°9** | MMAAAGIALPELIDR | 321-335 pour les protéines de SEQ N°2, 3, 4; 337-351 pour la protéine de séquence SEQ ID N°1 |
| **SEQ ID N°10** | NAGIATPAFWVINK | 142-155 pour les protéines de SEQ N°2, 3, 4; 158-171 pour la protéine de séquence SEQ ID N°1 |
| **SEQ ID N°11** | SAIEIAANINK | 23-33 pour les protéines de SEQ N°2, 3, 4; 39-49 pour la protéine de séquence SEQ ID N°1 |
| **SEQ ID N°12** | SGSSFGVK | 175-182 pour les protéines de SEQ N°2, 3, 4; 191-198 pour la protéine de séquence SEQ ID N°1 |
| **SEQ ID N°13** | SLTYIVAK | 134-141 pour les protéines de SEQ N°2, 3, 4; 150-157 pour la protéine de séquence SEQ ID N°1 |
| **SEQ ID N°14** | VDMFLQDNGR | 291-300 pour les protéines de SEQ N°2, 3, 4; 307-316 pour la protéine de séquence SEQ ID N°1 |
| **SEQ ID N°15** | VNSADELDY AIESAR | 184-198 pour les protéines de SEQ N°2, 3, 4; 200-214 pour la protéine de séquence SEQ ID N°1 |
| **SEQ ID N°16** | YEPL YIGITK | 36-45 pour les protéines de SEQ N°2, 3, 4; 52-61 pour la protéine de séquence SEQ ID N°1 |

Avantageusement, les marqueurs de type VanA choisis parmi les peptides de séquence SEQ ID N° 5 à SEQ ID N° 16 sont détectés après induction du mécanisme de résistance.

Avantageusement, les marqueurs de type VanA choisis parmi les peptides de séquence SEQ ID N° 6, SEQ ID N° 7, SEQ ID N° 8, SEQ ID N° 11, SEQ ID N° 12, SEQ ID N° 13, SEQ ID N° 15 et SEQ ID N° 16 sont détectés sans étape d'induction.

Selon un autre mode de réalisation de l'invention, la détection d'un mécanisme de résistance aux glycopeptides, lié à l'expression de la protéine VanB, est caractérisée par la détection d'au moins un peptide appartenant à la protéine VanB et à ses différents variants de séquences **SEQ ID N° 17** à **SEQ ID N° 28.**
**SEQ ID N°17 :**
**SEQ ID N°18 :**
**SEQ ID N°19 :**
**SEQ ID N°20 :**
**SEQ ID N°21 :**
**SEQ ID N°22 :**
**SEQ ID N°23 :**
**SEQ ID N°24 :**
**SEQ ID N°25 :**
**SEQ ID N°26 :**
**SEQ ID N°27 :**
**SEQ ID N°28 :**
lesdits marqueurs de type VanB étant choisis, de préférence, parmi les peptides de séquence **SEQ ID N°29** à **SEQ ID N°35** tels que définis ci-après :

| **Peptide SEQ ID N°** | **Séquence d'acides aminés** | **Position du peptide dans la ou les protéines VanB** |
|---|---|---|
| **SEQ ID N°29** | FDPHYIGITK | 36-45 pour les protéines de SEQ N°17, 18, 19, 20, 21, 22, 23, 24, 25, 26, 27, 28 |
| **SEQ ID N°30** | IDVAFPVLHGK | 90-100 pour les protéines de SEQ N°17, 18, 19, 20, 21, 22, 23, 24, 25, 26, 27, 28 |
| **SEQ ID N°31** | IHQENEPEK | 242-250 pour les protéines de SEQ N°17, 18, 19, 20, 21, 22, 23, 25, 26, 27, 28; 244-252 pour la protéine de séquence SEQ ID N°24 |
| **SEQ ID N°32** | LSHGIFR | 235-241 pour les protéines de SEQ N°17, 18, 19, 20, 21, 22, 23, 25, 26, 27, 28; 237-243 pour la protéine de séquence SEQ ID N°24 |
| **SEQ ID N°33** | SLAYILTK | 133-140 pour les protéines de SEQ N°17, 18, 19, 20, 21, 22, 23, 25, 26, 27, 28; 135-142 pour la protéine de séquence SEQ ID N°24 |
| **SEQ ID N°34** | THGLLVMK | 74-81 pour les protéines de SEQ N°17, 18, 19, 20, 21, 22, 23, 24, 25, 26, 27, 28 |
| **SEQ ID N°35** | VQETAK | 271-276 pour les protéines de SEQ N°17, 18, 19, 20, 21, 22, 23, 25, 26, 27, 28; 273-278 pour la protéine de séquence SEQ ID N°24 |

Avantageusement, les marqueurs de type VanB choisis parmi les peptides de séquence SEQ ID N° 29, SEQ ID N° 30, SEQ ID N° 32 et SEQ ID N° 33 sont détectés après induction du mécanisme de résistance.

Selon un autre mode de réalisation de l'invention, la détection d'un mécanisme de résistance aux glycopeptides, lié à l'expression de la protéine VanC, est caractérisée par la détection d'au moins un peptide appartenant à la protéine VanC et à ses différents variants de séquences **SEQ ID N° 36** à **SEQ ID N° 58.** Notamment les variants VanC1 sont caractérisés par la détection d'au moins un peptide appartenant à la protéine VanC1 et à ses différents variants de séquences **SEQ ID n°36** à **SEQ ID N°43,** les variants VanC2 ou VanC3 sont caractérisés par la détection d'au moins un peptide appartenant à la protéine VanC2 ou Van C3 et à ses différents variants de séquences **SEQ ID N°46** à **SEQ ID N°51** et **SEQ ID N°53** à **SEQ ID N°57,** les variants VanC4 sont caractérisés par la détection d'au moins un peptide appartenant à la protéine VanC4 et à ses différents variants de séquences **SEQ ID n°44, SEQ ID n°45, SEQ ID n°52** et **SEQ ID n°58**
**SEQ ID N°36** :
**SEQ ID N°37** :
**SEQ ID N°38** :
**SEQ ID N°39** :
**SEQ ID N°40 :**
**SEQ ID N°41 :**
**SEQ ID N°42 :**
**SEQ ID N°43** :
**SEQ ID N°44** :
**SEQ ID N°45** :
**SEQ ID N°46** :
**SEQ ID N°47** :
**SEQ ID N°48** :
**SEQ ID N°49 :**
**SEQ ID N°50 :**
**SEQ ID N°51 :**
**SEQ ID N°52** :
**SEQ ID N°53** :
**SEQ ID N°54** :
**SEQ ID N°55** :
**SEQ ID N°56** :
**SEQ ID N°57** :
**SEQ ID N°58** :
lesdits marqueurs de type VanC étant choisis, de préférence, parmi les peptides de séquence **SEQ ID N°59** à **SEQ ID N°71** tels que définis ci-après :

| **Peptide SEQ ID N°** | **Séquence d'acides aminés** | **Position du peptide dans la ou les protéines VanC** |
|---|---|---|
| **SEQ ID N°59** | EQAQLLYR | 279-286 pour les protéines de SEQ N°46, 47, 48, 49, 50, 51, 53, 54, 55, 56, 57; 272-279 pour la protéine de séquence SEQ ID N°36; 272-279 pour la protéine de séquence SEQ ID N°37; 272-279 pour la protéine de séquence SEQ ID N°38; 272-279 pour la protéine de séquence SEQ ID N°39; 272-279 pour la protéine de séquence SEQ ID N°40; 272-279 pour la protéine de séquence SEQ ID N°41; 273-280 pour la protéine de séquence SEQ ID N°42; 273-280 pour la protéine de séquence SEQ ID N°43 |
| **SEQ ID N°60** | FIQDHGFPIFIKPN EAGSSK | 164-183 pour les protéines de SEQ N°42, 43; 163-182 pour la protéine de séquence SEQ ID N°36; 163-182 pour la protéine de séquence SEQ ID N°37; 163-182 pour la protéine de séquence SEQ ID N°38; 163-182 pour la protéine de séquence SEQ ID N°39; 163-182 pour la protéine de séquence SEQ ID N°40; 163-182 pour la protéine de séquence SEQ ID N°41 |
| **SEQ ID N°61** | IVPDVLFPVLHG K | 87-99 pour les protéines de SEQ N°42, 43; 86-98 pour la protéine de séquence SEQ ID N°36; 86-98 pour la protéine de séquence SEQ ID N°37; 86-98 pour la protéine de séquence SEQ ID N°38; 86-98 pour la protéine de séquence SEQ ID N°39; 86-98 pour la protéine de séquence SEQ ID N°40; 86-98 pour la protéine de séquence SEQ ID N°41 |
| **SEQ ID N°62** | NCHQLTFSSQGFI LGEK | 69-85 pour les protéines de SEQ N°42, 43; 68-84 pour la protéine de séquence SEQ ID N°36; 68-84 pour la protéine de séquence SEQ ID N°37; 68-84 pour la protéine de séquence SEQ ID N°38; 68-84 pour la protéine de séquence SEQ ID N°39; 68-84 pour la protéine de séquence SEQ ID N°40; 68-84 pour la protéine de séquence SEQ ID N°41 |
| **SEQ ID N°63** | NDTWLEDHK | 60-68 pour les protéines de SEQ N°42, 43; 59-67 pour la protéine de séquence SEQ ID N°36; 59-67 pour la protéine de séquence SEQ ID N°37; 59-67 pour la protéine de séquence SEQ ID N°38; 59-67 pour la protéine de séquence SEQ ID N°39; 59-67 pour la protéine de séquence SEQ ID N°40; 59-67 pour la protéine de séquence SEQ ID N°41 |
| **SEQ ID N°64** | NLGLTGLAR | 281-289 pour les protéines de SEQ N°42, 43; 280-288 pour la protéine de séquence SEQ ID N°36; 280-288 pour la protéine de séquence SEQ ID N°37; 280-288 pour la protéine de séquence SEQ ID N°38; 280-288 pour la protéine de séquence SEQ ID N°39; 280-288 pour la protéine de séquence SEQ ID N°40; 280-288 pour la protéine de séquence SEQ ID N°41 |
| **SEQ ID N°65** | TALQSALTTAFA YGSTVLIQK | 192-212 pour les protéines de SEQ N°42, 43; 191-211 pour la protéine de séquence SEQ ID N°36; 191-211 pour la protéine de séquence SEQ ID N°37; 191-211 pour la protéine de séquence SEQ ID N°38; 191-211 pour la protéine de séquence SEQ ID N°39; 191-211 pour la protéine de séquence SEQ ID N°40; 191-211 pour la protéine de séquence SEQ ID N°41 |
| **SEQ ID N°66** | WLLHQLADTMG IASAPTLLLSR | 132-153 pour les protéines de SEQ N°42, 43; 131-152 pour la protéine de séquence SEQ ID N°36; 131-152 pour la protéine de séquence SEQ ID N°37; 131-152 pour la protéine de séquence SEQ ID N°38; 131-152 pour la protéine de séquence SEQ ID N°39; 131-152 pour la protéine de séquence SEQ ID N°40; 131-152 pour la protéine de séquence SEQ ID N°41 |
| **SEQ ID N°67** | YENDPATIDR | 154-163 pour les protéines de SEQ N°42, 43; 153-162 pour la protéine de séquence SEQ ID N°36; 153-162 pour la protéine de séquence SEQ ID N°37; 153-162 pour la protéine de séquence SEQ ID N°38; 153-162 pour la protéine de séquence SEQ ID N°39; 153-162 pour la protéine de séquence SEQ ID N°40; 153-162 pour la protéine de séquence SEQ ID N°41 |
| **SEQ ID N°68** | YQLISATITVPAP LPLALESQIK | 250-272 pour les protéines de SEQ N°42, 43; 249-271 pour la protéine de séquence SEQ ID N°36; 249-271 pour la protéine de séquence SEQ ID N°37; 249-271 pour la protéine de séquence SEQ ID N°38; 249-271 pour la protéine de séquence SEQ ID N°39; 249-271 pour la protéine de séquence SEQ ID N°40; 249-271 pour la protéine de séquence SEQ ID N°41 |
| **SEQ ID N°69** | ITVPAPLPETIET K | 263-276 pour les protéines de SEQ N°44, 45, 46, 47, 48, 49, 50, 51, 52, 53, 54, 55, 56, 57, 58 |
| **SEQ ID N°70** | QDTWLLDTK | 62-70 pour les protéines de SEQ N°44, 45, 46, 47, 48, 49, 50, 51, 52, 53, 54, 55, 56, 57, 58 |
| **SEQ ID N°71** | YQLISAK | 256-262 pour les protéines de SEQ N°44, 45, 46, 47, 48, 49, 50, 51, 52, 53, 54, 55, 56, 57, 58 |

Selon un autre mode de réalisation de l'invention, la détection d'un mécanisme de résistance aux glycopeptides, lié à l'expression de la protéine VanD, est caractérisée par la détection d'au moins un peptide appartenant à la protéine VanD et à ses différents variants de séquences **SEQ ID N° 72** à **SEQ ID N° 79.**
**SEQ ID N°72** :
**SEQ ID N°73** :
**SEQ ID N°74** :
**SEQ ID N°75** :
**SEQ ID N°76** :
**SEQ ID N°77** :
**SEQ ID N°78** :
**SEQ ID N°79** :
lesdits peptides de type VanD étant choisis, de préférence, parmi les peptides de séquence **SEQ ID N°80** à **SEQ ID N°83** tels que définis ci-après :

| **Peptide SEQ ID N°** | **Séquence d'acides aminés** | **Position du peptide dans la ou les protéines VanD** |
|---|---|---|
| **SEQ ID N°80** | GSENAVIR | 252-259 pour les protéines de SEQ N°72, 73, 74, 75, 76, 77, 78 |
| **SEQ ID N°81** | IDLFLR | 291-296 pour les protéines de SEQ N°72, 73, 74, 75, 76, 77, 78, 79 |
| **SEQ ID N°82** | IHQEAQPEK | 243-251 pour les protéines de SEQ N°72, 73, 74, 75, 76, 77, 78, 79 |
| **SEQ ID N°83** | SGSSFGVNK | 175-183 pour les protéines de SEQ N°72, 73, 74, 75, 76, 77, 78, 79 |

Avantageusement, les marqueurs de type VanD choisis parmi les peptides de séquence SEQ ID N° 80, SEQ ID N° 81 et SEQ ID N° 83 sont détectés après induction du mécanisme de résistance.

Avantageusement, les marqueurs de type VanD choisis parmi les peptides de séquence SEQ ID N° 80, SEQ ID N° 81 et SEQ ID N° 83 sont détectés sans étape d'induction.

Selon un autre mode de réalisation de l'invention, la détection d'un mécanisme de résistance aux glycopeptides, induit par l'expression de la protéine VanE, est caractérisée par la détection d'au moins un peptide appartenant à la protéine VanE de séquence **SEQ ID N° 84.**
**SEQ ID N°84** : lesdits marqueurs de type VanE étant choisis, de préférence, parmi les peptides de séquence **SEQ ID N°85** à **SEQ ID N°91** tels que définis ci-après :

| **Peptide SEQ ID N°** | **Séquence d'acides aminés** | **Position du peptide dans la ou les protéines VanE** |
|---|---|---|
| **SEQ ID N°85** | AIDEASK | 198-204 pour la protéine de SEQ N°84 |
| **SEQ ID N°86** | FPMMMNEIGMDYK | 318-330 pour la protéine de SEQ N°84 |
| **SEQ ID N°87** | IMLHQFAEAIGVK | 134-146 pour la protéine de SEQ N°84 |
| **SEQ ID N°88** | NMESIDYNVMK | 28-38 pour la protéine de SEQ N°84 |
| **SEQ ID N°89** | SAVAIIK | 21-27 pour la protéine de SEQ N°84 |
| **SEQ ID N°90** | STPSMIIEK | 147-155 pour la protéine de SEQ N°84 |
| **SEQ ID N°91** | YNLVTAEILLPAK | 251-263 pour la protéine de SEQ N°84 |

Avantageusement, le marqueur de type VanE correspondant au peptide de séquence SEQ ID N° 89 est détecté après induction du mécanisme de résistance.

Selon un autre mode de réalisation de l'invention, la détection d'un mécanisme de résistance aux glycopeptides, lié à l'expression de la protéine VanG, est caractérisée par la détection d'au moins un peptide appartenant à la protéine VanG et à ses différents variants de séquences **SEQ ID N° 92** à **SEQ ID N° 94.**
**SEQ ID N°92** :
**SEQ ID N°93** :
**SEQ ID N°94** :
lesdits marqueurs de type VanG étant choisis, de préférence, parmi les peptides de séquence **SEQ ID N°95** à **SEQ ID N°102** tels que définis ci-après :

| **Peptide SEQ ID N°** | **Séquence d'acides aminés** | **Position du peptide dans la ou les protéines VanG** |
|---|---|---|
| **SEQ ID N°95** | AGSSFGITK | 187-195 pour les protéines de SEQ N°92, 93, 94 |
| **SEQ ID N°96** | ALGCSGFSR | 289-297 pour les protéines de SEQ N°92, 93, 94 |
| **SEQ ID N°97** | IDAEAEK | 271-277 pour les protéines de SEQ N°92, 93, 94 |
| **SEQ ID N°98** | IYMPAR | 265-270 pour les protéines de SEQ N°92, 93, 94 |
| **SEQ ID N°99** | LIGLYVE | 343-349 pour les protéines de SEQ N°92, 93, 94 |
| **SEQ ID N°100** | L VSLAGISVPK | 146-156 pour les protéines de SEQ N°92, 93, 94 |
| **SEQ ID N°101** | VDEIELSSGFFDYTEK | 242-257 pour les protéines de SEQ N°92, 93, 94 |
| **SEQ ID N°102** | YPNMMK | 325-330 pour les protéines de SEQ N°92, 93, 94 |

Avantageusement, les marqueurs de type VanG choisis parmi les peptides de séquence SEQ ID N° 95, SEQ ID N° 96, SEQ ID N° 97, SEQ ID N° 98, SEQ ID N° 100 et SEQ ID N° 102 sont détectés après induction du mécanisme de résistance.

Avantageusement, les marqueurs de type VanG choisis parmi les peptides de séquence SEQ ID N° 97 et SEQ ID N° 100 sont détectés sans étape d'induction.

Selon un autre mode de réalisation de l'invention, la détection d'un mécanisme de résistance aux glycopeptides, induit par l'expression de la protéine VanL, est caractérisée par la détection d'au moins un peptide appartenant à la protéine VanL de séquence **SEQ ID N° 103.**
**SEQ ID N°103 :** lesdits peptides de type VanL étant choisis, de préférence, parmi les peptides de séquence **SEQ ID N°104** à **SEQ ID N°120** tels que définis ci-après :

| **Peptide SEQ ID N°** | **Séquence d'acides aminés** | **Position du peptide dans la ou les protéines VanL** |
|---|---|---|
| **SEQ ID N°104** | DIEYDVWQTDPSLQEIIPCFNNR | 59-81 pour la protéine de SEQ N°103 |
| **SEQ ID N°105** | EIGCAVLGNEK | 222-232 pour la protéine de SEQ N°103 |
| **SEQ ID N°106** | IAIIFGGQSSEYEVSLK | 7-23 pour la protéine de SEQ N°103 |
| **SEQ ID N°107** | IGIDLGGK | 42-49 pour la protéine de SEQ N°103 |
| **SEQ ID N°108** | LEDALTEAFK | 198-207 pour la protéine de SEQ N°103 |
| **SEQ ID N°109** | LINLAEEK | 339-346 pour la protéine de SEQ N°103 |
| **SEQ ID N°110** | LLGCSGLAR | 285-293 pour la protéine de SEQ N°103 |
| **SEQ ID N°111** | LLVGECDEVSLNSDFFDYTEK | 233-253 pour la protéine de SEQ N°103 |
| **SEQ ID N°112** | MMEAVGVTYK | 324-333 pour la protéine de SEQ N°103 |
| **SEQ ID N°113** | NDFPIFVKPNEAGSSK | 172-187 pour la protéine de SEQ N°103 |
| **SEQ ID N°114** | QAQLLYR | 278-284 pour la protéine de SEQ N°103 |
| **SEQ ID N°115** | SAPTLIIR | 150-157 pour la protéine de SEQ N°103 |
| **SEQ ID N°116** | STVSVLETLSTCNFEIIK | 24-41 pour la protéine de SEQ N°103 |
| **SEQ ID N°117** | VNIPASISVEFSNEMK | 261-276 pour la protéine de SEQ N°103 |
| **SEQ ID N°118** | WYLTTSNNK | 50-58 pour la protéine de SEQ N°103 |
| **SEQ ID N°119** | YLLHEFAQSVGVK | 137-149 pour la protéine de SEQ N°103 |
| **SEQ ID N°120** | YQMISAK | 254-260 pour la protéine de SEQ N°103 |

Selon un autre mode de réalisation de l'invention, la détection d'un mécanisme de résistance aux glycopeptides, induit par l'expression de la protéine VanM, est caractérisée par la détection d'au moins un peptide appartenant à la protéine VanM de séquence **SEQ ID N° 121.**
**SEQ ID N°121** : lesdits peptides de type VanM étant choisis, de préférence, parmi les peptides de séquence **SEQ ID N°122** à **SEQ ID N°139** tels que définis ci-après :

| **Peptide SEQ ID N°** | **Séquence d'acides aminés** | **Position du peptide dans la ou les protéines VanM** |
|---|---|---|
| **SEQ ID N°122** | DDKPAADSFTYPVFVKPAR | 156-174 pour la protéine de SEQ N°121 |
| **SEQ ID N°123** | GSENATITVPAELSAEER | 252-269 pour la protéine de SEQ N°121 |
| **SEQ ID N°124** | IAILFGGCSEEHNVSVK | 6-22 pour la protéine de SEQ N°121 |
| **SEQ ID N°125** | IDVVFSVLHGK | 91-101 pour la protéine de SEQ N°121 |
| **SEQ ID N°126** | IHQEAEPEK | 243-251 pour la protéine de SEQ N°121 |
| **SEQ ID N°127** | IVLNEVNTMPGFTSYSR | 301-317 pour la protéine de SEQ N°121 |
| **SEQ ID N°128** | LQHGIFR | 236-242 pour la protéine de SEQ N°121 |
| **SEQ ID N°129** | MHGLLIMQDK | 75-84 pour la protéine de SEQ N°121 |
| **SEQ ID N°130** | MMVSAGITIPELIDHLIVLAVK | 321-342 pour la protéine de SEQ N°121 |
| **SEQ ID N°131** | NAGIATPEFQVIYK | 142-155 pour la protéine de SEQ N°121 |
| **SEQ ID N°132** | SAAEIANNIDIGK | 23-35 pour la protéine de SEQ N°121 |
| **SEQ ID N°133** | SAVLSPDK | 66-73 pour la protéine de SEQ N°121 |
| **SEQ ID N°134** | SGSSYGVNK | 175-183 pour la protéine de SEQ N°121 |
| **SEQ ID N°135** | SLAYIIAK | 134-141 pour la protéine de SEQ N°121 |
| **SEQ ID N°136** | TCEKPCIDWDNEHCR | 51-65 pour la protéine de SEQ N°121 |
| **SEQ ID N°137** | VDMFLQDNGR | 291-300 pour la protéine de SEQ N°121 |
| **SEQ ID N°138** | VNSADELDSAIDLAR | 184-198 pour la protéine de SEQ N°121 |
| **SEQ ID N°139** | YEPIYIGITQSGVWK | 36-50 pour la protéine de SEQ N°121 |

Selon un autre mode de réalisation de l'invention, la détection d'un mécanisme de résistance aux glycopeptides, induit par l'expression de la protéine VanN, est caractérisée par la détection d'au moins un peptide appartenant à la protéine VanN de séquence **SEQ ID N° 140.**
**SEQ ID N°140** : lesdits peptides de type VanN étant choisis, de préférence, parmi les peptides de séquence **SEQ ID N°141** à **SEQ ID N°154** tels que définis ci-après :

| **Peptide SEQ ID N°** | **Séquence d'acides aminés** | **Position du peptide dans la protéine VanN** |
|---|---|---|
| **SEQ ID N°141** | ALTEAFQFSQTVILQK | 198-213 pour la protéine de SEQ N°140 |
| **SEQ ID N°142** | GFYDQAEK | 79-86 pour la protéine de SEQ N°140 |
| **SEQ ID N°143** | IALIFGGTSAEYEVSLK | 4-20 pour la protéine de SEQ N°140 |
| **SEQ ID N°144** | IGIASNGK | 39-46 pour la protéine de SEQ N°140 |
| **SEQ ID N°145** | IPVATSR | 264-270 pour la protéine de SEQ N°140 |
| **SEQ ID N°146** | LNEITPSFDGR | 68-78 pour la protéine de SEQ N°140 |
| **SEQ ID N°147** | NFTELYGFPIFIKPNEAGSSK | 164-184 pour la protéine de SEQ N°140 |
| **SEQ ID N°148** | QAQLLYQLLGCQGLAR | 275-290 pour la protéine de SEQ N°140 |
| **SEQ ID N°149** | SAASVLSVLENLNVEIYR | 21-38 pour la protéine de SEQ N°140 |
| **SEQ ID N°150** | SVGVMSTPTQLISSTDEQQVIK | 142-163 pour la protéine de SEQ N°140 |
| **SEQ ID N°151** | VHTEAELTK | 189-197 pour la protéine de SEQ N°140 |
| **SEQ ID N°152** | WYLTFSDNETIANDLWLQDK | 47-66 pour la protéine de SEQ N°140 |
| **SEQ ID N°153** | YLLHQFAK | 134-141 pour la protéine de SEQ N°140 |
| **SEQ ID N°154** | YQMTTAK | 251-257 pour la protéine de SEQ N°140 |

Le procédé de l'invention et ses avantages ressortiront de la suite de la présente description qui présente plusieurs exemples, non limitatifs, de mise en oeuvre dudit procédé.

### Exemple 1 : Préparation d'un échantillon primaire urinaire par enrichissement en microorganismes:

Le protocole suivant est mis en oeuvre en 16 étapes (les étapes 5 à 12 sont optionnelles et pourraient être omises si l'échantillon enrichi est ultérieurement traité selon les exemples 6 et suivants) :
1. Centrifugation de 5 mL d'urine contaminée, à 2000g pendant 30 secondes
2. Récupération du surnageant
3. Centrifugation à 15000g pendant 5 minutes
4. Elimination du surnageant
5. Lavage du culot avec 3 mL d'eau distillée par remise en suspension
6. Centrifugation à 15000g pendant 5 minutes
7. Elimination du surnageant
8. Mettre le culot en présence de solvant (8 volumes acétone pour 1 volume de méthanol) en dilution au 1/10
9. Laisser 1 heure à -20°C
10. Centrifugation à 15000g pendant 5 minutes
11. Elimination du surnageant
12. Mettre le culot en présence de solvant (8 volumes acétone pour 1 volume de méthanol) en dilution au 1/10
13. Laisser 1 heure à -20°C
14. Centrifugation à 15000g pendant 5 minutes
15. Elimination du surnageant
Le culot constitue l'échantillon enrichi en microorganisme

### Exemple 2 : Mise en culture de l'échantillon sur milieu de culture en absence d'antibiotique :

Les milieux de culture optimaux et les conditions de culture optimales sont différents selon les espèces de microorganisme. Par défaut l'échantillon est ensemencé sur différents milieux :
∘ gélose Columbia au sang de mouton (référence bioMérieux 43041) pendant 18 à 24 h à 35°C, en atmosphère aérobie ou anaérobie ;
∘ gélose TSA (référence bioMérieux 43011) pendant 18 à 24 h à 37°C.

### Exemple 3 : Mise en culture de l'échantillon sur milieu de culture en présence d'antibiotique :

L'échantillon est ensemencé sur milieu :
∘ gélose VRE (référence bioMérieux 43002) pendant 18 à 24 h à 35°C, en atmosphère aérobie ou anaérobie. Ce milieu contient de la vancomycine.

### Exemple 4 : Identification de microorganismes par profil biochimique :

L'identification est mise en oeuvre comme suit :
1. Sélection de colonies isolées obtenues selon l'exemple 2 ou l'exemple 3, ou par enrichissement de microorganismes présents dans un échantillon primaire selon l'exemple 1.
2. En respectant les conditions d'asepsie, transfert de 3,0 mL de solution saline stérile aqueuse (à 0,45-0,50 % de NaCl, de pH 4,5 à 7,0) dans un tube à essai en plastique transparent (polystyrène)
3. A l'aide d'un bâtonnet ou d'un écouvillon stérile, transfert d'un nombre suffisant de colonies identiques dans le tube de solution saline préparé à l'étape 2 et ajustement de la suspension bactérienne entre 0,50 et 0,63 McFarland avec un DENSICHEK étalonné du VITEK®
4. Positionnement du tube de suspension bactérienne et d'une carte d'identification VITEK® sur une cassette VITEK®
5. Chargement de la cassette dans l'instrument VITEK®
6. Les opérations de remplissage, scellage, incubation et lecture sont automatiques
7. Acquisition d'un profil biochimique
Identification avec le système VITEK® réalisée par comparaison à des profils biochimiques de souches connues

### Exemple 5 : Identification de microorganismes à partir d'un échantillon par MALDI-TOF

L'identification est mise en oeuvre comme suit :
1. Transfert, à l'aide d'une oese de 1µl, d'une portion de colonie de microorganisme obtenue selon l'exemple 2 ou 3, ou d'un échantillon enrichi selon l'exemple 1, et dépôt uniforme sur une plaque pour spectrométrie de masse par MALDI-TOF
2. Recouvrement du dépôt avec 1 µl de matrice. La matrice utilisée est une solution saturée d'HCCA (acide alpha-cyano-4-hydroxycinnamique) en solvant organique (50% d'acétonitrile et 2,5% d'acide trifluoroacétique)
3. Séchage à température ambiante
4. Introduction de la plaque dans le spectromètre de masse
5. Acquisition d'un spectre de masse
6. Comparaison du spectre obtenu avec les spectres contenus dans une base de connaissance
7. Identification du microorganisme par comparaison des pics obtenus à ceux de la base de connaissance

### Exemple 6 : Identification de microorganismes à partir d'un échantillon par ESI-MS

L'identification est mise en oeuvre comme suit :
1. Prélèvement d'une colonie de microorganisme, obtenue selon l'exemple 2 ou 3, ou d'un échantillon enrichi selon l'exemple 1, et mise en suspension dans 100µl d'eau déminéralisée.
2. Centrifugation à 3000g pendant 5 minutes.
3. Elimination du surnageant.
4. Remise en suspension dans 100µl d'eau déminéralisée.
5. Centrifugation à 3000g pendant 5 minutes.
6. Elimination du surnageant.
7. Remise en suspension dans 100µl d'un mélange Acétonitrile, eau déminéralisée, acide formique (50/50/0.1%).
8. Filtration avec un filtre de porosité 0.45 µm.
9. Injection dans un spectromètre de masse en mode MS simple.
10. Acquisition d'un spectre de masse.
11. Comparaison du spectre obtenu avec les spectres contenus dans une base de connaissance.
12. Identification du microorganisme par référence à des spectres de référence.

### Exemple 7 : Obtention de protéines digérées à partir de microorganismes

Le protocole suivant est mis en oeuvre en 17 étapes :
1. Prélèvement d'une colonie de microorganisme, obtenue selon l'exemple 2 ou 3, ou d'un échantillon enrichi selon l'exemple 1, et mise en suspension dans 10 à 100µl d'une solution d'hydrochlorure de guanidine 6M, 50 mM Tris-HCl, pH=8,0.
2. Ajout de dithiothréitol (DTT) pour obtenir une concentration finale de 5mM.
3. Réduction pendant 20 minutes à 95°C dans un bain-marie.
4. Refroidissement des tubes à température ambiante.
5. Ajout d'iodoacétamide pour obtenir une concentration finale de 12.5 mM.
6. Alkylation pendant 40 minutes à température ambiante et à l'obscurité.
7. Dilution d'un facteur 6 avec une solution de NH₄HCO₃ 50 mM, pH=8.0 pour obtenir une concentration finale en hydrochlorure de guanidine de 1M.
8. Ajout de 1 µg de trypsine.
9. Digestion à 37°C pendant 6 heures jusqu'à une nuit.
10. Ajout d'acide formique jusqu'à un pH inférieur à 4 pour stopper la réaction.
11. Le volume d'échantillon est complété à 1mL avec eau/acide formique 0,5% (v/v)
12. Equilibrage des colonnes HLB Oasis Waters avec 1ml de méthanol puis 1 ml H₂O/acide formique 0,1% (v/v)
13. Dépôt de l'échantillon qui s'écoule par gravité
14. Lavage avec 1 ml H₂O/acide formique 0,1% (v/v)
15. Elution avec 1ml d'un mélange 80% de méthanol et 20% d'eau/acide formique 0,1% (v/v)
16. L'éluat est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.
L'éluat est ensuite repris dans une solution eau/acide formique 0,5% (v/v) quantité suffisante pour (QSP) 200µl

### Exemple 8 : Identification de résistance aux glycopeptides, sans induction, de type VanA, VanB, VanC1, VanD, VanE et VanG:

Les échantillons Ech1 à Ech13 sont identifiés selon l'une des méthodes décrites dans les exemples 1 à 6. L'identification des espèces est reportée dans le **TABLEAU 1.**

**TABLEAU1 :**

| **Nom** | **Espèces** |
|---|---|
| Ech1 | *E. faecalis* |
| Ech2 | *E. gallinarum* |
| Ech3 | *E. faecalis* |
| Ech4 | *E. faecalis* |
| Ech5 | *E. faecalis* |
| Ech6 | *E. faecalis* |
| Ech7 | *E. faecalis* |
| Ech8 | *E. faecium* |
| Ech9 | *E. faecium* |
| Ech10 | *E. faecalis* |
| Ech11 | *E. faecium* |

Les échantillons Ech1 à Ech11 correspondent à une espèce pouvant comporter un mécanisme de résistance de type Van. La méthode suivante est alors mise en oeuvre pour rechercher un tel mécanisme.

La colonie de microorganisme est obtenue selon l'exemple 2, traitée selon l'exemple 7, puis un volume de 50µl de protéines digérées est injecté et analysé selon les conditions suivantes :
- Chaîne chromatographique Dionex Ultimate 3000 de la société Dionex Corporation (Sunnyvale, Etats Unis d'Amerique).
- Colonne Waters BEH130 C18, 2,1mm de diamètre interne, 100mm de long, taille de particule de 3,5µm (Waters, Saint-Quentin en Yvelines, France).
- Solvant A : H₂O + 0,1% acide formique.
- Solvant B : ACN + 0,1% acide formique.

Gradient HPLC défini dans le **TABLEAU 2** ci-après.

**TABLEAU 2 :**

| Temps (min) | Débit (µl) | Solvant A (%) | Solvant B (%) |
|---|---|---|---|
| 0 | 300 | 98 | 2 |
| 3 | 300 | 98 | 2 |
| 28 | 300 | 63 | 37 |
| 30 | 300 | 0 | 100 |
| 38 | 300 | 0 | 100 |
| 38.1 | 300 | 98 | 2 |
| 45 | 300 | 98 | 2 |

- L'éluat en sortie de colonne chromatographique est directement injecté dans la source d'ionisation du spectromètre de masse de type QTRAP® 5500 de la société Applied Biosystems (Foster City, Etats Unis d'Amérique).
- Les peptides, issus de la digestion des protéines du microorganisme, sont analysés par le spectromètre de masse en mode MRM. Seuls les peptides indiqués dans le **TABLEAU 3** sont détectés. Pour cela, le ou les fragments indiqués dans le **TABLEAU 3** sont détectés.

**TABLEAU 3 :**

| **Transition numéro** | **Protéine** | **Peptide** | **Etat de charge du précurseur** | **Ion fragment de première génération** |
|---|---|---|---|---|
| 1 | VanA | IHQEVEPEK | 2 | y5 monochargé |
| 2 | VanA | IHQEVEPEK | 2 | y6 monochargé |
| 3 | VanA | IHQEVEPEK | 2 | y7 monochargé |
| 4 | VanA | LIVLALK | 2 | y4 monochargé |
| 5 | VanA | LIVLALK | 2 | y5 monochargé |
| 6 | VanA | LIVLALK | 2 | y6 monochargé |
| 7 | VanA | LQYGIFR | 2 | y4 monochargé |
| 8 | VanA | LQYGIFR | 2 | y5 monochargé |
| 9 | VanA | LQYGIFR | 2 | y6 monochargé |
| 10 | VanA | MHGLLVK | 2 | b5 monochargé |
| 11 | VanA | MHGLLVK | 2 | y5 monochargé |
| 12 | VanA | MHGLLVK | 2 | y6 monochargé |
| 13 | VanA | MMAAAGIALPELIDR | 2 | y6 monochargé |
| 14 | VanA | MMAAAGIALPELIDR | 2 | y7 monochargé |
| 15 | VanA | MMAAAGIALPELIDR | 2 | y8 monochargé |
| 16 | VanA | NAGIATPAFWVINK | 2 | y10 monochargé |
| 17 | VanA | NAGIATPAFWVINK | 2 | y8 monochargé |
| 18 | VanA | NAGIATPAFWVINK | 2 | y9 monochargé |
| 19 | VanA | SAIEIAANINK | 2 | y6 monochargé |
| 20 | VanA | SAIEIAANINK | 2 | y7 monochargé |
| 21 | VanA | SAIEIAANINK | 2 | y8 monochargé |
| 22 | VanA | SGSSFGVK | 2 | y5 monochargé |
| 23 | VanA | SGSSFGVK | 2 | y6 monochargé |
| 24 | VanA | SGSSFGVK | 2 | y7 monochargé |
| 25 | VanA | SLTYIVAK | 2 | y4 monochargé |
| 26 | VanA | SLTYIVAK | 2 | y5 monochargé |
| 27 | VanA | SLTYIVAK | 2 | y6 monochargé |
| 28 | VanA | VDMFLQDNGR | 2 | y6 monochargé |
| 29 | VanA | VDMFLQDNGR | 2 | y7 monochargé |
| 30 | VanA | VDMFLQDNGR | 2 | y8 monochargé |
| 31 | VanA | VNSADELDYAIESAR | 2 | y6 monochargé |
| 32 | VanA | VNSADELDYAIESAR | 2 | y7 monochargé |
| 33 | VanA | VNSADELDYAIESAR | 2 | y8 monochargé |
| 34 | VanA | YEPLYIGITK | 2 | y7 monochargé |
| 35 | VanA | YEPLYIGITK | 2 | y8 monochargé |
| 36 | VanA | YEPLYIGITK | 2 | y8 dichargé |
| 37 | VanB | FDPHYIGITK | 2 | y6 monochargé |
| 38 | VanB | FDPHYIGITK | 2 | y7 dichargé |
| 39 | VanB | FDPHYIGITK | 2 | y8 dichargé |
| 40 | VanB | IDVAFPVLHGK | 2 | y6 monochargé |
| 41 | VanB | IDVAFPVLHGK | 2 | y7 monochargé |
| 42 | VanB | IDVAFPVLHGK | 2 | y8 monochargé |
| 43 | VanB | IHQENEPEK | 2 | y5 monochargé |
| 44 | VanB | IHQENEPEK | 2 | y6 monochargé |
| 45 | VanB | IHQENEPEK | 2 | y7 monochargé |
| 46 | VanB | LSHGIFR | 2 | y4 monochargé |
| 47 | VanB | LSHGIFR | 2 | y5 monochargé |
| 48 | VanB | LSHGIFR | 2 | y6 monochargé |
| 49 | VanB | SLAYILTK | 2 | y5 monochargé |
| 50 | VanB | SLAYILTK | 2 | y6 monochargé |
| 51 | VanB | SLAYILTK | 2 | y7 monochargé |
| 52 | VanB | THGLLVMK | 2 | b5 monochargé |
| 53 | VanB | THGLLVMK | 2 | b6 monochargé |
| 54 | VanB | THGLLVMK | 2 | y6 monochargé |
| 55 | VanB | VQETAK | 2 | y3 monochargé |
| 56 | VanB | VQETAK | 2 | y4 monochargé |
| 57 | VanB | VQETAK | 2 | y5 monochargé |
| 58 | VanC1 | EQAQLL YR | 2 | y3 monochargé |
| 59 | VanC1 | EQAQLL YR | 2 | y4 monochargé |
| 60 | VanC1 | EQAQLL YR | 2 | y6 monochargé |
| 61 | VanC1 | FIQDHGFPIFIKPNEAGSSK | 3 | b7 monochargé |
| 62 | VanC1 | FIQDHGFPIFIKPNEAGSSK | 3 | y8 monochargé |
| 63 | VanC1 | FIQDHGFPIFIKPNEAGSSK | 3 | y9 monochargé |
| 64 | VanC1 | IVPDVLFPVLHGK | 2 | y11 dichargé |
| 65 | VanC1 | IVPDVLFPVLHGK | 3 | y11 dichargé |
| 66 | VanC1 | IVPDVLFPVLHGK | 3 | y11 trichargé |
| 67 | VanC1 | NC[CAM]HQL TFSSQGFILGEK | 3 | y5 monochargé |
| 68 | VanC1 | NC[CAM]HQL TFSSQGFILGEK | 3 | y7 monochargé |
| 69 | VanC1 | NC[CAM]HQL TFSSQGFILGEK | 3 | y8 monochargé |
| 70 | VanC1 | NDTWLEDHK | 2 | y5 monochargé |
| 71 | VanC1 | NDTWLEDHK | 2 | y6 monochargé |
| 72 | VanC1 | NDTWLEDHK | 2 | y7 monochargé |
| 73 | VanC1 | NLGLTGLAR | 2 | y4 monochargé |
| 74 | VanC1 | NLGLTGLAR | 2 | y5 monochargé |
| 75 | VanC1 | NLGLTGLAR | 2 | y7 monochargé |
| 76 | VanC1 | TALQSALTTAFAYGSTVLIQK | 3 | y7 monochargé |
| 77 | VanC1 | TALQSALTTAFAYGSTVLIQK | 3 | y8 monochargé |
| 78 | VanC1 | TALQSALTTAFAYGSTVLIQK | 3 | y9 monochargé |
| 79 | VanC1 | WLLHQLADTMGIASAPTLLLSR | 3 | y10 monochargé |
| 80 | VanC1 | WLLHQLADTMGIASAPTLLLSR | 3 | y7 monochargé |
| 81 | VanC1 | WLLHQLADTMGIASAPTLLLSR | 3 | y9 monochargé |
| 82 | VanC1 | YENDPATIDR | 2 | b4 monochargé |
| 83 | VanC1 | YENDPATIDR | 2 | y6 monochargé |
| 84 | VanC1 | YENDPATIDR | 2 | y8 monochargé |
| 85 | VanC1 | YQLISATITVPAPLPLALESQIK | 3 | y11 dichargé |
| 86 | VanC1 | YQLISATITVPAPLPLALESQIK | 3 | y13 dichargé |
| 87 | VanC1 | YQLISATITVPAPLPLALESQIK | 3 | y9 monochargé |
| 88 | VanC2 | ITVPAPLPETIETK | 2 | y11 dichargé |
| 89 | VanC2 | ITVPAPLPETIETK | 2 | y7 monochargé |
| 90 | VanC2 | ITVPAPLPETIETK | 2 | y9 monochargé |
| 91 | VanC2 | QDTWLLDTK | 2 | y4 monochargé |
| 92 | VanC2 | QDTWLLDTK | 2 | y5 monochargé |
| 93 | VanC2 | QDTWLLDTK | 2 | y6 monochargé |
| 94 | VanC2 | YQLISAK | 2 | y3 monochargé |
| 95 | VanC2 | YQLISAK | 2 | y5 monochargé |
| 96 | VanC2 | YQLISAK | 2 | y6 monochargé |
| 97 | VanD | GSENAVIR | 2 | y4 monochargé |
| 98 | VanD | GSENAVIR | 2 | y5 monochargé |
| 99 | VanD | GSENAVIR | 2 | y6 monochargé |
| 100 | VanD | IDLFLR | 2 | y3 monochargé |
| 101 | VanD | IDLFLR | 2 | y4 monochargé |
| 102 | VanD | IDLFLR | 2 | y5 monochargé |
| 103 | VanD | IHQEAQPEK | 2 | y3 monochargé |
| 104 | VanD | IHQEAQPEK | 2 | y7 monochargé |
| 105 | VanD | IHQEAQPEK | 2 | y8 dichargé |
| 106 | VanD | SGSSFGVNK | 2 | y5 monochargé |
| 107 | VanD | SGSSFGVNK | 2 | y6 monochargé |
| 108 | VanD | SGSSFGVNK | 2 | y7 monochargé |
| 109 | VanE | AIDEASK | 2 | y4 monochargé |
| 110 | VanE | AIDEASK | 2 | y5 monochargé |
| 111 | VanE | AIDEASK | 2 | y6 monochargé |
| 112 | VanE | FPMMMNEIGMDYK | 2 | y9 monochargé |
| 113 | VanE | FPMMMNEIGMDYK | 2 | y12 dichargé |
| 114 | VanE | FPMMMNEIGMDYK | 2 | y5 monochargé |
| 115 | VanE | IMLHQFAEAIGVK | 2 | y10 dichargé |
| 116 | VanE | IMLHQFAEAIGVK | 2 | y8 monochargé |
| 117 | VanE | IMLHQFAEAIGVK | 2 | y9 monochargé |
| 118 | VanE | NMESIDYNVMK | 2 | y6 monochargé |
| 119 | VanE | NMESIDYNVMK | 2 | y7 monochargé |
| 120 | VanE | NMESIDYNVMK | 2 | y8 monochargé |
| 121 | VanE | SAVAIIK | 2 | y4 monochargé |
| 122 | VanE | SAVAIIK | 2 | y5 monochargé |
| 123 | VanE | SAVAIIK | 2 | y6 monochargé |
| 124 | VanE | STPSMIIEK | 2 | y6 monochargé |
| 125 | VanE | STPSMIIEK | 2 | y7 monochargé |
| 126 | VanE | STPSMIIEK | 2 | y7 dichargé |
| 127 | VanE | YNLVTAEILLPAK | 2 | y4 monochargé |
| 128 | VanE | YNLVTAEILLPAK | 2 | y8 monochargé |
| 129 | VanE | YNLVTAEILLPAK | 2 | y9 monochargé |
| 130 | VanG | AGSSFGITK | 2 | y5 monochargé |
| 131 | VanG | AGSSFGITK | 2 | y7 monochargé |
| 132 | VanG | AGSSFGITK | 2 | y8 monochargé |
| 133 | VanG | ALGC[CAM]SGFSR | 2 | y5 monochargé |
| 134 | VanG | ALGC[CAM]SGFSR | 2 | y6 monochargé |
| 135 | VanG | ALGC[CAM]SGFSR | 2 | y7 monochargé |
| 136 | VanG | IDAEAEK | 2 | y4 monochargé |
| 137 | VanG | IDAEAEK | 2 | y5 monochargé |
| 138 | VanG | IDAEAEK | 2 | y6 monochargé |
| 139 | VanG | IYMPAR | 2 | y3 monochargé |
| 140 | VanG | IYMPAR | 2 | y4 monochargé |
| 141 | VanG | IYMPAR | 2 | y5 monochargé |
| 142 | VanG | LIGLYVE | 2 | b4 monochargé |
| 143 | VanG | LIGLYVE | 2 | b5 monochargé |
| 144 | VanG | LIGLYVE | 2 | b6 monochargé |
| 145 | VanG | LVSLAGISVPK | 2 | y6 monochargé |
| 146 | VanG | LVSLAGISVPK | 2 | y7 monochargé |
| 147 | VanG | LVSLAGISVPK | 2 | y9 monochargé |
| 148 | VanG | VDEIELSSGFFDYTEK | 2 | y10 monochargé |
| 149 | VanG | VDEIELSSGFFDYTEK | 2 | y4 monochargé |
| 150 | VanG | VDEIELSSGFFDYTEK | 2 | y6 monochargé |
| 151 | VanG | YPNMMK | 2 | y3 monochargé |
| 152 | VanG | YPNMMK | 2 | y4 monochargé |
| 153 | VanG | YPNMMK | 2 | y5 monochargé |

Les transitions mentionnées dans le **TABLEAU 3** sont détectées en utilisant les paramètres figurant dans les **TABLEAU 4** et **5.**

**TABLEAU 4 :**

| Transition numéro | Temps rétention | (m/z) filtré en Q1 | (m/z) filtré en Q3 | Energie de collision (eV) |
|---|---|---|---|---|
| 1 | 8,75 | 554,79 | 601,32 | 29 |
| 2 | 8,75 | 554,79 | 730,36 | 29 |
| 3 | 8,75 | 554,79 | 858,42 | 29 |
| 4 | 19,71 | 385,28 | 444,32 | 22 |
| 5 | 19,71 | 385,28 | 543,39 | 22 |
| 6 | 19,71 | 385,28 | 656,47 | 22 |
| 7 | 17,91 | 448,75 | 492,29 | 25 |
| 8 | 17,91 | 448,75 | 655,36 | 25 |
| 9 | 17,91 | 448,75 | 783,41 | 25 |
| 10 | 13,32 | 399,24 | 552,3 | 23 |
| 11 | 13,32 | 399,24 | 529,37 | 23 |
| 12 | 13,32 | 399,24 | 666,43 | 23 |
| 13 | 23,29 | 786,42 | 742,41 | 32 |
| 14 | 23,29 | 786,42 | 855,49 | 31 |
| 15 | 23,29 | 786,42 | 926,53 | 34 |
| 16 | 22,44 | 751,41 | 1146,63 | 38 |
| 17 | 22,44 | 751,41 | 974,55 | 38 |
| 18 | 22,44 | 751,41 | 1075,59 | 38 |
| 19 | 15,89 | 572,32 | 630,36 | 25 |
| 20 | 15,89 | 572,32 | 743,44 | 26 |
| 21 | 15,89 | 572,32 | 872,48 | 25 |
| 22 | 10,67 | 384,7 | 537,3 | 20,5 |
| 23 | 10,67 | 384,7 | 624,34 | 18,5 |
| 24 | 10,67 | 384,7 | 681,36 | 19,5 |
| 25 | 15,29 | 447,77 | 430,3 | 25 |
| 26 | 15,29 | 447,77 | 593,37 | 25 |
| 27 | 15,29 | 447,77 | 694,41 | 25 |
| 28 | 16,64 | 597,78 | 702,35 | 29 |
| 29 | 16,64 | 597,78 | 849,42 | 29 |
| 30 | 16,64 | 597,78 | 980,46 | 27 |
| 31 | 18,52 | 826,89 | 646,35 | 41 |
| 32 | 18,52 | 826,89 | 809,42 | 41 |
| 33 | 18,52 | 826,89 | 924,44 | 41 |
| 34 | 19,2 | 598,83 | 807,5 | 31 |
| 35 | 19,2 | 598,83 | 904,55 | 22 |
| 36 | 19,2 | 598,83 | 452,78 | 25 |
| 37 | 15,89 | 595,81 | 694,41 | 33 |
| 38 | 15,89 | 595,81 | 416,24 | 35 |
| 39 | 15,89 | 595,81 | 464,77 | 28 |
| 40 | 19,1 | 598,35 | 650,4 | 29 |
| 41 | 19,1 | 598,35 | 797,47 | 28 |
| 42 | 19,1 | 598,35 | 868,5 | 28 |
| 43 | 4,97 | 562,27 | 616,29 | 30 |
| 44 | 4,97 | 562,27 | 745,34 | 30 |
| 45 | 4,97 | 562,27 | 873,39 | 30 |
| 46 | 12,85 | 415,24 | 492,29 | 23 |
| 47 | 12,85 | 415,24 | 629,35 | 23 |
| 48 | 12,85 | 415,24 | 716,38 | 23 |
| 49 | 17,9 | 454,78 | 637,39 | 25 |
| 50 | 17,9 | 454,78 | 708,43 | 25 |
| 51 | 17,9 | 454,78 | 821,51 | 25 |
| 52 | 14,32 | 449,76 | 522,3 | 25 |
| 53 | 14,32 | 449,76 | 621,37 | 25 |
| 54 | 14,32 | 449,76 | 660,41 | 25 |
| 55 | 1,53 | 338,19 | 319,2 | 20 |
| 56 | 1,53 | 338,19 | 448,24 | 20 |
| 57 | 1,53 | 338,19 | 576,3 | 20 |
| 58 | 14,16 | 510,78 | 451,27 | 24 |
| 59 | 14,16 | 510,78 | 564,35 | 25 |
| 60 | 14,16 | 510,78 | 763,45 | 27 |
| 61 | 19,45 | 744,72 | 845,39 | 43 |
| 62 | 19,45 | 744,72 | 789,37 | 41 |
| 63 | 19,45 | 744,72 | 917,47 | 40 |
| 64 | 22,56 | 717,43 | 611,35 | 30 |
| 65 | 22,56 | 478,62 | 611,35 | 16 |
| 66 | 22,56 | 478,62 | 407,9 | 19 |
| 67 | 19,53 | 655,98 | 559,34 | 34 |
| 68 | 19,53 | 655,98 | 763,43 | 27 |
| 69 | 19,53 | 655,98 | 891,49 | 25 |
| 70 | 12,98 | 579,26 | 641,33 | 31 |
| 71 | 12,98 | 579,26 | 827,4 | 29 |
| 72 | 12,98 | 579,26 | 928,45 | 27 |
| 73 | 16,92 | 457,77 | 416,26 | 22 |
| 74 | 16,92 | 457,77 | 517,31 | 21 |
| 75 | 16,92 | 457,77 | 687,41 | 22 |
| 76 | 27,6 | 728,74 | 788,89 | 29 |
| 77 | 27,6 | 728,74 | 845,51 | 31 |
| 78 | 27,6 | 728,74 | 1008,57 | 31 |
| 79 | 26,39 | 803,11 | 1028,61 | 35 |
| 80 | 26,39 | 803,11 | 799,5 | 30 |
| 81 | 26,39 | 803,11 | 957,57 | 35 |
| 82 | 11,46 | 597,28 | 522,18 | 26 |
| 83 | 11,46 | 597,28 | 672,37 | 33 |
| 84 | 11,46 | 597,28 | 901,44 | 26 |
| 85 | 26,58 | 822,81 | 604,87 | 30 |
| 86 | 26,58 | 822,81 | 688,91 | 26 |
| 87 | 26,58 | 822,81 | 998,59 | 39 |
| 88 | 19,29 | 754,93 | 598,33 | 30 |
| 89 | 19,29 | 754,93 | 817,43 | 45 |
| 90 | 19,29 | 754,93 | 1027,57 | 38 |
| 91 | 18,18 | 560,29 | 476,27 | 30 |
| 92 | 18,18 | 560,29 | 589,36 | 30 |
| 93 | 18,18 | 560,29 | 775,43 | 30 |
| 94 | 13,88 | 411,74 | 305,18 | 23 |
| 95 | 13,88 | 411,74 | 531,35 | 23 |
| 96 | 13,88 | 411,74 | 659,41 | 23 |
| 97 | 9,97 | 423,23 | 458,31 | 25 |
| 98 | 9,97 | 423,23 | 572,35 | 23 |
| 99 | 9,97 | 423,23 | 701,39 | 21 |
| 100 | 19,92 | 388,74 | 435,27 | 19 |
| 101 | 19,92 | 388,74 | 548,36 | 17 |
| 102 | 19,92 | 388,74 | 663,38 | 19 |
| 103 | 5,57 | 540,28 | 373,21 | 35 |
| 104 | 5,57 | 540,28 | 829,41 | 26 |
| 105 | 5,57 | 540,28 | 483,74 | 29 |
| 106 | 10,45 | 441,72 | 564,31 | 23 |
| 107 | 10,45 | 441,72 | 651,35 | 22 |
| 108 | 10,45 | 441,72 | 738,38 | 21 |
| 109 | 4,52 | 367,19 | 434,22 | 21 |
| 110 | 4,52 | 367,19 | 549,25 | 17 |
| 111 | 4,52 | 367,19 | 662,34 | 18 |
| 112 | 21,88 | 803,84 | 1100,48 | 41 |
| 113 | 21,88 | 803,84 | 730,31 | 38 |
| 114 | 21,88 | 803,84 | 613,27 | 46 |
| 115 | 20,03 | 728,9 | 550,3 | 39 |
| 116 | 20,03 | 728,9 | 834,47 | 41 |
| 117 | 20,03 | 728,9 | 962,53 | 39 |
| 118 | 16,81 | 672,3 | 769,35 | 26 |
| 119 | 16,81 | 672,3 | 882,44 | 26 |
| 120 | 16,81 | 672,3 | 969,47 | 26 |
| 121 | 13,59 | 351,23 | 444,32 | 18 |
| 122 | 13,59 | 351,23 | 543,39 | 16 |
| 123 | 13,59 | 351,23 | 614,42 | 17 |
| 124 | 14,51 | 503,27 | 720,4 | 28 |
| 125 | 14,51 | 503,27 | 817,45 | 19 |
| 126 | 14,51 | 503,27 | 409,23 | 20 |
| 127 | 22,39 | 722,92 | 428,29 | 37 |
| 128 | 22,39 | 722,92 | 854,53 | 37 |
| 129 | 22,39 | 722,92 | 955,58 | 37 |
| 130 | 12,77 | 434,23 | 565,33 | 24 |
| 131 | 12,77 | 434,23 | 739,4 | 21 |
| 132 | 12,77 | 434,23 | 796,42 | 22 |
| 133 | 12,41 | 477,73 | 553,27 | 27 |
| 134 | 12,41 | 477,73 | 713,3 | 26 |
| 135 | 12,41 | 477,73 | 770,33 | 24 |
| 136 | 7,63 | 388,2 | 476,24 | 22 |
| 137 | 7,63 | 388,2 | 547,27 | 19 |
| 138 | 7,63 | 388,2 | 662,3 | 19 |
| 139 | 12,85 | 375,7 | 343,21 | 22 |
| 140 | 12,85 | 375,7 | 474,25 | 22 |
| 141 | 12,85 | 375,7 | 637,31 | 22 |
| 142 | 20,72 | 403,74 | 397,28 | 23 |
| 143 | 20,72 | 403,74 | 560,34 | 23 |
| 144 | 20,72 | 403,74 | 659,41 | 23 |
| 145 | 19,66 | 542,34 | 600,37 | 23 |
| 146 | 19,66 | 542,34 | 671,41 | 25 |
| 147 | 19,66 | 542,34 | 871,52 | 23 |
| 148 | 22,14 | 939,94 | 1180,52 | 46 |
| 149 | 22,14 | 939,94 | 540,27 | 46 |
| 150 | 22,14 | 939,94 | 802,36 | 46 |
| 151 | 12,66 | 392,18 | 409,19 | 22 |
| 152 | 12,66 | 392,18 | 523,24 | 22 |
| 153 | 12,66 | 392,18 | 620,29 | 22 |

**TABLEAU 5 :**

| Transition numéro | Potentiel d'orifice | potentiel d'entrée avant Q0 | Potentiel en sortie de cellule de collision | Seuil de positivité |
|---|---|---|---|---|
| 1 | 80 | 10 | 35 | 2000 |
| 2 | 80 | 10 | 35 | 2000 |
| 3 | 80 | 10 | 35 | 2000 |
| 4 | 80 | 10 | 35 | 1400 |
| 5 | 80 | 10 | 35 | 2000 |
| 6 | 80 | 10 | 35 | 2000 |
| 7 | 80 | 10 | 35 | 2000 |
| 8 | 80 | 10 | 35 | 2000 |
| 9 | 80 | 10 | 35 | 1500 |
| 10 | 80 | 10 | 35 | 4000 |
| 11 | 80 | 10 | 35 | 4000 |
| 12 | 80 | 10 | 35 | 4000 |
| 13 | 120 | 10 | 18 | 2000 |
| 14 | 120 | 10 | 18 | 2000 |
| 15 | 120 | 10 | 18 | 2000 |
| 16 | 80 | 10 | 35 | 2000 |
| 17 | 80 | 10 | 35 | 2000 |
| 18 | 80 | 10 | 35 | 2000 |
| 19 | 90 | 10 | 15 | 2000 |
| 20 | 90 | 10 | 15 | 2000 |
| 21 | 90 | 10 | 15 | 2000 |
| 22 | 85 | 10 | 24 | 2000 |
| 23 | 85 | 10 | 24 | 2000 |
| 24 | 85 | 10 | 24 | 2000 |
| 25 | 80 | 10 | 35 | 2000 |
| 26 | 80 | 10 | 35 | 1500 |
| 27 | 80 | 10 | 35 | 2000 |
| 28 | 100 | 10 | 17 | 2000 |
| 29 | 100 | 10 | 17 | 2000 |
| 30 | 100 | 10 | 17 | 2000 |
| 31 | 80 | 10 | 35 | 1000 |
| 32 | 80 | 10 | 35 | 2000 |
| 33 | 80 | 10 | 35 | 2000 |
| 34 | 105 | 10 | 20 | 1400 |
| 35 | 105 | 10 | 20 | 2000 |
| 36 | 105 | 10 | 20 | 2000 |
| 37 | 100 | 10 | 11 | 2000 |
| 38 | 100 | 10 | 16 | 2000 |
| 39 | 100 | 10 | 11 | 2000 |
| 40 | 90 | 10 | 16 | 2000 |
| 41 | 90 | 10 | 18 | 2000 |
| 42 | 90 | 10 | 22 | 2000 |
| 43 | 80 | 10 | 35 | 2000 |
| 44 | 80 | 10 | 35 | 2000 |
| 45 | 80 | 10 | 35 | 2000 |
| 46 | 80 | 10 | 35 | 2000 |
| 47 | 80 | 10 | 35 | 2000 |
| 48 | 80 | 10 | 35 | 2000 |
| 49 | 80 | 10 | 35 | 2000 |
| 50 | 80 | 10 | 35 | 2000 |
| 51 | 80 | 10 | 35 | 2000 |
| 52 | 80 | 10 | 35 | 2000 |
| 53 | 80 | 10 | 35 | 2000 |
| 54 | 80 | 10 | 35 | 2000 |
| 55 | 80 | 10 | 35 | 2000 |
| 56 | 80 | 10 | 35 | 2000 |
| 57 | 80 | 10 | 35 | 2000 |
| 58 | 75 | 12 | 11 | 2000 |
| 59 | 75 | 12 | 14 | 2000 |
| 60 | 75 | 12 | 18 | 2000 |
| 61 | 140 | 10 | 35 | 2000 |
| 62 | 140 | 10 | 35 | 2000 |
| 63 | 140 | 10 | 35 | 2000 |
| 64 | 120 | 10 | 35 | 2000 |
| 65 | 70 | 10 | 35 | 2000 |
| 66 | 70 | 10 | 35 | 2000 |
| 67 | 100 | 10 | 35 | 2000 |
| 68 | 100 | 10 | 35 | 2000 |
| 69 | 100 | 10 | 35 | 2000 |
| 70 | 80 | 12 | 15 | 2000 |
| 71 | 80 | 12 | 19 | 2000 |
| 72 | 80 | 12 | 22 | 2000 |
| 73 | 70 | 12 | 10 | 4000 |
| 74 | 70 | 12 | 13 | 4000 |
| 75 | 70 | 12 | 16 | 4000 |
| 76 | 120 | 10 | 35 | 2000 |
| 77 | 120 | 10 | 35 | 2000 |
| 78 | 120 | 10 | 35 | 2000 |
| 79 | 120 | 10 | 35 | 2000 |
| 80 | 120 | 10 | 35 | 2000 |
| 81 | 120 | 10 | 35 | 2000 |
| 82 | 85 | 12 | 13 | 2000 |
| 83 | 85 | 12 | 16 | 2000 |
| 84 | 85 | 12 | 21 | 2000 |
| 85 | 100 | 10 | 35 | 2000 |
| 86 | 100 | 10 | 35 | 2000 |
| 87 | 100 | 10 | 35 | 2000 |
| 88 | 100 | 10 | 35 | 2000 |
| 89 | 100 | 10 | 35 | 2000 |
| 90 | 100 | 10 | 35 | 2000 |
| 91 | 80 | 10 | 35 | 2000 |
| 92 | 80 | 10 | 35 | 2000 |
| 93 | 80 | 10 | 35 | 2000 |
| 94 | 80 | 10 | 35 | 2000 |
| 95 | 80 | 10 | 35 | 2000 |
| 96 | 80 | 10 | 35 | 2000 |
| 97 | 75 | 10 | 11 | 2000 |
| 98 | 75 | 10 | 14 | 2000 |
| 99 | 75 | 10 | 16 | 1800 |
| 100 | 140 | 6 | 10 | 1300 |
| 101 | 140 | 6 | 14 | 2000 |
| 102 | 140 | 6 | 16 | 2000 |
| 103 | 110 | 12 | 10 | 2000 |
| 104 | 110 | 12 | 20 | 2000 |
| 105 | 110 | 12 | 6 | 2000 |
| 106 | 75 | 12 | 14 | 2000 |
| 107 | 75 | 12 | 16 | 2000 |
| 108 | 75 | 12 | 18 | 2000 |
| 109 | 65 | 12 | 18 | 2000 |
| 110 | 65 | 12 | 23 | 2000 |
| 111 | 65 | 12 | 15 | 2000 |
| 112 | 100 | 10 | 35 | 2000 |
| 113 | 100 | 10 | 35 | 2000 |
| 114 | 100 | 10 | 35 | 2000 |
| 115 | 140 | 9 | 14 | 2000 |
| 116 | 140 | 9 | 34 | 2000 |
| 117 | 140 | 9 | 24 | 2000 |
| 118 | 125 | 9 | 18 | 2000 |
| 119 | 125 | 9 | 21 | 2000 |
| 120 | 125 | 9 | 41 | 2000 |
| 121 | 70 | 12 | 10 | 2000 |
| 122 | 70 | 12 | 23 | 2000 |
| 123 | 70 | 12 | 14 | 2000 |
| 124 | 70 | 12 | 17 | 8000 |
| 125 | 70 | 12 | 20 | 2000 |
| 126 | 70 | 12 | 10 | 2000 |
| 127 | 80 | 10 | 35 | 2000 |
| 128 | 80 | 10 | 35 | 2000 |
| 129 | 80 | 10 | 35 | 2000 |
| 130 | 70 | 12 | 14 | 2000 |
| 131 | 70 | 12 | 18 | 2000 |
| 132 | 70 | 12 | 19 | 2000 |
| 133 | 80 | 12 | 14 | 2000 |
| 134 | 80 | 12 | 18 | 2000 |
| 135 | 80 | 12 | 18 | 2000 |
| 136 | 70 | 12 | 12 | 1600 |
| 137 | 70 | 12 | 13 | 2000 |
| 138 | 70 | 12 | 16 | 2000 |
| 139 | 80 | 10 | 35 | 2000 |
| 140 | 80 | 10 | 35 | 2000 |
| 141 | 80 | 10 | 35 | 2000 |
| 142 | 80 | 10 | 35 | 2000 |
| 143 | 80 | 10 | 35 | 2000 |
| 144 | 80 | 10 | 35 | 2000 |
| 145 | 80 | 12 | 16 | 2000 |
| 146 | 80 | 12 | 14 | 2000 |
| 147 | 80 | 12 | 21 | 2000 |
| 148 | 80 | 10 | 35 | 2000 |
| 149 | 80 | 10 | 35 | 2000 |
| 150 | 80 | 10 | 35 | 2000 |
| 151 | 80 | 10 | 35 | 3000 |
| 152 | 80 | 10 | 35 | 2000 |
| 153 | 80 | 10 | 35 | 2000 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 550,00 °C |
| Gaz de nébulisation: | 50,00 psi |
| Gaz chauffant: | 40,00 psi |
| Gaz de collision induisant dissociation : | 9,00 psi |
| Remplissage dynamique: | inactivé |
| Temps de cycle total: | 1.2 sec |
| Fenêtre de détection : | 90 sec |

Les aires obtenues pour chacune des transitions et pour chacun des microorganismes étudiés ont été mesurées. Lorsque l'aire d'une transition est supérieure ou égale au seuil de positivité décrit dans le **TABLEAU 5,** la détection de la transition est considérée comme positive et est notée « 1 » dans le **TABLEAU 6.** Lorsque l'aire d'une transition est inférieure au seuil de positivité décrit dans le **TABLEAU 5,** la détection de la transition est considérée comme négative et est notée « 0 » dans le **TABLEAU 6.** Lorsque les 3 transitions d'un même peptide sont notées « 1 », la détection du peptide est considérée comme positive. Un cas particulier fait exception à cette règle. Le peptide correspondant aux transitions numéros 49, 50 et 51 a une transition de très faible intensité. Dans ce cas précis, lorsque les transitions 49 et 50 sont notées « 1 », la détection de ce peptide sera considérée comme positive.

**TABLEAU 6 :**

| Transition numéro | Ech1 | Ech2 | Ech3 | Ech4 | Ech5 | Ech6 | Ech7 | Ech8 | Ech9 | Ech10 | Ech11 |
|---|---|---|---|---|---|---|---|---|---|---|---|
| 1 | 0 | 0 | 0 | 0 | 0 | 0 | 0 | 0 | 0 | 0 | 0 |
| 2 | 0 | 0 | 0 | 0 | 0 | 0 | 0 | 0 | 0 | 0 | 0 |
| 3 | 0 | 0 | 0 | 0 | 0 | 0 | 0 | 0 | 0 | 0 | 0 |
| 4 | 0 | 0 | 0 | 0 | 0 | 0 | 0 | 0 | 0 | 1 | 0 |
| 5 | 0 | 0 | 0 | 0 | 0 | 0 | 0 | 0 | 0 | 1 | 0 |
| 6 | 0 | 0 | 0 | 0 | 0 | 0 | 0 | 0 | 0 | 1 | 0 |
| 7 | 0 | 0 | 0 | 0 | 0 | 0 | 0 | 0 | 0 | 1 | 0 |
| 8 | 0 | 0 | 0 | 0 | 0 | 0 | 0 | 0 | 0 | 1 | 0 |
| 9 | 0 | 0 | 0 | 0 | 0 | 0 | 0 | 0 | 0 | 1 | 0 |
| 10 | 0 | 0 | 0 | 0 | 0 | 0 | 0 | 0 | 0 | 1 | 0 |
| 11 | 0 | 0 | 0 | 0 | 0 | 0 | 0 | 0 | 0 | 1 | 0 |
| 12 | 0 | 0 | 0 | 0 | 0 | 0 | 0 | 0 | 0 | 1 | 0 |
| 13 | 0 | 0 | 0 | 0 | 0 | 0 | 0 | 0 | 0 | 0 | 0 |
| 14 | 0 | 0 | 0 | 0 | 0 | 0 | 0 | 0 | 0 | 0 | 0 |
| 15 | 0 | 0 | 0 | 0 | 0 | 0 | 0 | 0 | 0 | 0 | 0 |
| 16 | 0 | 0 | 0 | 0 | 0 | 0 | 0 | 0 | 0 | 0 | 0 |
| 17 | 0 | 0 | 0 | 0 | 0 | 0 | 0 | 0 | 0 | 0 | 0 |
| 18 | 0 | 0 | 0 | 0 | 0 | 0 | 0 | 0 | 0 | 0 | 0 |
| 19 | 0 | 0 | 1 | 0 | 0 | 0 | 0 | 0 | 0 | 1 | 1 |
| 20 | 0 | 0 | 1 | 0 | 0 | 0 | 0 | 0 | 0 | 1 | 1 |
| 21 | 0 | 0 | 1 | 0 | 0 | 0 | 0 | 0 | 0 | 1 | 1 |
| 22 | 0 | 0 | 1 | 0 | 0 | 0 | 0 | 0 | 0 | 1 | 0 |
| 23 | 0 | 0 | 1 | 0 | 0 | 0 | 0 | 0 | 0 | 1 | 0 |
| 24 | 0 | 0 | 1 | 0 | 0 | 0 | 0 | 0 | 0 | 1 | 0 |
| 25 | 0 | 0 | 0 | 0 | 0 | 0 | 0 | 0 | 0 | 1 | 0 |
| 26 | 0 | 0 | 0 | 0 | 0 | 0 | 0 | 0 | 0 | 1 | 0 |
| 27 | 0 | 0 | 0 | 0 | 0 | 0 | 0 | 0 | 0 | 1 | 0 |
| 28 | 0 | 0 | 0 | 0 | 0 | 0 | 0 | 0 | 0 | 1 | 0 |
| 29 | 0 | 0 | 0 | 0 | 0 | 0 | 0 | 0 | 0 | 0 | 0 |
| 30 | 0 | 0 | 0 | 0 | 0 | 0 | 0 | 0 | 0 | 0 | 0 |
| 31 | 0 | 0 | 0 | 0 | 0 | 0 | 0 | 0 | 0 | 1 | 0 |
| 32 | 0 | 0 | 0 | 0 | 0 | 0 | 0 | 0 | 0 | 1 | 0 |
| 33 | 0 | 0 | 0 | 0 | 0 | 0 | 0 | 0 | 0 | 1 | 0 |
| 34 | 0 | 0 | 1 | 0 | 0 | 0 | 0 | 0 | 0 | 1 | 1 |
| 35 | 0 | 0 | 1 | 0 | 0 | 0 | 0 | 0 | 0 | 1 | 1 |
| 36 | 0 | 0 | 1 | 0 | 0 | 0 | 0 | 0 | 0 | 1 | 1 |
| 37 | 0 | 0 | 0 | 0 | 0 | 0 | 0 | 0 | 0 | 0 | 0 |
| 38 | 0 | 0 | 0 | 0 | 0 | 0 | 0 | 0 | 0 | 0 | 0 |
| 39 | 0 | 0 | 0 | 0 | 0 | 0 | 0 | 0 | 0 | 0 | 0 |
| 40 | 0 | 0 | 0 | 0 | 0 | 0 | 0 | 0 | 0 | 0 | 0 |
| 41 | 0 | 0 | 0 | 0 | 0 | 0 | 0 | 0 | 0 | 0 | 0 |
| 42 | 0 | 0 | 0 | 0 | 0 | 0 | 0 | 0 | 0 | 0 | 0 |
| 43 | 0 | 0 | 0 | 0 | 0 | 0 | 0 | 0 | 0 | 0 | 0 |
| 44 | 0 | 0 | 0 | 0 | 0 | 0 | 0 | 0 | 0 | 0 | 0 |
| 45 | 0 | 0 | 0 | 0 | 0 | 0 | 0 | 0 | 0 | 0 | 0 |
| 46 | 0 | 0 | 0 | 0 | 0 | 0 | 0 | 0 | 0 | 0 | 0 |
| 47 | 0 | 0 | 0 | 0 | 0 | 0 | 0 | 0 | 0 | 0 | 0 |
| 48 | 0 | 0 | 0 | 0 | 0 | 0 | 0 | 0 | 0 | 0 | 0 |
| 49 | 0 | 0 | 0 | 0 | 0 | 0 | 0 | 0 | 0 | 0 | 0 |
| 50 | 0 | 0 | 0 | 0 | 0 | 0 | 0 | 0 | 0 | 0 | 0 |
| 51 | 0 | 0 | 0 | 0 | 0 | 0 | 0 | 0 | 0 | 0 | 0 |
| 52 | 0 | 0 | 0 | 0 | 0 | 0 | 0 | 0 | 0 | 0 | 0 |
| 53 | 0 | 0 | 0 | 0 | 0 | 0 | 0 | 0 | 0 | 0 | 0 |
| 54 | 0 | 0 | 0 | 0 | 0 | 0 | 0 | 0 | 0 | 0 | 0 |
| 55 | 0 | 0 | 0 | 0 | 0 | 0 | 0 | 0 | 0 | 0 | 0 |
| 56 | 0 | 0 | 0 | 0 | 0 | 0 | 0 | 0 | 0 | 0 | 0 |
| 57 | 0 | 0 | 0 | 0 | 0 | 0 | 0 | 0 | 0 | 0 | 0 |
| 58 | 0 | 0 | 0 | 0 | 0 | 0 | 0 | 0 | 0 | 0 | 0 |
| 59 | 0 | 0 | 0 | 0 | 0 | 0 | 0 | 0 | 0 | 0 | 1 |
| 60 | 0 | 0 | 0 | 0 | 0 | 0 | 0 | 0 | 0 | 0 | 0 |
| 61 | 0 | 0 | 0 | 0 | 0 | 0 | 0 | 0 | 0 | 0 | 0 |
| 62 | 0 | 0 | 0 | 0 | 0 | 0 | 0 | 0 | 0 | 0 | 0 |
| 63 | 0 | 0 | 0 | 0 | 0 | 0 | 0 | 0 | 0 | 0 | 0 |
| 64 | 0 | 0 | 0 | 0 | 0 | 0 | 0 | 0 | 0 | 0 | 0 |
| 65 | 0 | 0 | 0 | 0 | 0 | 0 | 0 | 0 | 0 | 0 | 0 |
| 66 | 0 | 0 | 0 | 0 | 0 | 0 | 0 | 0 | 0 | 0 | 0 |
| 67 | 0 | 0 | 0 | 0 | 0 | 0 | 0 | 0 | 0 | 0 | 0 |
| 68 | 0 | 0 | 0 | 0 | 0 | 0 | 0 | 0 | 0 | 0 | 0 |
| 69 | 0 | 0 | 0 | 0 | 0 | 0 | 0 | 0 | 0 | 0 | 0 |
| 70 | 0 | 0 | 0 | 0 | 0 | 0 | 0 | 0 | 0 | 0 | 0 |
| 71 | 0 | 0 | 0 | 0 | 0 | 0 | 0 | 0 | 0 | 0 | 0 |
| 72 | 0 | 0 | 0 | 0 | 0 | 0 | 0 | 0 | 0 | 0 | 0 |
| 73 | 0 | 0 | 0 | 0 | 0 | 0 | 0 | 0 | 0 | 0 | 0 |
| 74 | 0 | 0 | 0 | 0 | 0 | 0 | 0 | 0 | 0 | 0 | 0 |
| 75 | 0 | 0 | 0 | 0 | 0 | 0 | 0 | 0 | 0 | 0 | 0 |
| 76 | 0 | 0 | 0 | 0 | 0 | 0 | 0 | 0 | 0 | 0 | 0 |
| 77 | 0 | 0 | 0 | 0 | 0 | 0 | 0 | 0 | 0 | 0 | 0 |
| 78 | 0 | 0 | 0 | 0 | 0 | 0 | 0 | 0 | 0 | 0 | 0 |
| 79 | 0 | 0 | 0 | 0 | 0 | 0 | 0 | 0 | 0 | 0 | 0 |
| 80 | 0 | 1 | 0 | 0 | 0 | 0 | 0 | 0 | 0 | 0 | 0 |
| 81 | 0 | 0 | 0 | 0 | 0 | 0 | 0 | 0 | 0 | 0 | 0 |
| 82 | 0 | 0 | 0 | 0 | 0 | 0 | 0 | 0 | 0 | 0 | 0 |
| 83 | 0 | 0 | 0 | 0 | 0 | 0 | 0 | 0 | 0 | 0 | 0 |
| 84 | 0 | 0 | 0 | 0 | 0 | 0 | 0 | 0 | 0 | 0 | 0 |
| 85 | 0 | 0 | 0 | 0 | 0 | 0 | 0 | 0 | 0 | 0 | 0 |
| 86 | 0 | 0 | 0 | 0 | 0 | 0 | 0 | 0 | 0 | 0 | 0 |
| 87 | 0 | 0 | 0 | 0 | 0 | 0 | 0 | 0 | 0 | 0 | 0 |
| 88 | 0 | 0 | 0 | 0 | 0 | 0 | 0 | 0 | 0 | 0 | 0 |
| 89 | 0 | 0 | 0 | 0 | 0 | 0 | 0 | 0 | 0 | 0 | 0 |
| 90 | 0 | 0 | 0 | 0 | 0 | 0 | 0 | 0 | 0 | 0 | 0 |
| 91 | 0 | 0 | 0 | 0 | 0 | 0 | 0 | 0 | 0 | 0 | 0 |
| 92 | 0 | 0 | 0 | 0 | 0 | 0 | 0 | 0 | 0 | 0 | 0 |
| 93 | 0 | 0 | 0 | 0 | 0 | 0 | 0 | 0 | 0 | 0 | 0 |
| 94 | 0 | 0 | 0 | 0 | 0 | 0 | 0 | 0 | 0 | 0 | 0 |
| 95 | 0 | 0 | 0 | 0 | 0 | 0 | 0 | 0 | 0 | 0 | 0 |
| 96 | 0 | 0 | 0 | 0 | 0 | 0 | 0 | 0 | 0 | 0 | 0 |
| 97 | 0 | 0 | 0 | 0 | 0 | 0 | 0 | 1 | 0 | 0 | 0 |
| 98 | 0 | 0 | 0 | 0 | 0 | 0 | 0 | 1 | 0 | 0 | 0 |
| 99 | 0 | 0 | 0 | 0 | 0 | 0 | 0 | 1 | 0 | 0 | 0 |
| 100 | 0 | 0 | 0 | 0 | 0 | 0 | 0 | 1 | 0 | 0 | 0 |
| 101 | 0 | 0 | 0 | 0 | 0 | 0 | 0 | 1 | 0 | 0 | 0 |
| 102 | 0 | 0 | 0 | 0 | 0 | 0 | 0 | 1 | 0 | 0 | 0 |
| 103 | 0 | 0 | 0 | 0 | 0 | 0 | 0 | 0 | 0 | 0 | 0 |
| 104 | 0 | 0 | 0 | 0 | 0 | 0 | 0 | 0 | 0 | 0 | 0 |
| 105 | 0 | 0 | 0 | 0 | 0 | 0 | 0 | 0 | 0 | 0 | 0 |
| 106 | 0 | 0 | 0 | 0 | 0 | 0 | 0 | 1 | 0 | 0 | 0 |
| 107 | 0 | 0 | 0 | 0 | 0 | 0 | 0 | 1 | 0 | 0 | 0 |
| 108 | 0 | 0 | 0 | 0 | 0 | 0 | 0 | 1 | 0 | 0 | 0 |
| 109 | 0 | 0 | 0 | 0 | 0 | 0 | 0 | 0 | 0 | 0 | 0 |
| 110 | 0 | 0 | 0 | 0 | 0 | 0 | 0 | 0 | 0 | 0 | 0 |
| 111 | 0 | 0 | 0 | 0 | 0 | 0 | 0 | 0 | 0 | 0 | 0 |
| 112 | 0 | 0 | 0 | 0 | 0 | 0 | 0 | 0 | 0 | 0 | 0 |
| 113 | 0 | 0 | 0 | 0 | 0 | 0 | 0 | 0 | 0 | 0 | 0 |
| 114 | 0 | 0 | 0 | 0 | 0 | 0 | 0 | 0 | 0 | 0 | 0 |
| 115 | 0 | 0 | 0 | 0 | 0 | 0 | 0 | 0 | 0 | 0 | 0 |
| 116 | 0 | 0 | 0 | 0 | 0 | 0 | 0 | 0 | 0 | 0 | 0 |
| 117 | 0 | 0 | 0 | 0 | 0 | 0 | 0 | 0 | 0 | 0 | 0 |
| 118 | 0 | 0 | 0 | 0 | 0 | 0 | 0 | 0 | 0 | 1 | 0 |
| 119 | 0 | 0 | 0 | 0 | 0 | 0 | 0 | 0 | 0 | 1 | 0 |
| 120 | 0 | 1 | 0 | 0 | 0 | 0 | 0 | 0 | 0 | 0 | 0 |
| 121 | 0 | 0 | 0 | 0 | 0 | 0 | 1 | 0 | 0 | 0 | 0 |
| 122 | 0 | 0 | 0 | 0 | 0 | 0 | 0 | 0 | 0 | 0 | 0 |
| 123 | 0 | 0 | 0 | 0 | 0 | 0 | 0 | 0 | 0 | 0 | 0 |
| 124 | 0 | 0 | 0 | 0 | 0 | 0 | 0 | 0 | 0 | 0 | 0 |
| 125 | 1 | 0 | 1 | 0 | 0 | 1 | 0 | 0 | 0 | 0 | 0 |
| 126 | 1 | 0 | 1 | 0 | 0 | 1 | 0 | 0 | 0 | 0 | 0 |
| 127 | 0 | 0 | 0 | 0 | 0 | 0 | 0 | 0 | 0 | 0 | 0 |
| 128 | 0 | 0 | 0 | 0 | 0 | 0 | 0 | 0 | 0 | 0 | 0 |
| 129 | 0 | 0 | 0 | 0 | 0 | 0 | 0 | 0 | 0 | 0 | 0 |
| 130 | 0 | 0 | 0 | 0 | 0 | 0 | 0 | 0 | 0 | 0 | 0 |
| 131 | 0 | 0 | 0 | 0 | 0 | 1 | 0 | 0 | 0 | 0 | 0 |
| 132 | 0 | 0 | 0 | 0 | 0 | 0 | 0 | 0 | 0 | 0 | 0 |
| 133 | 0 | 0 | 0 | 0 | 0 | 0 | 0 | 0 | 0 | 0 | 0 |
| 134 | 0 | 0 | 0 | 0 | 0 | 0 | 0 | 0 | 0 | 0 | 0 |
| 135 | 0 | 0 | 0 | 0 | 0 | 0 | 0 | 0 | 0 | 0 | 0 |
| 136 | 0 | 0 | 0 | 0 | 0 | 1 | 0 | 0 | 0 | 0 | 0 |
| 137 | 0 | 0 | 0 | 0 | 0 | 1 | 1 | 0 | 0 | 0 | 0 |
| 138 | 0 | 0 | 0 | 0 | 0 | 1 | 0 | 0 | 0 | 0 | 0 |
| 139 | 0 | 0 | 0 | 0 | 0 | 0 | 0 | 0 | 0 | 0 | 0 |
| 140 | 0 | 0 | 0 | 0 | 0 | 0 | 0 | 0 | 0 | 0 | 0 |
| 141 | 0 | 0 | 0 | 0 | 0 | 0 | 0 | 0 | 0 | 0 | 0 |
| 142 | 0 | 0 | 0 | 0 | 0 | 0 | 0 | 0 | 0 | 0 | 0 |
| 143 | 0 | 0 | 0 | 0 | 0 | 0 | 0 | 0 | 0 | 0 | 0 |
| 144 | 0 | 0 | 0 | 0 | 0 | 0 | 0 | 0 | 0 | 0 | 0 |
| 145 | 0 | 0 | 0 | 0 | 0 | 1 | 0 | 0 | 0 | 0 | 0 |
| 146 | 0 | 0 | 0 | 0 | 0 | 1 | 0 | 0 | 0 | 0 | 0 |
| 147 | 0 | 0 | 0 | 0 | 0 | 1 | 0 | 0 | 0 | 0 | 0 |
| 148 | 0 | 0 | 0 | 0 | 0 | 0 | 0 | 0 | 0 | 0 | 0 |
| 149 | 0 | 0 | 0 | 0 | 0 | 0 | 0 | 0 | 0 | 0 | 0 |
| 150 | 0 | 0 | 0 | 0 | 0 | 0 | 0 | 0 | 0 | 0 | 0 |
| 151 | 0 | 0 | 1 | 0 | 0 | 0 | 0 | 0 | 0 | 0 | 0 |
| 152 | 0 | 0 | 1 | 0 | 0 | 0 | 0 | 0 | 0 | 0 | 0 |
| 153 | 0 | 0 | 1 | 0 | 0 | 0 | 0 | 0 | 0 | 0 | 0 |

Les échantillons Ech1, Ech2, Ech4, Ech5, Ech7 et Ech9 ne présentent aucun peptide caractéristique de résistance de type Van. Les bactéries présentes dans les échantillons Ech1, Ech2, Ech4, Ech5, Ech7 et Ech9 peuvent être sensibles aux glycopeptides de type Vancomycine ou Teicoplanine.

Les échantillons Ech3, Ech10 et Ech11 comportent au moins un peptide caractéristique de VanA. Les bactéries présentes dans les échantillons Ech3, Ech10 et Ech11 expriment donc la ligase VanA qui leur confère une résistance à la Vancomycine et à la Teicoplanine.

L'échantillon Ech6 comporte au moins un peptide caractéristique de VanG. La bactérie présente dans l'échantillon Ech6 exprime donc la ligase VanG qui lui confère une résistance à la Vancomycine.

L'échantillon Ech8 comporte au moins un peptide caractéristique de VanD. La bactérie présente dans l'échantillon Ech8 exprime donc la ligase VanD qui lui confère une résistance à la Vancomycine et à la Teicoplanine.

Ainsi, de façon avantageuse plusieurs mécanismes de résistance aux glycopeptides sont recherchés simultanément. De façon particulièrement avantageuse, un mécanisme de résistance inductible, tel que le mécanisme de résistance lié à l'expression de VanA, est détecté sans phase d'induction lors de la culture du microorganisme. Ce procédé permet donc de rechercher les mécanismes de résistance aux glycopeptides sans *a priori* sur leur éventuel existence.

### Exemple 9 : Identification de résistance aux glycopeptides, avec induction, de type VanA, VanB, VanD, VanE et VanG:

Les échantillons Ech1 à Ech11 sont identifiés selon l'une des méthodes décrites dans les exemples 1 à 6. L'identification des espèces est reportée dans le **TABLEAU 7.**

**TABLEAU7:**

| **Nom** | **Espèces** |
|---|---|
| Ech12 | *E. faecalis* |
| Ech13 | *E. faecalis* |
| Ech14 | *E. faecalis* |
| Ech15 | *E. faecalis* |
| Ech16 | *E. faecalis* |
| Ech17 | *E. faecium* |
| Ech18 | *E. faecium* |
| Ech19 | *E. faecalis* |
| Ech20 | *E. faecium* |

Les échantillons Ech12 à Ech20 correspondent à une espèce pouvant comporter un mécanisme de résistance de type Van. La méthode suivante est alors mise en oeuvre pour rechercher un tel mécanisme.

La colonie de microorganisme est obtenue selon l'exemple 3 puis traité selon l'exemple 7 et analysé selon l'exemple 8.

Les aires obtenues pour chacune des transitions et pour chacun des microorganismes étudiés ont été mesurées. Lorsque l'aire d'une transition est supérieure ou égale au seuil de positivité décrit dans le **TABLEAU 5,** la détection de la transition est considérée comme positive et est notée « 1 » dans le **TABLEAU 8.** Lorsque l'aire d'une transition est inférieure au seuil de positivité décrit dans le **TABLEAU 5,** la détection de la transition est considérée comme négative et est notée « 0 » dans le **TABLEAU 8.** Lorsque les 3 transitions d'un même peptide sont notées « 1 », la détection du peptide est considérée comme positive. Un cas particulier fait exception à cette règle. Le peptide correspondant aux transitions numéros 49, 50 et 51 a une transition de très faible intensité. Dans ce cas précis, lorsque les transitions 49 et 50 sont notées « 1 », la détection de ce peptide sera considérée comme positive.

**TABLEAU 8 :**

| Transition numéro | Ech12 | Ech13 | Ech14 | Ech15 | Ech16 | Ech17 | Ech18 | Ech19 | Ech20 |
|---|---|---|---|---|---|---|---|---|---|
| 1 | 0 | 0 | 0 | 0 | 0 | 0 | 0 | 0 | 0 |
| 2 | 0 | 0 | 0 | 0 | 0 | 0 | 0 | 0 | 0 |
| 3 | 0 | 0 | 0 | 0 | 0 | 0 | 0 | 1 | 0 |
| 4 | 0 | 0 | 0 | 0 | 0 | 0 | 0 | 0 | 0 |
| 5 | 0 | 0 | 0 | 0 | 0 | 0 | 0 | 0 | 1 |
| 6 | 0 | 0 | 0 | 0 | 0 | 0 | 0 | 0 | 0 |
| 7 | 0 | 0 | 0 | 0 | 0 | 0 | 0 | 1 | 1 |
| 8 | 0 | 0 | 0 | 0 | 0 | 0 | 0 | 1 | 1 |
| 9 | 0 | 0 | 0 | 0 | 0 | 0 | 0 | 1 | 1 |
| 10 | 0 | 0 | 0 | 0 | 0 | 0 | 0 | 1 | 1 |
| 11 | 0 | 0 | 0 | 0 | 0 | 0 | 0 | 1 | 1 |
| 12 | 0 | 0 | 0 | 0 | 0 | 0 | 0 | 1 | 1 |
| 13 | 0 | 0 | 0 | 0 | 0 | 0 | 0 | 1 | 1 |
| 14 | 0 | 0 | 0 | 0 | 0 | 0 | 0 | 1 | 1 |
| 15 | 0 | 0 | 0 | 0 | 0 | 0 | 0 | 1 | 1 |
| 16 | 0 | 0 | 0 | 0 | 0 | 0 | 0 | 0 | 0 |
| 17 | 0 | 0 | 0 | 0 | 0 | 0 | 0 | 0 | 0 |
| 18 | 0 | 0 | 0 | 0 | 0 | 0 | 0 | 0 | 0 |
| 19 | 0 | 0 | 0 | 0 | 0 | 0 | 0 | 1 | 1 |
| 20 | 0 | 0 | 0 | 0 | 0 | 0 | 0 | 1 | 1 |
| 21 | 0 | 0 | 0 | 0 | 0 | 0 | 0 | 1 | 1 |
| 22 | 0 | 0 | 0 | 0 | 0 | 0 | 0 | 1 | 1 |
| 23 | 0 | 0 | 0 | 0 | 0 | 0 | 0 | 1 | 1 |
| 24 | 0 | 0 | 0 | 0 | 0 | 0 | 0 | 1 | 1 |
| 25 | 0 | 0 | 0 | 1 | 0 | 0 | 0 | 1 | 0 |
| 26 | 0 | 0 | 0 | 0 | 0 | 0 | 0 | 1 | 0 |
| 27 | 0 | 0 | 0 | 0 | 0 | 0 | 0 | 1 | 0 |
| 28 | 0 | 0 | 0 | 0 | 0 | 0 | 0 | 1 | 1 |
| 29 | 0 | 0 | 0 | 0 | 0 | 0 | 0 | 1 | 1 |
| 30 | 0 | 0 | 0 | 0 | 0 | 0 | 0 | 1 | 1 |
| 31 | 0 | 0 | 0 | 0 | 0 | 0 | 0 | 1 | 1 |
| 32 | 0 | 0 | 0 | 0 | 0 | 0 | 0 | 1 | 0 |
| 33 | 0 | 0 | 0 | 0 | 0 | 0 | 0 | 1 | 0 |
| 34 | 0 | 0 | 0 | 0 | 0 | 0 | 0 | 1 | 1 |
| 35 | 0 | 0 | 0 | 0 | 0 | 0 | 0 | 1 | 1 |
| 36 | 0 | 0 | 0 | 0 | 0 | 0 | 0 | 1 | 1 |
| 37 | 0 | 1 | 1 | 0 | 0 | 0 | 0 | 0 | 0 |
| 38 | 0 | 1 | 1 | 0 | 0 | 0 | 0 | 0 | 0 |
| 39 | 0 | 1 | 1 | 0 | 0 | 0 | 1 | 0 | 0 |
| 40 | 0 | 1 | 1 | 0 | 0 | 0 | 1 | 0 | 0 |
| 41 | 0 | 1 | 1 | 0 | 0 | 0 | 0 | 0 | 0 |
| 42 | 0 | 1 | 1 | 0 | 0 | 0 | 0 | 0 | 0 |
| 43 | 0 | 0 | 0 | 0 | 0 | 0 | 0 | 0 | 0 |
| 44 | 0 | 0 | 0 | 0 | 0 | 0 | 0 | 0 | 0 |
| 45 | 0 | 0 | 0 | 0 | 0 | 0 | 0 | 0 | 0 |
| 46 | 0 | 1 | 1 | 0 | 0 | 1 | 1 | 0 | 0 |
| 47 | 0 | 1 | 1 | 0 | 0 | 0 | 1 | 0 | 0 |
| 48 | 0 | 1 | 1 | 0 | 0 | 0 | 1 | 0 | 0 |
| 49 | 0 | 1 | 1 | 0 | 0 | 0 | 0 | 0 | 0 |
| 50 | 0 | 1 | 1 | 0 | 0 | 0 | 0 | 0 | 0 |
| 51 | 0 | 0 | 0 | 0 | 0 | 0 | 0 | 0 | 0 |
| 52 | 0 | 0 | 0 | 0 | 0 | 0 | 0 | 0 | 0 |
| 53 | 0 | 0 | 0 | 0 | 0 | 0 | 0 | 0 | 0 |
| 54 | 0 | 1 | 0 | 0 | 0 | 0 | 0 | 0 | 0 |
| 55 | 0 | 0 | 0 | 0 | 0 | 0 | 0 | 0 | 0 |
| 56 | 0 | 0 | 0 | 0 | 0 | 0 | 0 | 0 | 0 |
| 57 | 0 | 0 | 0 | 0 | 0 | 0 | 0 | 0 | 0 |
| 58 | 0 | 0 | 0 | 0 | 0 | 0 | 0 | 0 | 0 |
| 59 | 0 | 0 | 0 | 0 | 0 | 0 | 0 | 0 | 0 |
| 60 | 0 | 0 | 0 | 0 | 0 | 0 | 0 | 0 | 0 |
| 61 | 0 | 0 | 0 | 0 | 0 | 0 | 0 | 0 | 0 |
| 62 | 0 | 0 | 0 | 0 | 0 | 0 | 0 | 0 | 0 |
| 63 | 0 | 0 | 0 | 0 | 0 | 0 | 0 | 0 | 0 |
| 64 | 0 | 0 | 0 | 0 | 0 | 0 | 0 | 0 | 0 |
| 65 | 0 | 0 | 0 | 0 | 0 | 0 | 0 | 0 | 0 |
| 66 | 0 | 0 | 0 | 0 | 0 | 0 | 0 | 0 | 0 |
| 67 | 0 | 0 | 0 | 0 | 0 | 0 | 0 | 0 | 0 |
| 68 | 0 | 0 | 0 | 0 | 0 | 0 | 0 | 0 | 0 |
| 69 | 0 | 0 | 0 | 0 | 0 | 0 | 0 | 0 | 0 |
| 70 | 0 | 0 | 0 | 0 | 0 | 0 | 0 | 0 | 0 |
| 71 | 0 | 0 | 0 | 0 | 0 | 0 | 0 | 0 | 0 |
| 72 | 0 | 0 | 0 | 0 | 0 | 0 | 0 | 0 | 0 |
| 73 | 0 | 0 | 0 | 0 | 0 | 1 | 0 | 0 | 0 |
| 74 | 0 | 0 | 0 | 0 | 0 | 0 | 0 | 0 | 0 |
| 75 | 0 | 0 | 0 | 0 | 0 | 0 | 0 | 0 | 0 |
| 76 | 0 | 0 | 0 | 0 | 0 | 0 | 0 | 0 | 0 |
| 77 | 0 | 0 | 0 | 0 | 0 | 0 | 0 | 0 | 0 |
| 78 | 0 | 0 | 0 | 0 | 0 | 0 | 0 | 0 | 0 |
| 79 | 0 | 0 | 0 | 0 | 0 | 0 | 0 | 0 | 0 |
| 80 | 0 | 0 | 0 | 0 | 0 | 0 | 0 | 0 | 0 |
| 81 | 0 | 0 | 0 | 0 | 0 | 0 | 0 | 0 | 0 |
| 82 | 0 | 0 | 0 | 0 | 0 | 0 | 0 | 0 | 0 |
| 83 | 0 | 0 | 0 | 0 | 0 | 0 | 0 | 0 | 0 |
| 84 | 0 | 0 | 0 | 0 | 0 | 0 | 0 | 0 | 0 |
| 85 | 0 | 0 | 0 | 0 | 0 | 0 | 0 | 0 | 0 |
| 86 | 0 | 0 | 0 | 0 | 0 | 0 | 0 | 0 | 0 |
| 87 | 0 | 0 | 0 | 0 | 0 | 0 | 0 | 0 | 0 |
| 88 | 0 | 0 | 0 | 0 | 0 | 0 | 0 | 0 | 0 |
| 89 | 0 | 0 | 0 | 0 | 0 | 0 | 0 | 0 | 0 |
| 90 | 0 | 0 | 0 | 0 | 0 | 0 | 0 | 0 | 0 |
| 91 | 0 | 0 | 0 | 0 | 0 | 0 | 0 | 0 | 0 |
| 92 | 0 | 0 | 0 | 0 | 0 | 0 | 0 | 0 | 0 |
| 93 | 0 | 0 | 0 | 0 | 0 | 0 | 0 | 0 | 0 |
| 94 | 0 | 0 | 0 | 0 | 0 | 0 | 0 | 0 | 0 |
| 95 | 0 | 0 | 0 | 0 | 0 | 0 | 0 | 0 | 0 |
| 96 | 0 | 0 | 0 | 0 | 0 | 0 | 0 | 0 | 0 |
| 97 | 0 | 0 | 0 | 0 | 0 | 1 | 0 | 0 | 0 |
| 98 | 0 | 0 | 0 | 0 | 0 | 1 | 0 | 0 | 0 |
| 99 | 0 | 0 | 0 | 0 | 0 | 1 | 0 | 0 | 0 |
| 100 | 0 | 0 | 0 | 0 | 0 | 1 | 0 | 0 | 0 |
| 101 | 0 | 0 | 0 | 0 | 0 | 1 | 0 | 0 | 0 |
| 102 | 0 | 0 | 0 | 0 | 0 | 1 | 0 | 0 | 1 |
| 103 | 0 | 0 | 0 | 0 | 0 | 0 | 0 | 0 | 0 |
| 104 | 0 | 0 | 0 | 0 | 0 | 0 | 0 | 0 | 0 |
| 105 | 0 | 0 | 0 | 0 | 0 | 0 | 0 | 0 | 0 |
| 106 | 0 | 0 | 0 | 0 | 0 | 1 | 0 | 0 | 0 |
| 107 | 0 | 0 | 0 | 0 | 0 | 1 | 0 | 0 | 0 |
| 108 | 0 | 0 | 0 | 0 | 0 | 1 | 0 | 0 | 0 |
| 109 | 0 | 0 | 0 | 0 | 0 | 0 | 0 | 0 | 0 |
| 110 | 0 | 0 | 0 | 0 | 1 | 0 | 0 | 0 | 0 |
| 111 | 0 | 0 | 0 | 0 | 0 | 0 | 0 | 0 | 0 |
| 112 | 0 | 0 | 0 | 0 | 0 | 0 | 0 | 0 | 0 |
| 113 | 0 | 0 | 0 | 0 | 0 | 0 | 0 | 0 | 0 |
| 114 | 0 | 0 | 0 | 0 | 0 | 0 | 0 | 0 | 0 |
| 115 | 0 | 0 | 0 | 0 | 0 | 0 | 0 | 0 | 0 |
| 116 | 0 | 0 | 0 | 0 | 0 | 0 | 0 | 0 | 0 |
| 117 | 0 | 0 | 0 | 0 | 0 | 0 | 0 | 0 | 0 |
| 118 | 0 | 0 | 0 | 0 | 0 | 0 | 0 | 0 | 0 |
| 119 | 0 | 0 | 0 | 0 | 0 | 0 | 0 | 0 | 0 |
| 120 | 0 | 0 | 0 | 0 | 0 | 0 | 0 | 0 | 0 |
| 121 | 0 | 0 | 0 | 0 | 1 | 0 | 0 | 0 | 0 |
| 122 | 0 | 0 | 0 | 0 | 1 | 0 | 0 | 0 | 0 |
| 123 | 0 | 0 | 0 | 0 | 1 | 0 | 0 | 0 | 0 |
| 124 | 0 | 0 | 0 | 0 | 0 | 0 | 0 | 0 | 0 |
| 125 | 0 | 0 | 1 | 0 | 1 | 0 | 0 | 0 | 0 |
| 126 | 0 | 0 | 1 | 0 | 1 | 0 | 0 | 0 | 0 |
| 127 | 0 | 0 | 0 | 0 | 0 | 1 | 0 | 0 | 0 |
| 128 | 0 | 0 | 0 | 0 | 0 | 1 | 0 | 0 | 0 |
| 129 | 0 | 0 | 0 | 0 | 0 | 0 | 0 | 0 | 0 |
| 130 | 0 | 0 | 0 | 1 | 0 | 0 | 0 | 0 | 0 |
| 131 | 0 | 0 | 0 | 1 | 0 | 0 | 0 | 0 | 0 |
| 132 | 0 | 0 | 0 | 1 | 0 | 0 | 0 | 0 | 0 |
| 133 | 0 | 0 | 0 | 1 | 0 | 0 | 0 | 0 | 0 |
| 134 | 0 | 0 | 0 | 1 | 0 | 0 | 0 | 0 | 0 |
| 135 | 0 | 0 | 0 | 1 | 0 | 0 | 0 | 0 | 0 |
| 136 | 0 | 0 | 0 | 1 | 0 | 0 | 0 | 0 | 0 |
| 137 | 0 | 0 | 0 | 1 | 0 | 0 | 0 | 0 | 0 |
| 138 | 0 | 0 | 0 | 1 | 0 | 0 | 0 | 0 | 0 |
| 139 | 0 | 0 | 0 | 1 | 0 | 0 | 0 | 0 | 0 |
| 140 | 0 | 0 | 0 | 1 | 0 | 0 | 0 | 0 | 0 |
| 141 | 0 | 0 | 0 | 1 | 0 | 0 | 0 | 0 | 0 |
| 142 | 0 | 0 | 0 | 0 | 0 | 0 | 0 | 0 | 0 |
| 143 | 0 | 0 | 0 | 0 | 0 | 0 | 0 | 0 | 0 |
| 144 | 0 | 0 | 0 | 0 | 0 | 0 | 0 | 0 | 0 |
| 145 | 0 | 0 | 0 | 1 | 0 | 0 | 0 | 0 | 0 |
| 146 | 0 | 0 | 0 | 1 | 0 | 0 | 0 | 0 | 0 |
| 147 | 0 | 0 | 0 | 1 | 0 | 0 | 0 | 0 | 0 |
| 148 | 0 | 0 | 0 | 0 | 0 | 0 | 0 | 0 | 0 |
| 149 | 0 | 0 | 0 | 0 | 0 | 0 | 0 | 0 | 0 |
| 150 | 0 | 0 | 0 | 0 | 0 | 0 | 0 | 0 | 0 |
| 151 | 0 | 0 | 0 | 1 | 0 | 0 | 0 | 0 | 0 |
| 152 | 0 | 0 | 0 | 1 | 0 | 0 | 0 | 0 | 0 |
| 153 | 0 | 0 | 0 | 1 | 0 | 0 | 0 | 0 | 0 |

L'échantillon Ech12 ne présente aucun peptide caractéristique de résistance de type Van. La bactérie présente dans l'échantillon Ech12 peut être sensible aux glycopeptides de type Vancomycine ou Teicoplanine.

Les échantillons Ech19 et Ech20 comportent au moins un peptide caractéristique de VanA. Les bactéries présentes dans les échantillons Ech19 et Ech20 expriment donc la ligase VanA qui leur confère une résistance à la Vancomycine et à la Teicoplanine.

Les échantillons Ech13, Ech14 et Ech18 comportent au moins un peptide caractéristique de VanB. Les bactéries présentes dans les échantillons Ech13, Ech14 et Ech18 expriment donc la ligase VanB qui leur confère une résistance à la Vancomycine.

L'échantillon Ech15 comporte au moins un peptide caractéristique de VanG. La bactérie présente dans l'échantillon Ech15 exprime la ligase VanG qui lui confère une résistance à la Vancomycine.

L'échantillon Ech16 comporte au moins un peptide caractéristique de VanE. La bactérie présente dans l'échantillon Ech16 exprime la ligase VanE qui lui confère une résistance à la Vancomycine.

L'échantillon Ech17 comporte au moins un peptide caractéristique de VanD. La bactérie présente dans l'échantillon Ech17 exprime la ligase VanD qui lui confère une résistance à la Vancomycine et à la Teicoplanine.

Ainsi, de façon avantageuse plusieurs mécanismes de résistance aux glycopeptides peuvent être recherchés simultanément.

### Exemple 10 : Obtention de protéines digérées à partir de microorganismes

Le protocole suivant est mis en oeuvre en 16 étapes :
1. Prélèvement d'une colonie de microorganisme, obtenue selon l'exemple 8 et mise en suspension dans 100µl d'une solution de bicarbonate d'ammonium, 50 mM, pH=8,0 dans un tube contenant des billes de verre de 0.05mm à 2mm de diamètre
2. Ajout de dithiothréitol (DTT) pour obtenir une concentration finale de 5mM.
3. Réduction pendant 5 minutes à 95°C sur une sonde Hielscher (réglage instrument : amplitude :100, cycle :1)
4. Refroidissement des tubes à température ambiante.
5. Ajout d'iodoacétamide pour obtenir une concentration finale de 12.5 mM.
6. Alkylation pendant 5 minutes à température ambiante et à l'abri de la lumière.
7. Ajout de 1 µg de trypsine.
8. Digestion à 50°C pendant 15 minutes
9. Ajout d'acide formique jusqu'à un pH inférieur à 4 pour stopper la réaction.
10. Centrifugation pendant 5 minutes à 15 000g, le surnageant est récupérer et complété à 1 mL avec eau/acide formique 0,5% (v/v)
11. Equilibrage des colonnes HLB Oasis Waters avec 1ml de méthanol puis 1 ml H₂O/acide formique 0,1% (v/v)
12. Dépôt de l'échantillon qui s'écoule par gravité
13. Lavage avec 1 ml H₂O/acide formique 0,1% (v/v)
14. Elution avec 1 ml d'un mélange 80% de méthanol et 20% d'eau/acide formique 0,1% (v/v)
15. L'éluat est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.
16. L'éluat est ensuite repris dans une solution eau/acide formique 0,5% (v/v) quantité suffisante pour (QSP) 150µl

### Exemple 11 : Identification de résistance aux glycopeptides, sans induction, de type VanA:

Les échantillons Ech21 et Ech22 sont identifiés selon l'une des méthodes décrites dans les exemples 1 à 6. L'identification des espèces est reportée dans le **TABLEAU 9.**

**TABLEAU 9 :**

| **Nom** | **Espèces** |
|---|---|
| Ech21 | *E. faecalis* |
| Ech22 | *E*. *faecalis* |

Les échantillons Ech21 et Ech22 correspondent à l'espèce, *Enterococcus faecalis,* pouvant comporter un mécanisme de résistance de type Van. La méthode suivante est alors mise en oeuvre pour rechercher un tel mécanisme.

Chaque échantillon est traité selon l'exemple 10 et analysé selon l'exemple 8 hormis les éléments mentionnés ci-après.

Les transitions mentionnées dans le **TABLEAU 3** sont détectées en utilisant les paramètres figurant dans les **TABLEAUX 10 et 11.**

**TABLEAU 10 :**

| Transition numéro | Temps de rétention | (m/z) filtré en Q1 | (m/z) filtré en Q3 | Energie de collision (eV) |
|---|---|---|---|---|
| 1 | 8,75 | 554,79 | 601,32 | 29 |
| 2 | 8,75 | 554,79 | 730,36 | 29 |
| 3 | 8,75 | 554,79 | 858,42 | 29 |
| 4 | 19,71 | 385,28 | 444,32 | 22 |
| 5 | 19,71 | 385,28 | 543,39 | 22 |
| 6 | 19,71 | 385,28 | 656,47 | 22 |
| 7 | 17,91 | 448,75 | 492,29 | 25 |
| 8 | 17,91 | 448,75 | 655,36 | 25 |
| 9 | 17,91 | 448,75 | 783,41 | 25 |
| 10 | 13,32 | 399,24 | 552,3 | 23 |
| 11 | 13,32 | 399,24 | 529,37 | 23 |
| 12 | 13,32 | 399,24 | 666,43 | 23 |
| 13 | 23,29 | 786,42 | 742,41 | 32 |
| 14 | 23,29 | 786,42 | 855,49 | 31 |
| 15 | 23,29 | 786,42 | 926,53 | 34 |
| 16 | 22,44 | 751,41 | 1146,63 | 38 |
| 17 | 22,44 | 751,41 | 974,55 | 38 |
| 18 | 22,44 | 751,41 | 1075,59 | 38 |
| 19 | 15,89 | 572,32 | 630,36 | 25 |
| 20 | 15,89 | 572,32 | 743,44 | 26 |
| 21 | 15,89 | 572,32 | 872,48 | 25 |
| 22 | 10,67 | 384,7 | 537,3 | 20,5 |
| 23 | 10,67 | 384,7 | 624,34 | 18,5 |
| 24 | 10,67 | 384,7 | 681,36 | 19,5 |
| 25 | 15,29 | 447,77 | 430,3 | 25 |
| 26 | 15,29 | 447,77 | 593,37 | 25 |
| 27 | 15,29 | 447,77 | 694,41 | 25 |
| 28 | 16,64 | 597,78 | 702,35 | 29 |
| 29 | 16,64 | 597,78 | 849,42 | 29 |
| 30 | 16,64 | 597,78 | 980,46 | 27 |
| 31 | 18,52 | 826,89 | 646,35 | 41 |
| 32 | 18,52 | 826,89 | 809,42 | 41 |
| 33 | 18,52 | 826,89 | 924,44 | 41 |
| 34 | 19,2 | 598,83 | 807,5 | 31 |
| 35 | 19,2 | 598,83 | 904,55 | 22 |
| 36 | 19,2 | 598,83 | 452,78 | 25 |
| 37 | 15,89 | 595,81 | 694,41 | 33 |
| 38 | 15,89 | 595,81 | 416,24 | 35 |
| 39 | 15,89 | 595,81 | 464,77 | 28 |
| 40 | 19,1 | 598,35 | 650,4 | 29 |
| 41 | 19,1 | 598,35 | 797,47 | 28 |
| 42 | 19,1 | 598,35 | 868,5 | 28 |
| 43 | 4,97 | 562,27 | 616,29 | 30 |
| 44 | 4,97 | 562,27 | 745,34 | 30 |
| 45 | 4,97 | 562,27 | 873,39 | 30 |
| 46 | 12,85 | 415,24 | 492,29 | 23 |
| 47 | 12,85 | 415,24 | 629,35 | 23 |
| 48 | 12,85 | 415,24 | 716,38 | 23 |
| 49 | 17,9 | 454,78 | 637,39 | 25 |
| 50 | 17,9 | 454,78 | 708,43 | 25 |
| 51 | 17,9 | 454,78 | 821,51 | 25 |
| 52 | 14,32 | 449,76 | 522,3 | 25 |
| 53 | 14,32 | 449,76 | 621,37 | 25 |
| 54 | 14,32 | 449,76 | 660,41 | 25 |
| 55 | 1,53 | 338,19 | 319,2 | 20 |
| 56 | 1,53 | 338,19 | 448,24 | 20 |
| 57 | 1,53 | 338,19 | 576,3 | 20 |
| 58 | 14,16 | 510,78 | 451,27 | 24 |
| 59 | 14,16 | 510,78 | 564,35 | 25 |
| 60 | 14,16 | 510,78 | 763,45 | 27 |
| 61 | 19,45 | 744,72 | 845,39 | 43 |
| 62 | 19,45 | 744,72 | 789,37 | 41 |
| 63 | 19,45 | 744,72 | 917,47 | 40 |
| 64 | 22,56 | 717,43 | 611,35 | 30 |
| 65 | 22,56 | 478,62 | 611,35 | 16 |
| 66 | 22,56 | 478,62 | 407,9 | 19 |
| 67 | 19,53 | 655,98 | 559,34 | 34 |
| 68 | 19,53 | 655,98 | 763,43 | 27 |
| 69 | 19,53 | 655,98 | 891,49 | 25 |
| 70 | 12,98 | 579,26 | 641,33 | 31 |
| 71 | 12,98 | 579,26 | 827,4 | 29 |
| 72 | 12,98 | 579,26 | 928,45 | 27 |
| 73 | 16,92 | 457,77 | 416,26 | 22 |
| 74 | 16,92 | 457,77 | 517,31 | 21 |
| 75 | 16,92 | 457,77 | 687,41 | 22 |
| 76 | 27,6 | 728,74 | 788,89 | 29 |
| 77 | 27,6 | 728,74 | 845,51 | 31 |
| 78 | 27,6 | 728,74 | 1008,57 | 31 |
| 79 | 26,39 | 803,11 | 1028,61 | 35 |
| 80 | 26,39 | 803,11 | 799,5 | 30 |
| 81 | 26,39 | 803,11 | 957,57 | 35 |
| 82 | 11,46 | 597,28 | 522,18 | 26 |
| 83 | 11,46 | 597,28 | 672,37 | 33 |
| 84 | 11,46 | 597,28 | 901,44 | 26 |
| 85 | 26,58 | 822,81 | 604,87 | 30 |
| 86 | 26,58 | 822,81 | 688,91 | 26 |
| 87 | 26,58 | 822,81 | 998,59 | 39 |
| 88 | 19,29 | 754,93 | 598,33 | 30 |
| 89 | 19,29 | 754,93 | 817,43 | 45 |
| 90 | 19,29 | 754,93 | 1027,57 | 38 |
| 91 | 18,18 | 560,29 | 476,27 | 30 |
| 92 | 18,18 | 560,29 | 589,36 | 30 |
| 93 | 18,18 | 560,29 | 775,43 | 30 |
| 94 | 13,88 | 411,74 | 305,18 | 23 |
| 95 | 13,88 | 411,74 | 531,35 | 23 |
| 96 | 13,88 | 411,74 | 659,41 | 23 |
| 97 | 9,97 | 423,23 | 458,31 | 25 |
| 98 | 9,97 | 423,23 | 572,35 | 23 |
| 99 | 9,97 | 423,23 | 701,39 | 21 |
| 100 | 19,92 | 388,74 | 435,27 | 19 |
| 101 | 19,92 | 388,74 | 548,36 | 17 |
| 102 | 19,92 | 388,74 | 663,38 | 19 |
| 103 | 5,57 | 540,28 | 373,21 | 35 |
| 104 | 5,57 | 540,28 | 829,41 | 26 |
| 105 | 5,57 | 540,28 | 483,74 | 29 |
| 106 | 10,45 | 441,72 | 564,31 | 23 |
| 107 | 10,45 | 441,72 | 651,35 | 22 |
| 108 | 10,45 | 441,72 | 738,38 | 21 |
| 109 | 4,52 | 367,19 | 434,22 | 21 |
| 110 | 4,52 | 367,19 | 549,25 | 17 |
| 111 | 4,52 | 367,19 | 662,34 | 18 |
| 112 | 21,88 | 803,84 | 1100,48 | 41 |
| 113 | 21,88 | 803,84 | 730,31 | 38 |
| 114 | 21,88 | 803,84 | 613,27 | 46 |
| 115 | 20,03 | 728,9 | 550,3 | 39 |
| 116 | 20,03 | 728,9 | 834,47 | 41 |
| 117 | 20,03 | 728,9 | 962,53 | 39 |
| 118 | 16,81 | 672,3 | 769,35 | 26 |
| 119 | 16,81 | 672,3 | 882,44 | 26 |
| 120 | 16,81 | 672,3 | 969,47 | 26 |
| 121 | 13,59 | 351,23 | 444,32 | 18 |
| 122 | 13,59 | 351,23 | 543,39 | 16 |
| 123 | 13,59 | 351,23 | 614,42 | 17 |
| 124 | 14,51 | 503,27 | 720,4 | 28 |
| 125 | 14,51 | 503,27 | 817,45 | 19 |
| 126 | 14,51 | 503,27 | 409,23 | 20 |
| 127 | 22,39 | 722,92 | 428,29 | 37 |
| 128 | 22,39 | 722,92 | 854,53 | 37 |
| 129 | 22,39 | 722,92 | 955,58 | 37 |
| 130 | 12,77 | 434,23 | 565,33 | 24 |
| 131 | 12,77 | 434,23 | 739,4 | 21 |
| 132 | 12,77 | 434,23 | 796,42 | 22 |
| 133 | 12,41 | 477,73 | 553,27 | 27 |
| 134 | 12,41 | 477,73 | 713,3 | 26 |
| 135 | 12,41 | 477,73 | 770,33 | 24 |
| 136 | 7,63 | 388,2 | 476,24 | 22 |
| 137 | 7,63 | 388,2 | 547,27 | 19 |
| 138 | 7,63 | 388,2 | 662,3 | 19 |
| 139 | 12,85 | 375,7 | 343,21 | 22 |
| 140 | 12,85 | 375,7 | 474,25 | 22 |
| 141 | 12,85 | 375,7 | 637,31 | 22 |
| 142 | 20,72 | 403,74 | 397,28 | 23 |
| 143 | 20,72 | 403,74 | 560,34 | 23 |
| 144 | 20,72 | 403,74 | 659,41 | 23 |
| 145 | 19,66 | 542,34 | 600,37 | 23 |
| 146 | 19,66 | 542,34 | 671,41 | 25 |
| 147 | 19,66 | 542,34 | 871,52 | 23 |
| 148 | 22,14 | 939,94 | 1180,52 | 46 |
| 149 | 22,14 | 939,94 | 540,27 | 46 |
| 150 | 22,14 | 939,94 | 802,36 | 46 |
| 151 | 12,66 | 392,18 | 409,19 | 22 |
| 152 | 12,66 | 392,18 | 523,24 | 22 |
| 153 | 12,66 | 392,18 | 620,29 | 22 |

**TABLEAU 11 :**

| Transition numéro | Potentiel d'orifice | potentiel d'entrée avant Q0 | Potentiel en sortie de cellule de collision | Seuil de positivité |
|---|---|---|---|---|
| 1 | 80 | 10 | 35 | 2000 |
| 2 | 80 | 10 | 35 | 2000 |
| 3 | 80 | 10 | 35 | 2000 |
| 4 | 80 | 10 | 35 | 600 |
| 5 | 80 | 10 | 35 | 2000 |
| 6 | 80 | 10 | 35 | 1000 |
| 7 | 80 | 10 | 35 | 2000 |
| 8 | 80 | 10 | 35 | 2000 |
| 9 | 80 | 10 | 35 | 2000 |
| 10 | 80 | 10 | 35 | 2000 |
| 11 | 80 | 10 | 35 | 2000 |
| 12 | 80 | 10 | 35 | 2000 |
| 13 | 120 | 10 | 18 | 2000 |
| 14 | 120 | 10 | 18 | 2000 |
| 15 | 120 | 10 | 18 | 2000 |
| 16 | 80 | 10 | 35 | 2000 |
| 17 | 80 | 10 | 35 | 2000 |
| 18 | 80 | 10 | 35 | 2000 |
| 19 | 90 | 10 | 15 | 2000 |
| 20 | 90 | 10 | 15 | 2000 |
| 21 | 90 | 10 | 15 | 2000 |
| 22 | 85 | 10 | 24 | 2000 |
| 23 | 85 | 10 | 24 | 2000 |
| 24 | 85 | 10 | 24 | 2000 |
| 25 | 80 | 10 | 35 | 2000 |
| 26 | 80 | 10 | 35 | 2000 |
| 27 | 80 | 10 | 35 | 2000 |
| 28 | 100 | 10 | 17 | 2000 |
| 29 | 100 | 10 | 17 | 2000 |
| 30 | 100 | 10 | 17 | 2000 |
| 31 | 80 | 10 | 35 | 2000 |
| 32 | 80 | 10 | 35 | 2000 |
| 33 | 80 | 10 | 35 | 2000 |
| 34 | 105 | 10 | 20 | 2000 |
| 35 | 105 | 10 | 20 | 2000 |
| 36 | 105 | 10 | 20 | 2000 |
| 37 | 100 | 10 | 11 | 2000 |
| 38 | 100 | 10 | 16 | 2000 |
| 39 | 100 | 10 | 11 | 2000 |
| 40 | 90 | 10 | 16 | 2000 |
| 41 | 90 | 10 | 18 | 2000 |
| 42 | 90 | 10 | 22 | 2000 |
| 43 | 80 | 10 | 35 | 2000 |
| 44 | 80 | 10 | 35 | 2000 |
| 45 | 80 | 10 | 35 | 2000 |
| 46 | 80 | 10 | 35 | 2000 |
| 47 | 80 | 10 | 35 | 2000 |
| 48 | 80 | 10 | 35 | 2000 |
| 49 | 80 | 10 | 35 | 2000 |
| 50 | 80 | 10 | 35 | 2000 |
| 51 | 80 | 10 | 35 | 2000 |
| 52 | 80 | 10 | 35 | 2000 |
| 53 | 80 | 10 | 35 | 2000 |
| 54 | 80 | 10 | 35 | 2000 |
| 55 | 80 | 10 | 35 | 2000 |
| 56 | 80 | 10 | 35 | 2000 |
| 57 | 80 | 10 | 35 | 2000 |
| 58 | 75 | 12 | 11 | 2000 |
| 59 | 75 | 12 | 14 | 2000 |
| 60 | 75 | 12 | 18 | 2000 |
| 61 | 140 | 10 | 35 | 2000 |
| 62 | 140 | 10 | 35 | 2000 |
| 63 | 140 | 10 | 35 | 2000 |
| 64 | 120 | 10 | 35 | 2000 |
| 65 | 70 | 10 | 35 | 2000 |
| 66 | 70 | 10 | 35 | 2000 |
| 67 | 100 | 10 | 35 | 2000 |
| 68 | 100 | 10 | 35 | 2000 |
| 69 | 100 | 10 | 35 | 2000 |
| 70 | 80 | 12 | 15 | 3500 |
| 71 | 80 | 12 | 19 | 2500 |
| 72 | 80 | 12 | 22 | 4500 |
| 73 | 70 | 12 | 10 | 2000 |
| 74 | 70 | 12 | 13 | 2000 |
| 75 | 70 | 12 | 16 | 2000 |
| 76 | 120 | 10 | 35 | 2000 |
| 77 | 120 | 10 | 35 | 2000 |
| 78 | 120 | 10 | 35 | 2000 |
| 79 | 120 | 10 | 35 | 2000 |
| 80 | 120 | 10 | 35 | 2000 |
| 81 | 120 | 10 | 35 | 2000 |
| 82 | 85 | 12 | 13 | 2000 |
| 83 | 85 | 12 | 16 | 2000 |
| 84 | 85 | 12 | 21 | 2000 |
| 85 | 100 | 10 | 35 | 2000 |
| 86 | 100 | 10 | 35 | 2000 |
| 87 | 100 | 10 | 35 | 2000 |
| 88 | 100 | 10 | 35 | 2000 |
| 89 | 100 | 10 | 35 | 4000 |
| 90 | 100 | 10 | 35 | 2000 |
| 91 | 80 | 10 | 35 | 4000 |
| 92 | 80 | 10 | 35 | 3500 |
| 93 | 80 | 10 | 35 | 6000 |
| 94 | 80 | 10 | 35 | 2000 |
| 95 | 80 | 10 | 35 | 2000 |
| 96 | 80 | 10 | 35 | 2000 |
| 97 | 75 | 10 | 11 | 2000 |
| 98 | 75 | 10 | 14 | 2000 |
| 99 | 75 | 10 | 16 | 2000 |
| 100 | 140 | 6 | 10 | 2000 |
| 101 | 140 | 6 | 14 | 2000 |
| 102 | 140 | 6 | 16 | 2000 |
| 103 | 110 | 12 | 10 | 2000 |
| 104 | 110 | 12 | 20 | 2000 |
| 105 | 110 | 12 | 6 | 2000 |
| 106 | 75 | 12 | 14 | 2000 |
| 107 | 75 | 12 | 16 | 2000 |
| 108 | 75 | 12 | 18 | 2000 |
| 109 | 65 | 12 | 18 | 2000 |
| 110 | 65 | 12 | 23 | 2000 |
| 111 | 65 | 12 | 15 | 2000 |
| 112 | 100 | 10 | 35 | 2000 |
| 113 | 100 | 10 | 35 | 2000 |
| 114 | 100 | 10 | 35 | 2000 |
| 115 | 140 | 9 | 14 | 2000 |
| 116 | 140 | 9 | 34 | 2000 |
| 117 | 140 | 9 | 24 | 2000 |
| 118 | 125 | 9 | 18 | 2000 |
| 119 | 125 | 9 | 21 | 2000 |
| 120 | 125 | 9 | 41 | 2000 |
| 121 | 70 | 12 | 10 | 2000 |
| 122 | 70 | 12 | 23 | 2000 |
| 123 | 70 | 12 | 14 | 2000 |
| 124 | 70 | 12 | 17 | 2500 |
| 125 | 70 | 12 | 20 | 2500 |
| 126 | 70 | 12 | 10 | 2500 |
| 127 | 80 | 10 | 35 | 2000 |
| 128 | 80 | 10 | 35 | 2000 |
| 129 | 80 | 10 | 35 | 2000 |
| 130 | 70 | 12 | 14 | 2000 |
| 131 | 70 | 12 | 18 | 2000 |
| 132 | 70 | 12 | 19 | 2000 |
| 133 | 80 | 12 | 14 | 2000 |
| 134 | 80 | 12 | 18 | 2000 |
| 135 | 80 | 12 | 18 | 2000 |
| 136 | 70 | 12 | 12 | 2000 |
| 137 | 70 | 12 | 13 | 2000 |
| 138 | 70 | 12 | 16 | 2000 |
| 139 | 80 | 10 | 35 | 2000 |
| 140 | 80 | 10 | 35 | 2000 |
| 141 | 80 | 10 | 35 | 2000 |
| 142 | 80 | 10 | 35 | 2000 |
| 143 | 80 | 10 | 35 | 2000 |
| 144 | 80 | 10 | 35 | 2000 |
| 145 | 80 | 12 | 16 | 2000 |
| 146 | 80 | 12 | 14 | 2000 |
| 147 | 80 | 12 | 21 | 2000 |
| 148 | 80 | 10 | 35 | 2000 |
| 149 | 80 | 10 | 35 | 2000 |
| 150 | 80 | 10 | 35 | 2000 |
| 151 | 80 | 10 | 35 | 2800 |
| 152 | 80 | 10 | 35 | 2000 |
| 153 | 80 | 10 | 35 | 2000 |

• Les autres paramètres machine utilisés sont les suivants :

| | |
|---|---|
| Type de balayage: | MRM |
| MRM planifié: | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 550,00 °C |
| Gaz de nébulisation: | 50,00 psi |
| Gaz chauffant: | 40,00 psi |
| Gaz de collision induisant dissociation : | 9,00 psi |
| Remplissage dynamique: | inactivé |
| Temps de cycle total: | 1.2 sec |
| Fenêtre de détection: | 90 sec |

Les aires obtenues pour chacune des transitions et pour chacun des microorganismes étudiés ont été mesurées. Lorsque l'aire d'une transition est supérieure ou égale au seuil de positivité décrit dans le **TABLEAU 11,** la détection de la transition est considérée comme positive et est notée « 1 » dans le **TABLEAU 12.** Lorsque l'aire d'une transition est inférieure au seuil de positivité décrit dans le **TABLEAU 11,** la détection de la transition est considérée comme négative et est notée « 0 » dans le **TABLEAU 12.** Lorsque les 3 transitions d'un même peptide sont notées « 1 », la détection du peptide est considérée comme positive. Un cas particulier fait exception à cette règle. Le peptide correspondant aux transitions numéros 49, 50 et 51 a une transition de très faible intensité. Dans ce cas précis, lorsque les transitions 49 et 50 sont notées « 1 », la détection de ce peptide sera considérée comme positive.

**TABLEAU 12 :**

| Transition numéro | Ech21 | Ech22 |
|---|---|---|
| 1 | 0 | 1 |
| 2 | 0 | 1 |
| 3 | 0 | 1 |
| 4 | 1 | 1 |
| 5 | 1 | 1 |
| 6 | 1 | 1 |
| 7 | 1 | 1 |
| 8 | 1 | 1 |
| 9 | 1 | 1 |
| 10 | 1 | 1 |
| 11 | 1 | 1 |
| 12 | 1 | 1 |
| 13 | 0 | 1 |
| 14 | 0 | 1 |
| 15 | 0 | 1 |
| 16 | 0 | 1 |
| 17 | 0 | 1 |
| 18 | 0 | 1 |
| 19 | 1 | 1 |
| 20 | 1 | 1 |
| 21 | 1 | 1 |
| 22 | 1 | 1 |
| 23 | 1 | 1 |
| 24 | 1 | 1 |
| 25 | 0 | 1 |
| 26 | 0 | 1 |
| 27 | 0 | 1 |
| 28 | 0 | 1 |
| 29 | 0 | 1 |
| 30 | 0 | 1 |
| 31 | 0 | 1 |
| 32 | 0 | 1 |
| 33 | 0 | 1 |
| 34 | 1 | 1 |
| 35 | 1 | 1 |
| 36 | 1 | 1 |
| 37 | 0 | 0 |
| 38 | 0 | 0 |
| 39 | 0 | 0 |
| 40 | 0 | 0 |
| 41 | 0 | 0 |
| 42 | 0 | 0 |
| 43 | 0 | 0 |
| 44 | 0 | 0 |
| 45 | 0 | 0 |
| 46 | 0 | 0 |
| 47 | 0 | 0 |
| 48 | 0 | 0 |
| 49 | 0 | 0 |
| 50 | 0 | 0 |
| 51 | 0 | 0 |
| 52 | 0 | 0 |
| 53 | 0 | 0 |
| 54 | 0 | 0 |
| 55 | 0 | 0 |
| 56 | 0 | 0 |
| 57 | 0 | 0 |
| 58 | 0 | 0 |
| 59 | 0 | 0 |
| 60 | 0 | 0 |
| 61 | 0 | 0 |
| 62 | 0 | 0 |
| 63 | 0 | 0 |
| 64 | 0 | 0 |
| 65 | 0 | 0 |
| 66 | 0 | 0 |
| 67 | 0 | 0 |
| 68 | 0 | 0 |
| 69 | 0 | 0 |
| 70 | 0 | 0 |
| 71 | 0 | 0 |
| 72 | 0 | 0 |
| 73 | 0 | 0 |
| 74 | 0 | 0 |
| 75 | 0 | 0 |
| 76 | 0 | 0 |
| 77 | 0 | 0 |
| 78 | 0 | 0 |
| 79 | 0 | 0 |
| 80 | 0 | 0 |
| 81 | 0 | 0 |
| 82 | 0 | 0 |
| 83 | 0 | 0 |
| 84 | 0 | 0 |
| 85 | 0 | 0 |
| 86 | 0 | 0 |
| 87 | 0 | 0 |
| 88 | 0 | 0 |
| 89 | 0 | 0 |
| 90 | 0 | 0 |
| 91 | 0 | 0 |
| 92 | 0 | 0 |
| 93 | 0 | 0 |
| 94 | 0 | 0 |
| 95 | 0 | 0 |
| 96 | 0 | 0 |
| 97 | 0 | 0 |
| 98 | 0 | 0 |
| 99 | 0 | 0 |
| 100 | 0 | 0 |
| 101 | 0 | 0 |
| 102 | 0 | 0 |
| 103 | 0 | 0 |
| 104 | 0 | 0 |
| 105 | 0 | 0 |
| 106 | 0 | 0 |
| 107 | 0 | 0 |
| 108 | 0 | 0 |
| 109 | 0 | 0 |
| 110 | 0 | 0 |
| 111 | 0 | 0 |
| 112 | 0 | 0 |
| 113 | 0 | 0 |
| 114 | 0 | 0 |
| 115 | 0 | 0 |
| 116 | 0 | 0 |
| 117 | 0 | 0 |
| 118 | 0 | 0 |
| 119 | 0 | 0 |
| 120 | 0 | 0 |
| 121 | 0 | 0 |
| 122 | 0 | 0 |
| 123 | 0 | 0 |
| 124 | 0 | 0 |
| 125 | 0 | 0 |
| 126 | 0 | 0 |
| 127 | 0 | 0 |
| 128 | 0 | 0 |
| 129 | 0 | 0 |
| 130 | 0 | 0 |
| 131 | 0 | 0 |
| 132 | 0 | 0 |
| 133 | 0 | 0 |
| 134 | 0 | 0 |
| 135 | 0 | 0 |
| 136 | 0 | 0 |
| 137 | 0 | 0 |
| 138 | 0 | 0 |
| 139 | 0 | 0 |
| 140 | 0 | 0 |
| 141 | 0 | 0 |
| 142 | 0 | 0 |
| 143 | 0 | 0 |
| 144 | 0 | 0 |
| 145 | 0 | 0 |
| 146 | 0 | 0 |
| 147 | 0 | 0 |
| 148 | 0 | 0 |
| 149 | 0 | 0 |
| 150 | 0 | 0 |
| 151 | 0 | 0 |
| 152 | 0 | 0 |
| 153 | 0 | 0 |

Les échantillons Ech21 et Ech22 comportent au moins un peptide caractéristique de VanA. Les bactéries présentes dans les échantillons Ech21 et Ech22 expriment la ligase VanA qui leur confère une résistance à la Vancomycine et à la Teicoplanine.

Ainsi, de façon particulièrement avantageuse, un mécanisme de résistance inductible, tel que le mécanisme de résistance lié à l'expression de VanA, est détecté sans phase d'induction lors de la culture du microorganisme, après une phase de préparation d'échantillon réalisée en moins de 3 heures. Ce procédé permet donc de rechercher rapidement les mécanismes de résistance aux glycopeptides sans *a priori* sur leur éventuel existence.

### Exemple 12 : Identification de résistance aux glycopeptides, sans Induction :

Les échantillons Ech23 et Ech24 sont identifiés selon l'une des méthodes décrites dans les exemples 1 à 6. L'identification des espèces est reportée dans le **TABLEAU 13.**

**TABLEAU 13 :**

| **Nom** | **Espèces** |
|---|---|
| Ech23 | *E. faecium* |
| Ech24 | *E. faecalis* |
| Echt25 | *E. faecium* |

Les échantillons Ech23, Ech24 et Ech25 correspondent à l'espèce, *Enterococcus Faecalis ou Enterococcus faecium,* pouvant comporter un mécanisme de résistance de type Van.

La méthode suivante est alors mise en oeuvre pour rechercher un tel mécanisme. Chaque échantillon est traité selon l'exemple 10 avec les modifications suivantes :
- Etape 1 : une ligne de colonie de 3 cm est prélevée avec une oese de 10µl (contre une colonie précédemment).
- Etape 7 : ajout de 2 µg de trypsine.

Chaque échantillon est analysé selon l'exemple 8 hormis les éléments mentionnés ci-après.

Les transitions mentionnées dans le **TABLEAU 3** sont détectées en utilisant les paramètres figurant dans les **TABLEAUX 4 et 11.**

Comme dans l'exemple 8 les aires positives sont notées « 1 » dans le **TABLEAU 14.** Les peptides positifs sont détectés selon les règles de l'exemple 8.

**TABLEAU 14 :**

| Transition numéro | **Ech23** | **Ech24** | **Ech25** |
|---|---|---|---|
| 1 | 0 | 0 | 1 |
| 2 | 0 | 0 | 1 |
| 3 | 0 | 0 | 1 |
| 4 | 0 | 0 | 1 |
| 5 | 0 | 0 | 1 |
| 6 | 0 | 0 | 1 |
| 7 | 0 | 0 | 1 |
| 8 | 0 | 0 | 1 |
| 9 | 0 | 0 | 1 |
| 10 | 0 | 0 | 1 |
| 11 | 0 | 0 | 1 |
| 12 | 0 | 0 | 1 |
| 13 | 0 | 0 | 0 |
| 14 | 0 | 0 | 0 |
| 15 | 0 | 0 | 0 |
| 16 | 0 | 0 | 0 |
| 17 | 0 | 0 | 0 |
| 18 | 0 | 0 | 0 |
| 19 | 0 | 0 | 1 |
| 20 | 0 | 0 | 1 |
| 21 | 0 | 0 | 1 |
| 22 | 0 | 0 | 1 |
| 23 | 0 | 0 | 1 |
| 24 | 0 | 0 | 1 |
| 25 | 0 | 0 | 1 |
| 26 | 0 | 0 | 1 |
| 27 | 0 | 0 | 1 |
| 28 | 0 | 0 | 1 |
| 29 | 0 | 0 | 1 |
| 30 | 0 | 0 | 1 |
| 31 | 0 | 0 | 1 |
| 32 | 0 | 0 | 1 |
| 33 | 0 | 0 | 1 |
| 34 | 0 | 0 | 1 |
| 35 | 0 | 0 | 1 |
| 36 | 0 | 0 | 1 |
| 37 | 1 | 0 | 0 |
| 38 | 1 | 0 | 0 |
| 39 | 1 | 0 | 0 |
| 40 | 1 | 0 | 0 |
| 41 | 1 | 0 | 0 |
| 42 | 1 | 0 | 0 |
| 43 | 1 | 0 | 0 |
| 44 | 1 | 0 | 0 |
| 45 | 1 | 0 | 0 |
| 46 | 1 | 0 | 0 |
| 47 | 1 | 0 | 0 |
| 48 | 1 | 0 | 0 |
| 49 | 1 | 0 | 0 |
| 50 | 1 | 0 | 0 |
| 51 | 1 | 0 | 0 |
| 52 | 1 | 0 | 0 |
| 53 | 1 | 0 | 0 |
| 54 | 1 | 0 | 0 |
| 55 | 1 | 0 | 0 |
| 56 | 1 | 0 | 0 |
| 57 | 1 | 0 | 0 |
| 58 | 0 | 0 | 0 |
| 59 | 0 | 0 | 0 |
| 60 | 0 | 0 | 0 |
| 61 | 0 | 0 | 0 |
| 62 | 0 | 0 | 0 |
| 63 | 0 | 0 | 0 |
| 64 | 0 | 0 | 0 |
| 65 | 0 | 0 | 0 |
| 66 | 0 | 0 | 0 |
| 67 | 0 | 0 | 0 |
| 68 | 0 | 0 | 0 |
| 69 | 0 | 0 | 0 |
| 70 | 0 | 0 | 0 |
| 71 | 0 | 0 | 0 |
| 72 | 0 | 0 | 0 |
| 73 | 0 | 0 | 0 |
| 74 | 0 | 0 | 0 |
| 75 | 0 | 0 | 0 |
| 76 | 0 | 0 | 0 |
| 77 | 0 | 0 | 0 |
| 78 | 0 | 0 | 0 |
| 79 | 0 | 0 | 0 |
| $80 | 0 | 0 | 0 |
| 81 | 0 | 0 | 0 |
| 82 | 0 | 0 | 0 |
| 83 | 0 | 0 | 0 |
| 84 | 0 | 0 | 0 |
| 85 | 0 | 0 | 0 |
| 86 | 0 | 0 | 0 |
| 87 | 0 | 0 | 0 |
| 88 | 0 | 0 | 0 |
| 89 | 0 | 0 | 0 |
| 90 | 0 | 0 | 0 |
| 91 | 1 | 0 | 1 |
| 92 | 1 | 0 | 1 |
| 93 | 0 | 0 | 0 |
| 94 | 0 | 0 | 0 |
| 95 | 0 | 0 | 0 |
| 96 | 0 | 0 | 0 |
| 97 | 0 | 0 | 0 |
| 98 | 0 | 0 | 0 |
| 99 | 0 | 0 | 0 |
| 100 | 0 | 0 | 0 |
| 101 | 0 | 0 | 0 |
| 102 | 0 | 0 | 0 |
| 103 | 0 | 0 | 0 |
| 104 | 0 | 0 | 0 |
| 105 | 0 | 0 | 0 |
| 106 | 0 | 0 | 0 |
| 107 | 0 | 0 | 0 |
| 108 | 0 | 0 | 0 |
| 109 | 0 | 0 | 0 |
| 110 | 0 | 0 | 0 |
| 111 | 0 | 0 | 0 |
| 112 | 0 | 0 | 0 |
| 113 | 0 | 0 | 0 |
| 114 | 0 | 0 | 0 |
| 115 | 0 | 0 | 0 |
| 116 | 0 | 0 | 0 |
| 117 | 0 | 0 | 0 |
| 118 | 0 | 0 | 0 |
| 119 | 0 | 0 | 0 |
| 120 | 0 | 0 | 0 |
| 121 | 0 | 0 | 0 |
| 122 | 0 | 0 | 0 |
| 123 | 0 | 0 | 0 |
| 124 | 0 | 0 | 0 |
| 125 | 0 | 0 | 0 |
| 126 | 0 | 0 | 0 |
| 127 | 0 | 0 | 0 |
| 128 | 0 | 0 | 0 |
| 129 | 0 | 0 | 0 |
| 130 | 0 | 0 | 0 |
| 131 | 0 | 0 | 0 |
| 132 | 0 | 0 | 0 |
| 133 | 0 | 0 | 0 |
| 134 | 0 | 0 | 0 |
| 135 | 0 | 0 | 0 |
| 136 | 0 | 0 | 0 |
| 137 | 0 | 0 | 0 |
| 138 | 0 | 0 | 0 |
| 139 | 0 | 0 | 0 |
| 140 | 0 | 0 | 0 |
| 141 | 0 | 0 | 0 |
| 142 | 0 | 0 | 0 |
| 143 | 0 | 0 | 0 |
| 144 | 0 | 0 | 0 |
| 145 | 0 | 0 | 0 |
| 146 | 0 | 0 | 0 |
| 147 | 0 | 0 | 0 |
| 148 | 0 | 0 | 0 |
| 149 | 0 | 0 | 0 |
| 150 | 0 | 0 | 0 |
| 151 | 0 | 0 | 0 |
| 152 | 0 | 0 | 0 |
| 153 | 0 | 0 | 0 |

L'échantillon Ech23 comporte au moins un peptide caractéristique de VanB. La bactérie présente dans l'échantillon Ech23 exprime la ligase VanB qui lui confère une résistance à la Vancomycine.

L'échantillon Ech25 comporte au moins un peptide caractéristique de VanA. La bactérie présente dans l'échantillon Ech25 exprime la ligase VanA qui lui confère une résistance à la Vancomycine et à la Teicoplanine.

A l'inverse l'échantillon Ech24 ne présente aucun peptide caractéristique de résistance de type Van. La bactérie présente dans l'échantillon Ech24 peut être sensible aux glycopeptides de type Vancomycine ou Teicoplanine.

Ainsi, de façon particulièrement avantageuse, un mécanisme de résistance inductible, tel que le mécanisme de résistance lié à l'expression de VanB, est détecté sans phase d'induction lors de la culture du microorganisme, après une phase de préparation d'échantillon réalisée en moins de 3 heures. De façon également très avantageuse, tous les mécanismes de résistance aux glycopeptides peuvent être recherchés et détectés simultanément. Ainsi 2 mécanismes de résistance, VanB et VanA sont détectés respectivement dans les échantillons Ech23 et Ech25 en utilisant la même méthode.

Ce procédé permet donc de rechercher rapidement les mécanismes de résistance aux glycopeptides sans *a priori* sur leur éventuel existence.

### Références bibliographiques

[1] J. Anhalt & C. Fenselau, 1975, Anal. Chem., 47(2) :219-225.
[2] A. Fox et al, ed., 1990, Analytical microbiology methods : chromatography and mass spectrometry, Plenum Press, New York, N.Y.
[3] M. Claydon et al, 1996, Nature Biotech. 14 :1584-1586.
[4] T. Krishnamurthy & P. Ross, 1996, Rapid Com. Mass Spec., 10 :1992-1996.
[5] P. Seng et al. 2009, Clin. Infect. Dis., 49 :543-551.
[6] C. Fenselau et al., 2008, Appl. Environ. Microbiol., 904-906.
[7] S. Hofstadler et al., 2005, Int..J Mass Spectrom., 242:23-41.
[8] D. Ecker, 2008, Nat. Rev. Microbiol., 6(7):553-558.
[9] Sujatha,S. & Praharaj,l. Glycopeptide Résistance in Gram-Positive Cocci: A Review. Interdiscip. Perspect. Infect. Dis 2012, 781679 (2012
[10] W.-J. Chen et al., 2008, Anal. Chem., 80 : 9612-9621
[11] D. Lopez-Ferrer et al., 2008, Anal. Chem., 80 :8930-8936
[12] D. Lopez-Ferrer et al., 2005, J. Proteome res., 4(5) : 1569-1574
[13] T. Fortin et al., 2009, Mol. Cell Proteomics, 8(5) : 1006-1015.
[14] H. Keshishian et al., 2007, Mol. Cell Proteomics, 2212-2229.
[15] J. Stal-Zeng et al., 2007, Mol. Cell Proteomics, 1809-1817.
[16] Gaskell, Electrospray: principles and practise, 1997, J. Mass Spectrom., 32, 677-688).
[17] V. Fusaro et al., 2009, Nature Biotech. 27, 190-198.
[18] J. Mead et al., 15 nov 2008, Mol. Cell Proteomics, E-pub.
[19] F. Desiere et al., 2006, Nucleic Acids Res., 34(database issue) : D655-8).
[20] L. Anderson & C. Hunter, 2006, Mol. Cell Proteomics, 573-588).
[21] B. Han & R. Higgs, 2008, Brief Funct Genomic Proteomic.,7(5):340-54).
[22] K.-Y. Wang et al., 2008, Anal Chem, 80(16) 6159-6167).
[23] J. Bundy & C. Fenselau, 1999, Anal. Chem. 71 : 1460-1463.
[24] K-C Ho et al., 2004, Anal. Chem. 76 : 7162-7268.
[25] Y.S. Lin et al., 2005, Anal. Chem., 77 : 1753-1760.
[26] S. Vaidyanathan et al., 2001, Anal. Chem., 73 :4134-4144.
[27] R. Everley et al., 2009, J. Microbiol. Methods, 77:152-158.
[28] Mânes N. et al., 2007, Mol. & Cell. Proteomics, 6(4): 717-727.
[29] R. Nandakumar et al., 2009, Oral Microbiology Immunology, 24 :347-352).
[30] L. Hernychova et al., 2008, Anal. Chem., 80 :7097-7104.
[31] J.-M. Pratt et al., 2006, Nat. Protoc., 1:1029-1043.
[32] V. Brun et al., 2007, Mol. Cell Proteomics, 2139-2149.

## Revendications

1. Procédé de détection, pour au moins un microorganisme compris dans un échantillon, d'au moins un marqueur de résistance à un glycopeptide qui est la vancomycine, comprenant la détection, par spectrométrie de masse de type MRM, d'au moins un peptide dudit microorganisme, qui sont des peptides de type Van choisis parmi les peptides de séquence SEQ ID N° 5 à 16, 29 à 35, 59 à 71, 80 à 83, 85 à 91, 95 à 102, 104 à 120, 122 à 139 ou 141 à 154.

2. Procédé de détection selon l'une des revendications 1, comprenant une étape préalable à l'étape de détection consistant en l'induction du mécanisme de résistance par mise en contact préalable dudit échantillon avec ledit glycopeptide.

3. Procédé de détection selon la revendication 1, dans lequel lesdits marqueurs de résistance sont des protéines de type VanA choisis parmi les peptides de séquence SEQ ID N° 5 à SEQ ID N° 16.

4. Procédé de détection selon la revendication 3, dans lequel lesdits marqueurs de résistance sont des marqueurs de type VanA choisis parmi les peptides de séquence SEQ ID N° 6, SEQ ID N° 7, SEQ ID N° 8, SEQ ID N° 11, SEQ ID N° 12, SEQ ID N° 13, SEQ ID N° 15, SEQ ID N° 16.

5. Procédé de détection selon la revendication 2, dans lequel lesdits marqueurs de résistance sont des peptides de type VanA choisis parmi les peptides de séquence SEQ ID N° 5 à SEQ ID N° 16.

6. Procédé de détection selon la revendication 1, dans lequel lesdits marqueurs de résistance sont des marqueurs de type VanB choisis parmi les peptides de séquence SEQ ID N° 29 à SEQ ID N° 35.

7. Procédé de détection selon la revendication 2, dans lequel lesdits marqueurs de résistance sont des marqueurs de type VanB choisis parmi les peptides de séquence SEQ ID N° 29, SEQ ID N° 30, SEQ ID N° 32 et SEQ ID N° 33.

8. Procédé de détection selon la revendication 1, dans lequel lesdits marqueurs de résistance sont des marqueurs de type VanC choisis parmi les peptides de séquence SEQ ID N° 59 à SEQ ID N° 71.

9. Procédé de détection selon la revendication 1, dans lequel lesdits marqueurs de résistance sont des marqueurs de type VanD choisis parmi les peptides de séquence SEQ ID N° 80 à SEQ ID N° 83.

10. Procédé de détection selon la revendication 9, dans lequel lesdits marqueurs de résistance sont des marqueurs de type VanD choisis parmi les peptides de séquence SEQ ID N° 80, SEQ ID N° 81 et SEQ ID N° 83.

11. Procédé de détection selon la revendication 2, dans lequel lesdits marqueurs de résistance sont des marqueurs de type VanD choisis parmi les peptides de séquence SEQ ID N° 80, SEQ ID N° 81 et SEQ ID N° 83

12. Procédé de détection selon la revendication 1, dans lequel lesdits marqueurs de résistance sont des marqueurs de type VanE choisis parmi les peptides de séquence SEQ ID N° 85 à SEQ ID N° 91.

13. Procédé de détection selon la revendication 2, dans lequel ledit marqueur de résistance est un marqueur de type VanE correspondant au peptide de séquence SEQ ID N° 89.

14. Procédé de détection selon la revendication 1, dans lequel lesdits marqueurs de résistance sont des marqueurs de type VanG choisis parmi les peptides de séquence SEQ ID N° 95 à SEQ ID N° 102.

15. Procédé de détection selon la revendication 14, dans lequel lesdits marqueurs de résistance sont des marqueurs de type VanG choisis parmi les peptides SEQ ID N° 97 et SEQ ID N° 100.

16. Procédé de détection selon la revendication 2, dans lequel lesdits marqueurs de résistance sont des marqueurs de type VanG choisis parmi les peptides de séquence SEQ ID N° 95, SEQ ID N° 96, SEQ ID N° 97, SEQ ID N° 98, SEQ ID N° 100 et SEQ ID N° 102.

17. Procédé de détection selon la revendication 1, dans lequel lesdits marqueurs de résistance sont des marqueurs de type VanL choisis parmi les peptides de séquence SEQ ID N° 104 à SEQ ID N° 120.

18. Procédé de détection selon la revendication 1, dans lequel lesdits marqueurs de résistance sont des marqueurs de type VanM choisis parmi les peptides de séquence SEQ ID N° 122 à SEQ ID N° 139.

19. Procédé de détection selon la revendication 1, dans lequel lesdits marqueurs de résistance sont des marqueurs de type VanN choisis parmi les peptides de séquence SEQ ID N° 141 à SEQ ID N° 154.

## Patentansprüche

1. Verfahren zum Nachweisen von mindestens einem Marker für Resistenz gegen ein Glykopeptid, bei dem es sich um Vancomycin handelt, bei mindestens einem in einer Probe befindlichen Mikroorganismus umfassend den Nachweis von mindestens einem Peptid, wobei es sich um Peptide des Van-Typs ausgewählt aus den Peptiden der Sequenz SEQ ID Nr. 5 bis 16, 29 bis 35, 59 bis 71, 80 bis 83, 85 bis 91, 95 bis 102, 104 bis 120, 122 bis 139 oder 141 bis 154' handelt, des Mikroorganismus mittels MRM-Typ-Massenspektrometrie.

2. Verfahren zum Nachweisen nach einem der Ansprüche 1, umfassend einen Schritt vor dem Schritt des Nachweisens, der darin besteht, dass man den Resistenzmechanismus dadurch induziert, dass man die Probe zuvor mit dem Glykopeptid in Kontakt bringt.

3. Verfahren zum Nachweisen nach Anspruch 1, bei dem die Resistenzmarker Proteine des VanA-Typs sind, die aus den Peptiden der Sequenz SEQ ID Nr. 5 bis SEQ ID Nr. 16 ausgewählt sind.

4. Verfahren zum Nachweisen nach Anspruch 3, bei dem die Resistenzmarker Marker des VanA-Typs sind, die aus den Peptiden der Sequenz SEQ ID Nr. 6, SEQ ID Nr. 7, SEQ ID Nr. 8, SEQ ID Nr. 11, SEQ ID Nr. 12, SEQ ID Nr. 13, SEQ ID Nr. 15, SEQ ID Nr. 16 ausgewählt sind.

5. Verfahren zum Nachweisen nach Anspruch 2, bei dem die Resistenzmarker Peptide des VanA-Typs sind, die aus den Peptiden der Sequenz SEQ ID Nr. 5 bis SEQ ID Nr. 16 ausgewählt sind.

6. Verfahren zum Nachweisen nach Anspruch 1, bei dem die Resistenzmarker Marker des VanB-Typs sind, die aus den Peptiden der Sequenz SEQ ID Nr. 29 bis SEQ ID Nr. 35 ausgewählt sind.

7. Verfahren zum Nachweisen nach Anspruch 2, bei dem die Resistenzmarker Marker des VanB-Typs sind, die aus den Peptiden der Sequenz SEQ ID Nr. 29, SEQ ID Nr. 30, SEQ ID Nr. 32 und SEQ ID Nr. 33 ausgewählt sind.

8. Verfahren zum Nachweisen nach Anspruch 1, bei dem die Resistenzmarker Marker des VanC-Typs sind, die aus den Peptiden der Sequenz SEQ ID Nr. 59 bis SEQ ID Nr. 71 ausgewählt sind.

9. Verfahren zum Nachweisen nach Anspruch 1, bei dem die Resistenzmarker Marker des VanD-Typs sind, die aus den Peptiden der Sequenz SEQ ID Nr. 80 bis SEQ ID Nr. 83 ausgewählt sind.

10. Verfahren zum Nachweisen nach Anspruch 9, bei dem die Resistenzmarker Marker des VanD-Typs sind, die aus den Peptiden der Sequenz SEQ ID Nr. 80, SEQ ID Nr. 81 und SEQ ID Nr. 83 ausgewählt sind.

11. Verfahren zum Nachweisen nach Anspruch 2, bei dem die Resistenzmarker Marker des VanD-Typs sind, die aus den Peptiden der Sequenz SEQ ID Nr. 80, SEQ ID Nr. 81 und SEQ ID Nr. 83 ausgewählt sind.

12. Verfahren zum Nachweisen nach Anspruch 1, bei dem die Resistenzmarker Marker des VanE-Typs sind, die aus den Peptiden der Sequenz SEQ ID Nr. 85 bis SEQ ID Nr. 91 ausgewählt sind.

13. Verfahren zum Nachweisen nach Anspruch 2, bei dem der Resistenzmarker ein Marker des VanE-Typs ist, der dem Peptid der Sequenz SEQ ID Nr. 89 entspricht.

14. Verfahren zum Nachweisen nach Anspruch 1, bei dem die Resistenzmarker Marker des VanG-Typs sind, die aus den Peptiden der Sequenz SEQ ID Nr. 95 bis SEQ ID Nr. 102 ausgewählt sind.

15. Verfahren zum Nachweisen nach Anspruch 14, bei dem die Resistenzmarker Marker des VanG-Typs sind, die aus den Peptiden der Sequenz SEQ ID Nr. 97 und SEQ ID Nr. 100 ausgewählt sind.

16. Verfahren zum Nachweisen nach Anspruch 2, bei dem die Resistenzmarker Marker des VanG-Typs sind, die aus den Peptiden der Sequenz SEQ ID Nr. 95, SEQ ID Nr. 96, SEQ ID Nr. 97, SEQ ID Nr. 98, SEQ ID Nr. 100 und SEQ ID Nr. 102 ausgewählt sind.

17. Verfahren zum Nachweisen nach Anspruch 1, bei dem die Resistenzmarker Marker des VanL-Typs sind, die aus den Peptiden der Sequenz SEQ ID Nr. 104 bis SEQ ID Nr. 120 ausgewählt sind.

18. Verfahren zum Nachweisen nach Anspruch 1, bei dem die Resistenzmarker Marker des VanM-Typs sind, die aus den Peptiden der Sequenz SEQ ID Nr. 122 bis SEQ ID Nr. 139 ausgewählt sind.

19. Verfahren zum Nachweisen nach Anspruch 1, bei dem die Resistenzmarker Marker des VanN-Typs sind, die aus den Peptiden der Sequenz SEQ ID Nr. 141 bis SEQ ID Nr. 154 ausgewählt sind.

## Claims

1. A method of detection, for at least one microorganism contained in a sample, of at least one marker of resistance to a glycopeptide which is vancomycin, comprising detection, by mass spectrometry of MRM type, of at least one peptide of said microorganism, which are peptides of the Van type selected from the peptides of sequence SEQ ID No. 5 to 16, 29 to 35, 5 to 71, 80 to 83, 85 to 91, 95 to 102, 104 to 120, 122 to 139 or 141 to 154.

2. The method of detection as claimed in claim 1, comprising a step prior to the detection step consisting of induction of the mechanism of resistance by bringing said sample into contact with said glycopeptide beforehand.

3. The method of detection as claimed in claim 1, in which said markers of resistance are proteins of the VanA type selected from the peptides of sequence SEQ ID No. 5 to SEQ ID No. 16.

4. The method of detection as claimed in claim 3, in which said resistance markers are markers of the VanA type selected from the peptides of sequence SEQ ID No. 6, SEQ ID No. 7, SEQ ID No. 8, SEQ ID No. 11, SEQ ID No. 12, SEQ ID No. 13, SEQ ID No. 15, SEQ ID No. 16.

5. The method of detection as claimed in claim 2, in which said markers of resistance are peptides of the VanA type selected from the peptides of sequence SEQ ID No. 5 to SEQ ID No. 16.

6. The method of detection as claimed in claim 1, in which said resistance markers are markers of the VanB type selected from the peptides of sequence SEQ ID No. 29 to SEQ ID No. 35.

7. The method of detection as claimed in claim 2, in which said resistance markers are markers of the VanB type selected from the peptides of sequence SEQ ID No. 29, SEQ ID No. 30, SEQ ID No. 32 and SEQ ID No. 33.

8. The method of detection as claimed in claim 1, in which said resistance markers are markers of the VanC type selected from the peptides of sequence SEQ ID No. 59 to SEQ ID No. 71.

9. The method of detection as claimed in claim 1, in which said resistance markers are markers of the VanD type selected from the peptides of sequence SEQ ID No. 80 to SEQ ID No. 83.

10. The method of detection as claimed in claim 9, in which said resistance markers are markers of the VanD type selected from the peptides of sequence SEQ ID No. 80, SEQ ID No. 81 and SEQ ID No. 83.

11. The method of detection as claimed in claim 2, in which said resistance markers are markers of the VanD type selected from the peptides of sequence SEQ ID No. 80, SEQ ID No. 81 and SEQ ID No. 83

12. The method of detection as claimed in claim 1, in which said resistance markers are markers of the VanE type selected from the peptides of sequence SEQ ID No. 85 to SEQ ID No. 91.

13. The method of detection as claimed in claim 2, in which said resistance marker is a marker of the VanE type corresponding to the peptide of sequence SEQ ID No. 89.

14. The method of detection as claimed in claim 1, in which said resistance markers are markers of the VanG type selected from the peptides of sequence SEQ ID No. 95 to SEQ ID No. 102.

15. The method of detection as claimed in claim 2, in which said resistance markers are markers of the VanG type selected from the peptides SEQ ID No. 97 and SEQ ID No. 100.

16. The method of detection as claimed in claim 2, in which said resistance markers are markers of the VanG type selected from the peptides of sequence SEQ ID No. 95, SEQ ID No. 96, SEQ ID No. 97, SEQ ID No. 98, SEQ ID No. 100 and SEQ ID No. 102.

17. The method of detection as claimed in claim 1, in which said resistance markers are markers of the VanL type selected from the peptides of sequence SEQ ID No. 104 to SEQ ID No. 120.

18. The method of detection as claimed in claim 1, in which said resistance markers are markers of the VanM type selected from the peptides of sequence SEQ ID No. 122 to SEQ ID No. 139.

19. The method of detection as claimed in claim 1, in which said resistance markers are markers of the VanN type selected from the peptides of sequence SEQ ID No. 141 to SEQ ID No. 154.
